# EUROPEAN PATENT APPLICATION

(11) **EP 1 826 266 A1**
(43) Date of publication of application: **29.08.2007**
(21) Application number: 06003721.5
(22) Date of filing: 23.02.2006
(51) Int. Cl.: C12N 9/02, D21C 5/00

(54) **Polypeptides with laccase activity**

(71) Applicant: Helmholtz-Zentrum für Infektionsforschung GmbH, 38124 Braunschweig (DE); Vialactia Bioscience (NZ) Limited, Newmarket, Auckland (NZ)
(72) Inventor: Strömpl, Carsten, 38173 Evessen (DE); Golyshin, Peter, Dr., 38302 Wolfenbüttel (DE); Ferrer, Manuel, Dr., 28007 Madrid (ES); Chernikova, Tatyana, Dr., 700007 Tashkent (UZ); Golyshina, Olga, Dr., 38302 Wolfenbüttel (DE); Timmis, Kenneth, Prof. Dr., 38300 Wolfenbüttel (DE); Elborough, Kieran, Pukekohe Franklin (NZ); Jarvis, Graeme, Paraparaumu 6010 Wellington (NZ)
(74) Representative: Graf von Stosch, Andreas

(57) **Abstract**

The invention relates to a new laccase from rumen, namely the RL5 laccase. In particular, the invention relates to a polypeptide comprising the amino acid sequence shown in Figure 9 and comprising at least the amino acids No. 80 to No. 150 of the amino acid sequence shown in Figure 9, respectively. The invention also relates to the use of polypeptides further comprising the amino acid sequence of a DUF152 domain with a core sequence as shown in Figure 16 as laccases. Furthermore, the invention relates to methods for producing inventive polypeptides and their use.

SEQUENCE LISTING

<110> GBF - Gesellschaft fuer Biotechnologische Forschung mbH

<120> Polypeptides with laccase activity

<130> GB01P006EP

<140> EP 06003721.5
<141> 2006-02-23

<160> 16

<170> PatentIn version 3.3

<210> 1
<211> 8
<212> PRT
<213> Unknown

<220>
<223> Description of sequence: Common core peptide sequence of DUF 152
domains (see description p. 6 and Fig. 16)

<400> 1

His Ala Gly Trp Arg Gly Thr Val
1               5


<210> 2
<211> 44
<212> DNA
<213> Artificial

<220>
<223> Description of sequence: Primer sequence RL5F-Nde for
purification of RL5 variants (see description p. 38)

<400> 2
gaaggagata tacatatgat agaacttgag aaattggatt ttgc 44


<210> 3
<211> 55
<212> DNA
<213> Artificial

<220>
<223> Description of sequence: Primer sequence RL5R-Kpn for
purification of RL5 variants (see description p. 38-39)

<400> 3
tggtacctta gtggtggtgg tggtggtgtt tcctatatat tccggtgaag gtgcg 55


<210> 4
<211> 30
<212> DNA
<213> Artificial

<220>
<223> Description of sequence: Primer sequence BT4389FNdeI for cloning
and purification of DUF152 hypothetical proteins (see description
p. 40)

<400> 4
acatatgatt tcaatcacaa aagataaaag 30

<210>  5
<211>  48
<212>  DNA
<213>  Artificial

<220>
<223>  Description of sequence: Primer sequence BT4389RKpn for cloning
       and purification of DUF152 hypothetical proteins (see description
       p. 40)

<400>  5
tggtacctta gtggtggtgg tggtggtgtt atttatgaat catgatga                    48


<210>  6
<211>  26
<212>  DNA
<213>  Artificial

<220>
<223>  Description of sequence: Primer sequence DUF152NdeIF for cloning
       and purification of DUF152 hypothetical proteins (see description
       p. 40)

<400>  6
acatatgagt aagctgattg tcccgc                                            26


<210>  7
<211>  28
<212>  DNA
<213>  Artificial

<220>
<223>  Description of sequence: Primer sequence DUF152XhoIR for cloning
       and purification of DUF152 hypothetical proteins (see description
       p. 40)

<400>  7
tctcgagcca aatgaaactt gccatacg                                          28


<210>  8
<211>  262
<212>  PRT
<213>  Unknown

<220>
<223>  Description of sequence: Amino acid sequence of RL5 laccase: 3217
       to 2414: frame 1 - 268 amino acids (see Figure 9)

<400>  8

Met Ile Glu Leu Glu Lys Leu Asp Phe Ala Lys Ser Val Glu Gly Val
1               5                   10                  15


Glu Ala Phe Ser Thr Thr Arg Gly Gln Val Asp Gly Arg Asn Ala Tyr
            20                  25                  30


Ser Gly Val Asn Leu Cys Asp Tyr Val Gly Asp Asp Ala Leu Arg Val
        35                  40                  45


Leu Asp Ala Arg Leu Thr Leu Ala Met Gln Leu Gly Val Asp Leu Asp

50                  55                  60

Asp Leu Val Met Pro Arg Gln Thr His Ser Cys Arg Val Ala Val Ile
65                  70                  75                  80

Asp Glu Arg Phe Arg Ala Leu Asp Ile Asp Glu Gln Glu Ala Ala Leu
                85                  90                  95

Glu Gly Val Asp Ala Leu Val Thr Arg Leu Gln Gly Ile Val Ile Gly
            100                 105                 110

Val Asn Thr Ala Asp Cys Val Pro Ile Val Leu Val Asp Ser Gln Ala
        115                 120                 125

Gly Ile Val Ala Val Ser His Ala Gly Trp Arg Gly Thr Val Gly Arg
    130                 135                 140

Ile Ala Lys Ala Val Val Glu Glu Met Cys Arg Gln Gly Ala Thr Val
145                 150                 155                 160

Asp Arg Ile Gln Ala Ala Met Gly Pro Ser Ile Cys Gln Asp Cys Phe
                165                 170                 175

Glu Val Gly Asp Glu Val Val Glu Ala Phe Lys Lys Ala His Phe Asn
            180                 185                 190

Leu Asn Asp Ile Val Val Arg Asn Pro Ala Thr Gly Lys Ala His Ile
        195                 200                 205

Asp Leu Arg Ala Ala Asn Arg Ala Val Leu Val Ala Ala Gly Val Pro
    210                 215                 220

Ala Ala Asn Ile Val Glu Ser Gln His Cys Ser Arg Cys Glu His Thr
225                 230                 235                 240

Ser Phe Phe Ser Ala Arg Arg Leu Gly Ile Asn Ser Gly Arg Thr Phe
                245                 250                 255

Thr Gly Ile Tyr Arg Lys
            260

<210>  9
<211>  270
<212>  PRT
<213>  Bacteroides thetaiotaomicron

<220>
<221>  misc_feature
<223>  Description of sequence: amino acid sequence of protein BT4289
       with unknown function DUF152 from Bacteroides thetaiotaomicron
       (Gene Bank accession number NP_813300) (see Fig. 10)

<400>  9

Met Ile Ser Ile Thr Lys Asp Lys Arg Met Leu Gly Tyr Glu Ser Leu
1               5                   10                  15

Ser Ser Tyr Ser Asn Ile Ser His Phe Val Thr Thr Arg Gln Gly Gly
            20                  25                  30

Cys Ser Glu Gly Asn Tyr Ala Ser Phe Asn Cys Thr Pro Tyr Ser Gly
        35                  40                  45

Asp Glu Ala Glu Lys Val Arg Arg Asn Gln Thr Leu Leu Met Glu Gly
    50                  55                  60

Met Ser Gln Ile Pro Glu Glu Leu Val Ile Pro Val Gln Thr His Glu
65                  70                  75                  80

Thr Asn Tyr Leu Leu Ile Gly Asp Ala Tyr Leu Ser Ala Ser Ser Gln
                85                  90                  95

Gln Arg Gln Glu Met Leu His Gly Val Asp Ala Leu Ile Thr Arg Glu
            100                 105                 110

Pro Gly Tyr Cys Leu Cys Ile Ser Thr Ala Asp Cys Val Pro Val Leu
        115                 120                 125

Val Tyr Asp Lys Lys His Gly Ala Ile Ala Ala Ile His Ala Gly Trp
    130                 135                 140

Arg Gly Thr Val Ala Tyr Ile Val Arg Asp Thr Leu Leu Arg Met Glu
145                 150                 155                 160

Lys Glu Phe Gly Thr Ser Gly Glu Asp Val Val Ala Cys Ile Gly Pro
                165                 170                 175

Ser Ile Ser Leu Ala Ser Phe Glu Val Gly Glu Glu Val Tyr Glu Ala
            180                 185                 190

Phe Gln Lys Asn Gly Phe Asp Met Pro Arg Ile Ser Ile Arg Lys Glu
        195                 200                 205

Glu Thr Gly Lys His His Ile Asp Leu Trp Glu Ala Asn Arg Met Gln
    210                 215                 220

Ile Leu Ala Phe Gly Val Pro Ser Gly Gln Val Glu Leu Ala Arg Ile
225                 230                 235                 240

Cys Thr Tyr Ile His His Asp Glu Phe Phe Ser Ala Arg Arg Leu Gly
                245                 250                 255

Ile Lys Ser Gly Arg Ile Leu Ser Gly Ile Met Ile His Lys
            260                 265                 270

<210>  10
<211>  243
<212>  PRT
<213>  Escherichia coli


<220>
<221>  misc_feature
<223>  Description of sequence: amino acid sequence of protein YifH with
       unknown function DUF152 from Escherichia coli (Gene Bank
       accession number AAG57706) (see Fig. 11)

<400>  10

Met Ser Lys Leu Ile Val Pro Gln Trp Pro Leu Pro Lys Gly Val Ala
1               5                   10                  15

Ala Cys Ser Ser Thr Arg Ile Gly Gly Val Ser Leu Pro Pro Tyr Asp
            20                  25                  30

Ser Leu Asn Leu Gly Ala His Cys Gly Asp Asn Pro Asp His Val Glu
        35                  40                  45

Glu Asn Arg Lys Arg Leu Phe Ala Ala Gly Asn Leu Pro Ser Lys Pro
    50                  55                  60

Val Trp Leu Glu Gln Val His Gly Lys Asp Val Leu Asn Leu Thr Gly
65                  70                  75                  80

Glu Pro Tyr Ala Ser Lys Arg Ala Asp Ala Ser Tyr Ser Asn Thr Pro
                85                  90                  95

Gly Arg Val Cys Ala Val Met Thr Ala Asp Cys Leu Pro Val Leu Phe
            100                 105                 110

Cys Asn Arg Ala Gly Thr Glu Val Ala Ala Ala His Ala Gly Trp Arg
        115                 120                 125

Gly Leu Cys Ala Gly Val Leu Glu Glu Thr Val Ser Cys Phe Ala Asp
    130                 135                 140

Asn Pro Glu Asn Ile Leu Ala Trp Leu Gly Pro Ala Ile Gly Pro Arg
145                 150                 155                 160

Ala Phe Glu Val Gly Ala Glu Val Arg Glu Ala Phe Met Ala Val Asp
                165                 170                 175

Ala Glu Ala Ser Thr Ala Phe Ile Gln His Gly Asp Lys Tyr Leu Ala
            180                 185                 190

Asp Ile Tyr Gln Leu Ala Arg Gln Arg Leu Ala Asn Val Gly Val Glu
        195                 200                 205

Gln Ile Phe Gly Gly Asp Arg Cys Thr Tyr Thr Glu Asn Glu Thr Phe

210                 215                 220

Phe Ser Tyr Arg Arg Asp Lys Thr Thr Gly Arg Met Ala Ser Phe Ile
225                 230                 235                 240

Trp Leu Ile

<210>  11
<211>  5596
<212>  DNA
<213>  Unknown

<220>
<223>  Description of sequence: Nucleic acid sequence of RL5 laccase,
       Contig[RL5] 5596 bases, 487 checksum. The sequence of the whole
       DNA insert. Underlined the DNA sequence of RL5 laccase (opposite
       direction). (See Fig. 12)

<400>  11
ggatccagta gtcggggatg cccatcgcca tgcggtagtc aaagccgatg ccgccgtcct      60

tgaaagtgcg ggccagtccg ggcatgccgc tcatttcctc ggcaatggtg atggcatggg     120

gattgatggt gtggatcagc acgttggcca gcgtgagata ggtgatggca ttggtgtcct     180

cgccgccatt gtaataatcg tcataactgc cgaatgcctg tcccaagccg tggctgtaat     240

agagcatcga ggtcacgcca tcgaagcgga agccgtcaaa cttgtactcc tgcagccaga     300

acttgcagtt ggacagcagg aaattcaaca cggcattctt gccgtaatcg aagcacaacg     360

agtcccaagc cggatgttcg cgcttgtcat cgccgtagaa gtacaagtcg cccgtgccgt     420

caaagcagcc caaaccctcg acctcgttct tcaccgcatg ggagtgcacg atgtccatga     480

taacggccac gcccgcggca tgggcgtcat cgatgaggtg tttcaactcc tcgggagtgc     540

cgaaacgcga cgaggctgca taaaaactcg acacatggta gccgaacgag ccgtagtaag     600

gatgctcctg gatggccatg atctggatgg cattgtagcc gtcagcaacg atgcgcggca     660

atacgttcac gcggaactcc tcataggtac ccacaccctc gcggttctgc gccatgccga     720

tgtggcactc atagatgagc agcggacccg tgttgggcac aaagtccttg accttgaact     780

cgtagggctg ttcgggtgcc cacacctgtg ccgagaagat taaggtgttc tcgtcctgca     840

ccacgcgcat ggcataggcc gggatgcgct cgccttctcc gccctgccag tgcacgcgca     900

gcttgtacag gtcgccgtgg tgcatggcgc gctcgggcaa gacgatttcc caattgccgt     960

tgtccagccg tttcagcttg taggggcac actcgcgcca gtcgttgaac tggccgataa    1020

gataaatctc ggtagcgtta ggtgcccatt cacggaacac ccagttgccg tcggtcgtgc    1080

ggtgcaagcc atagtagttg taggcattgg caaactcgac cagattgccc gtgcctcccg    1140

ttagctcggc tatcttgttc acagcatcct gatgccgccc gttgatggcg tcgctgtaag    1200

gctccagcca cgggtcatcg ttgataatcg caagattctt tttcttcctc attttcggtc    1260

agttcttatt aagtgatgag cacaaaggta caaattaaac ctcaatatca ttgcttcttt    1320

acaccgcttt ttgcatttct gccccgaaat cgattgcaat tcatcgagaa aaatgaagaa    1380

aaaacgcgag aatttgatgc attattaaaa aaaacattaa atttgcactc tttagaggag 1440
aataataaca acttaaaaat agcattatca ctatgagact agaaggaaga cgcgaaagcg 1500
aaaatgttga agatcgccgc agaatgggca ccggtaccaa gattggtctg ggtggcatcg 1560
gcggactgat tattgccgga ctgatcaccc tgctgatggg tggcaacatc ggcgacgtgt 1620
tacaacaggc tggtggcatg gctatgcaga acgagaccgt acaggaaggc gagttcagcg 1680
aggaggaaca ggaactggcc accttctcca agcaggtcat tgccagcacc gaggacatct 1740
ggtcacagat tttcagagag tatggaatcg gcgaatatcc cgctcccacg ctggtgctgt 1800
acaccggtac gacccaaacc gcttgcggac agggtaatgc cgccgtcgga ccgttctact 1860
gctcgggcga ccagaaggtt tatctcgacc tttcattctt ccagaccatg gaccgtcaat 1920
tgggcgtgaa gggtgacaac tccagccttg ccaaggccta tgtcatcgcc cacgaggtgg 1980
gtcaccacat cgagtacctg atgggcaccc tggacaaggc tcaccagcgc atgcaggcca 2040
ccaaccagac caatgccaac aagtacagcg tgcgtctgga actcatggcc gacttctatg 2100
ccggcgtgtg ggcacactat gagagcagga tgttcggctc catctccgac aaggacctgc 2160
aggaagccat cgactgcgct cagaagatcg gtgacgacta cctgcagaag cgttcacagg 2220
gctatgcaca gcccgagagc ttcacccacg gcaccagcgc acagcgcatg aagtggttca 2280
agctgggtta ccagaccggc gacgtgcgcc gcgccaccac cttccaggaa agcgacgcca 2340
ccctgtaaaa cccattgaca acagtatcca aaatcgtgac tgggaagtta tcctcggtca 2400
cggttttgtt ttatttccta tatattccgg tgaaggtgcg gccgctgttg atgcccaagc 2460
ggcgggctga gaagaaactg gtgtgctcgc agcgggagca atgctgtgac tcgacgatat 2520
tggccgcggg cacgccggct gcaaccagca cagcgcgatt ggcggcgcgc aggtcgatgt 2580
gggccttgcc agtggcgggg ttgcgcacta cgatatcatt gaggttgaaa tgggcctttt 2640
tgaaagcttc gaccacttcg tcacccacct cgaaacagtc ctggcagatg ctgggcccca 2700
tcgctgcctg aatgcggtcg acagtagcgc cttgacggca catttcctca accacagcct 2760
tggcaatacg tcccaccgtg ccacgccagc ccgcgtggga cacggcgaca ataccggctt 2820
gactgtcaac caggacgatg ggcacgcaat cggccgtatt cacgccgatg acgatgcctt 2880
gcagccttgt caccaatgcg tctacaccct cgagggccgc ctcctgctcg tcgatgtcca 2940
gcgcgcggaa acgctcgtcg atgacggcca cacggcacga atgcgtctgc cgcggcatga 3000
ccaggtcatc gaggtcaaca cccagctgca tcgccagcgt gagccgcgca tccagcaccc 3060
gcagggcatc gtcgcccaca tagtcacaca agttcacgcc cgagtaggca ttgcggccat 3120
ccacctggcc ccgtgtcgtc gagaaggcct caacgccctc gactgacttt gcaaaatcca 3180
atttctcaag ttctatcatg acaacaaagg taacatttta ttcccattag ttcaccttag 3240
atcgaagaaa tctcagtacc tttgcacatc atgacactga atacggccac catagtcaac 3300
tacaagaaca tcgccgaggc gcggttggag ttctcgccca agctgaactg cctgatcggc 3360
aacaacgggc agggcaagac caacgtgctc gatgccatct attacctgag catgtgccgc 3420

agttttgcct cgacaagcga caacagcgcg gtcatacgcc atggcgagcc ctacatgatg 3480
ctccagggca gttacacccg tcaagagaca ccgctggaaa tcagcgtcgc ccttcagcgc 3540
ggcaagcgca aggtcgtgcg ccgcgacggc aaggaatacc agagactgag ccaacacatc 3600
ggcctgttgc ccgtggtcat ggtgtcgccc atggactggg atctggtgcg cggcagcggt 3660
gaggagcgcc gccgtttcat ggacctgatc atctcacaga acgacaacga atacctcgat 3720
gccctcatcc gctacaacaa ggccgtcgag cagcgcaatg ccatgatcaa gaaggagatg 3780
cgcgacccgt tgctgtatga gaccgtcgag caggccatgg cacagcatgc cgccttgatc 3840
caccagcgcc gcagccagtg ggtagagcaa ttcctgccca tcttcatgca ctactatcat 3900
gccgtggccg gtgacaacga gacggtgagc ctgcactaca agagccacct caacgacggc 3960
acgatgcagg agcacttggc cgccacacgc gagcgcgacc tcatcatcgg ccacaccaca 4020
cgcggcatcc accgcgacga catcgagctc atgctcgacg attaccccat gcgccacacg 4080
ggcagccagg gccagtgcaa gacctacacc attgccctga ggttcgccca gtttgacttc 4140
ctcaaggcca acaacgccac cgtgcccatc ctgctgcttg acgatatctt tgaccgcctc 4200
gatgccagcc gtgtcgagcg catcgtcgat gttgtgtcaa gcgaccgttt cggccagatc 4260
ttcattaccg acaccaaccg cacccatctc gacgaaatcg tcgcccgtca tggtggcggc 4320
cactgcctga tgcaggtcga gaacggcaac gtcacaacgc tgaaaggagg agaacaggca 4380
tgaaacgcac cgaggccaaa aacgtcggcc aaatcatcaa cgacctgctc aagaaggaaa 4440
atctggacgt tgccctcgac gagcaccgcg ccagtgccct gtggccccaa atcgtgggcg 4500
acggcatcaa ccgctacacc atctcgcgaa gcgtgacggg cggcgtgatg accgtacgcc 4560
tgtcttcggc ctcgctggcc aacgagctga tgctcatccg cgccagcatc atccaacgca 4620
tcaacgaggc cctgggccgc gaaatcatcc acgaaatcat cttcaaataa cgacaatgaa 4680
tcacgcaatc gaatcaccat accccttcaa gtgttcaacc cccatgcagc tgcgtttcaa 4740
cgacgtggac gcactgggcc acgtcaacaa ttcggtctat ttccagttct ttgacctggg 4800
caagacgcat tatttcaaag gattgaaagt gcaggccgac atcgactggc gccgtcccac 4860
ggtcatcatc gccaatgtga actgcagttt ccttaagccg accctgttca acgagcccgt 4920
cgatgtgctc acccagtgcc tgcgcttggg caacaagagc ctgacgctgt tgcagcatgt 4980
ggtcaatagc gacacccgcg aggtgaaagc cacctgtgcc accgtgatgg tcaacatggc 5040
tcccgagacc aaccagccca gcccgatccc tcaggtgtgg cgcgacgcca tcgtcgcctt 5100
tgaggggcac gagtaaaggg aataagaaca aaaactaagg actaaagact tttcctcgcc 5160
ttgcgtgtgg ctatgagatt gattgcccag cccacggcca taatcataac agccaataac 5220
agcatgccac ctcccgcaat gattttcatt tgcagcatct catcaggtga gatttgcttg 5280
ttgagagggg caagagcatc agcaccaaag aatgcgaaaa tcaacccata ctcgattaag 5340
acgatgggca gtaagacgaa tgcctcacgc caaagggtgc cccaaaatcc gtaaccgaaa 5400
agctgcatat aaccaacgat gtaatagaac atcgttaata ggactatagc gatattaaag 5460

gcaatgagat ttaccagcac cagatatatc aagaagaaat tcagcaccac catcagcact 5520

gaaaagagga cttgaatgaa aaagccctcg ggcagactgt gacgggtatt gcgcggcgcg 5580

taccggaaca tgatcc 5596

<210> 12
<211> 813
<212> DNA
<213> Bacteroides thetaiotaomicron

<220>
<221> misc_feature
<223> Description of sequence: Nucleic acid sequence of protein BT4389
      with unknown function DUF152 from Bacteroides thetaiotaomicron
      (Gene Bank accession number NP_813300) (see Fig. 13)

<400> 12
atgatttcaa tcacaaaaga taaaagaatg ttggggtatg agtcgttaag ctcatactcc 60

aacatttctc attttgtaac tacccggcag ggagggtgca gcgagggaaa ttatgcgtca 120

ttcaactgta cgccttacag tggcgacgag gcagaaaagg tccggcggaa tcagacgctt 180

ctgatggaag gaatgtcgca gatacccgaa gaactggtga ttcctgtgca gacccatgag 240

acgaattacc tgctgatcgg cgatgcttat ttatcggctt cgtctcaaca acgacaggag 300

atgctgcacg gagtggacgc tttgattacc cgcgagccgg gatattgtct ttgtatttct 360

actgcggatt gtgtgcccgt gttggtatat gataagaaac atggtgccat agccgccatt 420

catgccggat ggagagggac ggtagcgtac atcgtacgtg atactttgtt gcggatggaa 480

aaagagtttg gcacaagtgg agaagatgtg gtggcctgta tcggtccgag tatctcattg 540

gcttcttttg aagtgggtga ggaagtgtac gaagcatttc agaagaatgg ttttgatatg 600

ccccgtattt ctatcaggaa agaggaaacc ggaaaacatc atatcgactt gtgggaagcg 660

aaccgaatgc aaatccttgc tttcggtgtg ccgtccggac aggtggagct tgcccggata 720

tgtacgtata ttcatcacga cgagtttttc tcggcaaggc gtttgggcat taagtccgga 780

cggattctgt cggggatcat gattcataaa taa 813

<210> 13
<211> 729
<212> DNA
<213> Escherichia coli

<220>
<221> misc_feature
<223> Description of sequence: Nucleic acid sequence of protein YifH
      with unknown function DUF152 from Escherichia coli (Gene Bank
      accession number AAG57706) (see Fig. 14)

<400> 13
atgagcaaac tgattgtgcc gcagtggccg ctgccgaaag gcgtggcggc gtgcagcagc 60

acccgtattg gcggcgtgag cctgccgccg tatgatagcc tgaacctggg cgcgcattgc 120

ggcgataacc cggatcatgt ggaagaaaac cgtaaacgtc tgtttgcggc gggcaacctg 180

ccgagcaaac cggtgtggct ggaacaggtg catggcaaag atgtgctgaa cctgaccggc 240

gaaccgtatg cgagcaaacg tgcggatgcg agctatagca acaccccggg ccgtgtgtgc 300

gcggtgatga ccgcggattg cctgccggtg ctgttttgca accgtgcggg caccgaagtg 360

gcggcggcgc atgcgggctg gcgtggcctg tgcgcgggcg tgctggaaga aaccgtgagc 420

tgctttgcgg ataacccgga aaacattctg gcgtggctgg gcccggcgat tggcccgcgt 480

gcgtttgaag tgggcgcgga agtgcgtgaa gcgtttatgg cggtggatgc ggaagcgagc 540

accgcgttta ttcagcatgg cgataaatat ctggcggata tttatcagct ggcgcgtcag 600

cgtctggcga acgtgggcgt ggaacagatt tttggcggcg atcgttgcac ctataccgaa 660

aacgaaacct tttttagcta tcgtcgtgat aaaaccaccg gccgtatggc gagctttatt 720

tggctgatt 729

<210> 14
<211> 729
<212> DNA
<213> Escherichia coli

<220>
<221> misc_feature
<223> Description of sequence: Nucleic acid sequence of the protein
YifH with unknown function DUF152 from Escherichia coli (Gene
Bank accession number AAG57706) (see Fig. 15)

<400> 14
atgagcaaac tgattgtgcc gcagtggccg ctgccgaaag gcgtggcggc gtgcagcagc 60

acccgtattg gcggcgtgag cctgccgccg tatgatagcc tgaacctggg cgcgcattgc 120

ggcgataacc cggatcatgt ggaagaaaac cgtaaacgtc tgtttgcggc gggcaacctg 180

ccgagcaaac cggtgtggct ggaacaggtg catggcaaag atgtgctgaa cctgaccggc 240

gaaccgtatg cgagcaaacg tgcggatgcg agctatagca acaccccggg ccgtgtgtgc 300

gcggtgatga ccgcggattg cctgccggtg ctgttttgca accgtgcggg caccgaagtg 360

gcggcggcgc atgcgggctg gcgtggcctg tgcgcgggcg tgctggaaga aaccgtgagc 420

tgctttgcgg ataacccgga aaacattctg gcgtggctgg gcccggcgat tggcccgcgt 480

gcgtttgaag tgggcgcgga agtgcgtgaa gcgtttatgg cggtggatgc ggaagcgagc 540

accgcgttta ttcagcatgg cgataaatat ctggcggata tttatcagct ggcgcgtcag 600

cgtctggcga acgtgggcgt ggaacagatt tttggcggcg atcgttgcac ctataccgaa 660

aacgaaacct tttttagcta tcgtcgtgat aaaaccaccg gccgtatggc gagctttatt 720

tggctgatt 729

<210> 15
<211> 270
<212> PRT
<213> Bacteroides fragilis

<220>
<221>  misc_feature
<223>  Description of sequence: Hypothetical protein sequence B.fra from
      Bacteroides fragilis (Accession No. AE006841.1) (see Fig. 17)

<400>  15

Met Ile Ser Leu Thr Asp Asp Arg Lys Met Leu Gly Tyr Gly Leu Leu
1               5                   10                  15

Gly Ala Tyr Pro Asn Ile Ser His Phe Val Thr Thr Arg His Gly Gly
            20                  25                  30

Tyr Ser Glu Gly Ala Tyr Ala Ser Phe Asn Cys Ser Pro Phe Ser Gly
        35                  40                  45

Asp Lys Leu Glu Arg Val Glu Lys Asn Gln Thr Leu Leu Phe Gln Ser
    50                  55                  60

Leu Ser Gln Ala Pro Arg His Leu Ile Ile Pro Phe Gln Thr His Gly
65                  70                  75                  80

Thr Lys Ile Leu Pro Val Asp Glu Lys Phe Leu Gly Ala Ser Gly Gln
                85                  90                  95

Gln Gln Gln Glu Met Leu Asn Gly Ile Asp Ala Leu Ile Thr Thr Glu
            100                 105                 110

Pro Gly Cys Cys Ile Cys Ile Ser Thr Ala Asp Cys Ile Pro Val Leu
        115                 120                 125

Leu Tyr Asp Arg Val His His Ala Val Ala Ala Val His Ala Gly Trp
    130                 135                 140

Arg Gly Thr Val Glu Tyr Ile Val Gly His Thr Leu Glu Lys Met Arg
145                 150                 155                 160

Ala Val Phe Gly Thr Glu Gly Gln Asp Val Ile Ala Cys Ile Gly Pro
                165                 170                 175

Gly Ile Ser Leu Gln Ser Phe Glu Val Gly Asp Glu Val Tyr Glu Ala
            180                 185                 190

Phe Arg Leu Asn Gly Phe Asp Met Ser Arg Ile Ser Phe Arg His Ser
        195                 200                 205

Val Thr His Lys Tyr His Ile Asp Leu Trp Glu Ala Asn Arg Gln Gln
    210                 215                 220

Leu Leu Asp Phe Gly Val Pro Gly Val Gln Ile Glu Ile Ala Asp Ile
225                 230                 235                 240

Cys Thr Tyr Ile Arg His Glu Asp Phe Phe Ser Ala Arg Arg Leu Gly

245                 250                 255

Ile Lys Ser Gly Arg Ile Leu Ser Gly Ile Met Ile Asn Ser
            260                 265                 270

<210>  16
<211>  256
<212>  PRT
<213>  Cytophaga hutchinsonii

<220>
<221>  misc_feature
<223>  Description of sequence: Hypothetical protein sequence from
       Cytophaga hutchinsonii (Accession No. AABD03000010.1)) (see Fig.
       17)

<400>  16

Met Gln Gln Leu Leu Ile Asp Gln Gln Gln His Trp Gln Phe Thr Val
1               5                   10                  15

Phe Asn Ala Trp Pro Ser Val Lys His Ile Val Thr Gly Arg Asn Pro
            20                  25                  30

His Ile His Arg Gly Asn Ile Ala Gly Leu Asn Tyr Gly Leu Asn Val
        35                  40                  45

Pro Asp Asp Pro Leu Ala Val Ala Gln Asn Arg Thr Glu Ile Ala Gln
    50                  55                  60

Leu Leu His Ala Ser Asp Ala Ala Val Val Ile Pro Phe Gln Thr His
65                  70                  75                  80

Ser Asn Asn Ile Ala Val Ile Asp Asp Ser Asn Lys Asp His Ile Phe
                85                  90                  95

Glu Asn Thr Asp Ala Leu Ile Thr Asn Val Pro Gly Ile Ile Ile Gly
            100                 105                 110

Thr Leu Ser Ala Asp Cys Val Pro Ile Leu Leu Ala Asp Pro Val Ala
        115                 120                 125

His Val Ile Ala Ser Ile His Ala Gly Trp Lys Gly Thr Val Ser Glu
    130                 135                 140

Ile Ala Lys His Thr Val Gln Arg Met Gln Lys Asp Phe Asn Cys Leu
145                 150                 155                 160

Pro Glu Asn Ile Leu Ala Gly Ile Gly Pro Ser Ile Ser Ala Ala Cys
                165                 170                 175

Tyr Glu Val Gly Glu Glu Val Ala Met His Phe Ser Asp Ser Cys Lys
            180                 185                 190

Ser Asp Ser Ser Asn Gly Lys Thr Cys Ile Asp Leu Trp Lys Ala Asn
        195                 200                 205
Gln Ala Gln Leu Ile Glu Ala Gly Leu Leu Pro Glu His Ile Glu Ile
    210                 215                 220
Ala Gly Arg Cys Thr Phe Ser Asn Pro Ala Asn Phe Tyr Ser Ala Arg
225                 230                 235                 240
Arg Asp Gly Ile Gln Thr Gly Arg Met Gly Ser Phe Ile Leu Phe Asn
                245                 250                 255

## Description

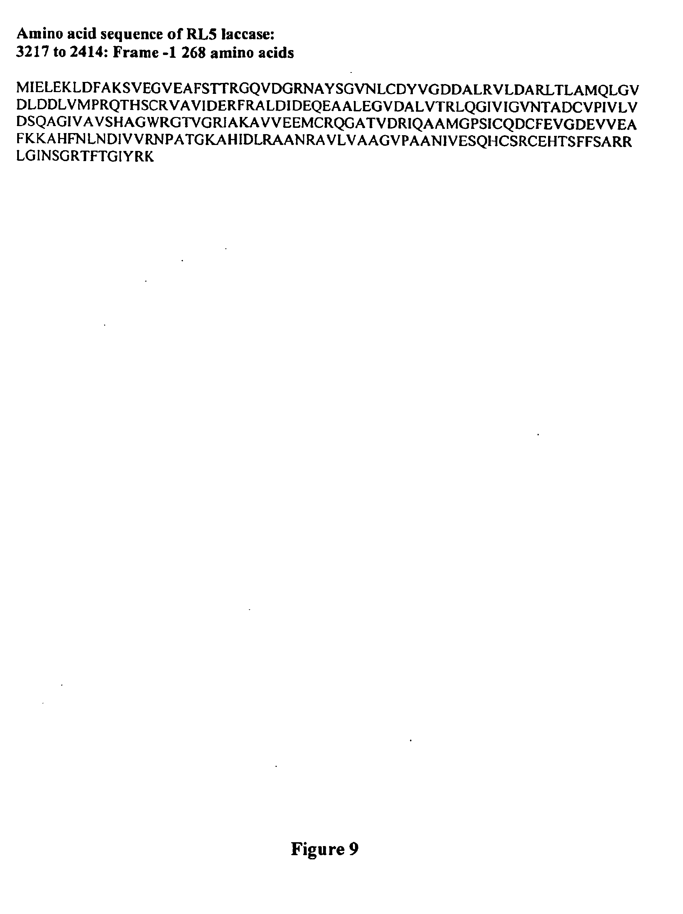

The invention relates to polypeptides having laccase activity. In particular, the invention relates to a polypeptide comprising the amino acids No. 80 to No. 150 of the amino acid sequence shown in Figure 9, respectively. Moreover, the present inventions relates to a yet unknown family of laccases.

Laccases (EC 1.10.3.2), also designated as benzenediol oxygen oxidoreductases, have been found widespread in eukaryotes, namely in fungi and less frequently in plants (Mayer, A.M. et al., 2002; Ruijssenaars, H.J. et al, 2003; Claus, H., 2003) as well as in prokaryotes (Claus, H., 2004; Solano et al., 2001; Martins et al., 2002; Mayer and Staples, 2002; Valderrama et al., 2003). They are involved in the patho-genicity, immunity and morphogenesis of organisms and the metabolic turnover of complex organic substrates, such as lignin or humic matter. For example, they are involved in the pigmentation process of fungal spores, the regeneration of tobacco plants, the lignification of cell walls and the delignification during white rot of wood and they act as fungal virulence factors.

Laccases are multi-copper enzymes of wide substrate specificity and high non-specific oxidation capacity that use molecular oxygen (dioxygen) to oxidize various aromatic compounds, such as phenol, polyphenols, aromatic amines, polycyclic aromatic hydrocarbons, as well as non-aromatic compounds, such as non-phenolic substrates, e.g., ferrocyanide. They oxidize their substrates by a one-electron transfer and reduce dioxygen to water. Laccases are also capable of performing polymerisation, depolymerisation, methylation and demethylation reactions (Sariaslani, 1989; Kawai et al., 1988; Bourbonnais and Price, 1990; Eggert et al., 1996; Solomon et al., 1996; Stolz, 2001; Shah and Nerud, 2002; Claus, 2004).

Laccases have been thoroughly studied, including their three-dimensional structure, by means of X-ray crystallography (Antorini et al., 2002; Bertrand et al., 2002a,b; Hakulinen et al., 2002; Piontek et al., 2002). A comparison among laccases has shown conserved regions in which histidine residues are abundant and important to bind four copper atoms distributed in two parts that are essential for the enzymatic activity (Solomon et al., 1996). The enzymes generally contain one each of type 1 (T1), type 2 (T2), type 3 (T3) copper sites per subunit. The T1 copper site, which has the highest oxidation potential, is assumed to be the first electron acceptor. The other three copper ions are located in two sites: one in the T2 copper site and two in a binuclear T3 copper site. The T2 and T3 copper sites are close together and these three copper ions form a trinuclear cluster, named T2/T3, where the electrons accepted in the T1 site are driven for substrate oxidation and oxygen reduction to water (Solomon et al., 1996; McGuirl and Dooley, 1999).

By reason of their high non-specific oxidation capacity and their wide substrate specificity, laccases are of great commercial and biotechnological interest. The application areas of laccases may be generally divided into:
- industrial-technical applications, e.g., for the use as delignifying agent to delignify woody tissues; in biobleaching of kraft pulp; in the production of wood-based compounded material; in the production of ethanol; in textile applications, such as cotton fiber whitening, dye finishing, dye decolourisation and detoxification, laundry cleaning; in detergent application, for example for bleaching stains or to inhibit so-called "dye-transfer" between cloths of different colours during wash procedures;
- food applications, e.g., as food improvement, for example for bread-making applications; to remove or modify problematic phenolic saccharides from clear fruit juice or fermented alcohol beverages, such as wine and beer, to improve the clarity, colour appearance, flavour, aroma, taste or stability;
- application for environment protection, e.g., for the use in biodegradation; in bioremediation; in biodetoxification; in biodecontamination;
- application as biosensors and bioreporters in combination with co-substrates that are either chromogenic, fluorogenic, chemiluminescent or electroactive.
- application for organic syntheses, e.g., for the use in asymmetric chiral synthesis, for example to transform prochiral aldehydes or ketones to chiral alcohol, hydroxyl acids, amino acids etc.; for synthesis of polymers, such as polyphenolic polymers; for synthesis of medicinal agents including triazolo(benzo)cycloalkyl thiadiazines, vinblastine, antibiotics and dimerized vindoline; and
- medical and personal care applications, e.g., for the use in products for disinfection, skin care, hair care, dental care; as deodorants for personal-hygiene products, such as toothpaste, mouthwash, chewing gum, detergent and soap.

It is desired to apply laccases without restricted substrate specificity, with high stability properties, optimum kinetic parameters and activity over a broad pH range for use in the above application areas. Ideally, laccases should be stable under the conditions given for a certain period of time.

However, there is still a strong need for laccases having activity over broad pH and temperature range. Some known laccases show activity under acidic (3.0 - 5.0), neutral or slightly alkaline (7.0 - 8.0) conditions (see Machczynski et al., 2004, and references therein). In case known laccases exhibit alkaline activity (up to pH 9.2), they lose their function under more acidic conditions, e.g. pH 6.0, (see Machczynski et al., 2004; Ruijssenaars and Hartmans, 2004).

Another problem in the field is due to lack of laccases showing long-term stability and activity under elevated temperature conditions. In particular, there is a need for laccases showing both stability/activity over a broad pH range and temperature insensitivity. In general, laccases show a temperature optimum at 40 °C to 50 °C (see, e.g., Koroleva et al, Biochemistry (Moscow), 2001, vol. 66, No. 6, pp. 618 - 622(5)). Although Boghos Dedeyan et al. (Appl Environ Microbiol. 2000 March; 66(3): 925-929) describe a laccase with optimum activity at 75°C (converting SGZ as substrate), use of this enzyme was restricted to acidic pH conditions (pH 4.5). Moreover, its stability was limited to just 1 hour.

Although, a wide variety of laccases has been characterized, none of them exhibits a broad profile for use in various industrial applications. There is no laccase known today, which combines long-term stability and activity over a broad temperature and pH range.

Therefore, it is an object of the invention to provide laccases, which may be used for various industrial applications due to insensitivity to diverse process conditions.

This object is solved by a polypeptide comprising amino acids No. 80 to No. 150 of the amino acid sequence shown in Figure 9 or a functional fragment or a functional derivative thereof. Preferably, the polypeptide comprises amino acids No. 75 to No. 170, more preferably No. 60 to No. 195 and even more preferably No. 50 to No. 210 of the amino acid sequence shown in Figure 9. A polypeptide comprising or consisting of the entire amino acid sequence shown in Figure 9 is most preferred.

This object is also solved by the identification of members of a large class of conserved proteins containing a DUF152 domain of a function yet unknown in the art. According to the present invention, these proteins are characterized as laccases exhibiting oxidative laccase-like activity.

The invention is based on the discovery that microbial diversity constitutes a largely unexplored source for new enzymes that can be exploited in biocatalytic processes. In particular, a symbiotic rumen ecosystem consists of mostly obligate anaerobic microorganism including fungi, protozoa, archaea and bacteria. The rumen ecosystem represents an anaerobic or microaerophilic environment characterized by a polymer plant substrate turnover. Peroxidases (lignin peroxidases) and Laccases (phenol oxidases) are central to the digestibility of the plant polymer lignin (Krause et al., 2003); along with a number of fibrolytic enzymes needed to degrade components of plant cell walls (such as hemicellulose), e.g. xylases, β-xylanases, arabino-furanosidase, cellulases, glucanohydrolases, glucosidases and endoglucanases and esterases.

According to the invention, a metagenome library of bovine rumen microflora was constructed and a bacteriophage lambda-based expression library was established from DNA extracted from bovine rumen fluid. The expression library was screened for laccase activity and a gene encoding the inventive polypeptide with laccase activity, named RL5, without significant homology to any known enzymes of this function, was isolated.

This inventive RL5 laccase was identified as a protein, which is related to a major class of conserved (hypothetical) proteins containing a so-called DUF152 domain of unknown function. RL5 laccase is a protein of a length of 268 amino acids, a molecular mass of 28.28 kDa and a pl of 5.19 forming a dimeric protein of Mᵣ 57 kDa, whereby the molecular mass of the inventive monomer (~28 kDa) is much lower than the monomeric size of known bacterial, plant and fungal laccases having a molecular weight of 50 to 110 kDa (see, e.g., Solomon, 1996; Claus, 2003). The smallest laccase ever reported with a monomeric molecular mass of 36 kDa (dimer of 69 kDa) was isolated from *Streptomyces coelicor* (Machcynski et al., 2004). The catalytic activity of the inventive RL5 laccase was analyzed and recombinant expression in *Escherichia coli* unambiguously demonstrated its oxidizing multipotency for a variety of laccase substrates at an unusually broad pH range: from 3.5 to 9.0. Moreover, copper binding sites were identified and temperature, kinetic and electrochemical properties analyzed. Biochemical and spectroscopic analysis revealed that RL5 belongs to a yet unknown family of laccases containing a DUF152 domain of yet unknown function. DUF 152 domains are characterized by a common core peptide sequence (one-letter-code) HAGWRGTV.

To identify other members of said new family of laccases and to prove that other conserved hypothetical proteins containing the DUF152 domain of unknown function are multi-copper proteins with laccase activity, hypothetical DUF152 domain containing proteins BT4389 from *B. thetaiotaomicron* (Gene Bank accession number ND_813300) and YifH from *Escherichia coli* (Gene Bank accession number AAG57706) were analyzed. BT4389 and YifH share 57% and 68% sequence similarity, respectively, with RL5. BT4389 and YifH were identified as proteins of a length of 270 amino acids, a molecular mass of about 30 kDa (predicted Mᵣ: 30,118 kDa) and a pl of 5.84 (BT4389) and a length of 243 amino acids, a molecular mass of 25 kDa (predicted Mᵣ: 26,337 Da) and a pl of 5 (YifH), respectively. Analysis of the native proteins revealed that they exist as dimers (~60 kDa and 55 kDa, respectively) and contain 4.0 mol of copper per monomer, similar to those values found for the inventive RL5 protein.

The catalytic activity of BT4389 and YifH was analyzed and recombinant expression in *Escherichia coli* unambiguously demonstrated their oxidizing multipotency for a variety of laccase substrates at an unusually by broad pH range: from 4.0 to 9.0, which corresponds to those values found for RL5 laccase. Therefore, also DUF152 domain containing proteins BT4389 and YifH were identified as laccases. Taken together, cloning and expression of (hypothetical) proteins BT4389 and YifH as well as biochemical and spectroscopic analyses unambiguously provides support for the finding that proteins containing a DUF152 domain are laccases. Thus, according to the invention, the function of a large class of conserved (hypothetical) bacterial proteins exhibiting laccase activity was identified. The use of said polypeptides as laccases is disclosed herewith.

In this context, it has to be noted that the terms "RL5", "RL5 laccase", "laccase RL5" and "RL5 protein" are used herein synonymously and designate a polypeptide according to the invention having laccase function. The same applies to the terms "BT4389", "BT4389 laccase", "laccase BT4389" and "BT4389 protein" as well as "YifH", "YifH laccase", "laccase YifH" and "YifH protein".

Polypeptides according to the invention exhibit oxidizing laccase activity under conditions as outlined above. The term "polypeptide" is intended to encompass an amino acid sequence consisting preferably of less than 300 amino acids. Generally, the polypeptide comprises more than 70 amino acids, more preferably more than 100, 120 or 150 amino acids, even more preferably more than 200 amino acids. The amino acids are connected to one another by amide bonds between the C-terminal carboxyl group and the N-terminal alpha-amino group of a neighboring amino acid. Inventive polypeptides comprise RL5 laccases and the polypeptides containing a DUF152 domain, which were identified as laccases according to the invention.

Polypeptides of the present invention exhibiting laccase activity typically oxidize aromatic and/or non-aromatic compounds as substrates. Preferably, aromatic substrates are selected from the group consisting of (substituted) phenols such as methoxyphenolic compounds, polyphenols, aromatic amines and polycyclic aromatic hydrocarbons. More preferably, aromatic substrates are selected from the group consisting of (2,6)-dimethoxyphenol (DMP), guaiacol, (2-methoxyphenol), 4-hydoxy-3,5-dimethoxycinnamic acid (sinapinic acid), 4-hydroxy-3-methoxycinnamic acid (ferulic acid) and 4-methoxybenzyl alcohol. Non-aromatic substrates are preferably selected from non-phenolic substances, in particular aliphatic amines, benzyl alcohols, syringaldazine (SGZ), veratryl alcohol and 2,2'-Azino-bis(3-ethylbenzthiazoline-6-sulfonic acid) (ABTS). A characteristic naturally occurring substrate of the inventive laccase is lignin. However, DMP is a preferred test compound to test for laccase activity of polypeptides of the invention.

Kinetic parameters of polypeptides of the invention (i.e. Kₘ, k_{cat} and k_{cat}/Kₘ values) are by far superior to those of laccases known in the art. E.g., inventive RL5 laccase has a Kₘ of about 5 times less than reported for other laccase enzymes, whereas its k_{cat} values are significantly increased (factor of 40). The k_{cat}/Kₘ ratio of RL5 laccase significantly exceeds published values (as published, e.g., in Solomon et al., 1996, and reference therein; Chefets et al., 1998; Klonowska et al., 2002; Machczynski et al., 2004).

Therefore, polypeptides of the present invention are characterized by a Kₘ typically between 0.20 µM and 35.0 µM, more preferably between 0.30 µM and 28.0 µM, most preferably between 0.40 µM and 2 µM. Polypeptides of the present invention may as well be characterized by k_{cat} values, preferably from 800 min⁻¹ to 80,000 min⁻¹, more preferably from 1,000 min⁻¹ to 60,000 min⁻¹, even more preferably from 10,000 min⁻¹ to 40,000 min⁻¹, most preferably from 20,000 min⁻¹ to 40,000 min⁻¹. Consequently, k_{cat}/Kₘ ratios are calculated for polypeptides of the invention which are preferably from 40 min⁻¹µM⁻¹ to 170,000 min⁻¹µM⁻¹, more preferably from 1,000 min⁻¹µM⁻¹ to 165,000 min⁻¹µM⁻¹, even more preferably from 50,000 min⁻¹µM⁻¹ to 160,000 min⁻¹µm⁻¹, and most preferably from 90,000 min⁻¹µM⁻¹ to 155,000 min⁻¹µM⁻¹.

Polypeptides of the invention are typically active within a broad pH range typically from 3.5 to 9.0. While there will be a pH optimum, inventive polypeptides maintain more than 50%, preferably more than 70 % of its maximum activity over the whole pH range from 3.5 to 9. Although, inventive polypeptides remain typically active between pH 3.5 and 9, it is preferred to provide laccases with optimum activity under acidic conditions (pH < 7), more preferably from pH 4.0 to 6.0, most preferably from pH 5.0-6.0.

Also, polypeptides of the invention are typically stable and active by conserving their tertiary structure within a broad temperature range, preferably from 20°C to 70°C. Typically, increasing activity is observed along with elevated temperatures. Therefore, polypeptides of the present invention show an activity optimum from 20°C to 75°C, more preferably from 40°C to 70°C, even more preferably from 50°C to 65°C, most preferably at around 60°C.

Moreover, inventive polypeptides remain stable and enzymatically active for a comparatively long period of time irrespective of the specific conditions (temperature, pH) given. This holds in particular for the preferred temperature and pH range as disclosed above. Inventive polypeptides typically continue with their catalyzing reaction for at least four hours (e.g. under temperature conditions as high as 60°C). The term "high stability" is intended to encompass inventive polypeptides being enzymatically active at an essentially constant level (without loss of more than 50% of its maximum activity). The term "long period of time" is intended to mean that inventive polypeptides are enzymatically active (at least 50% of its initial activity under the conditions given) for at least one hour, preferably for at least two hours, more preferably for at least 12 hours, even more preferably for at least 24 hours and most preferably for at least 72 hours.

The inventive polypeptides may be isolated from a rumen ecosystem, preferably from bovine rumen, more preferably from New Zealand dairy cow or it may be synthesized either corresponding to a naturally occurring sequence or as a functional non-native sequence, by synthetic methods or, preferably, by recombinant methods well known in the art.

The present invention also encompasses functional fragments and/or functional derivatives of inventive polypeptides exhibiting laccase activity. "Functional", e.g., functional fragment or functional derivative according to the invention, means that the polypeptides exhibit laccase activity, preferably, laccase-like oxidizing activity, particularly oxidizing activity for laccase substrates e.g. DMP or ABTS, or catalyzes polymerisation, depolymerisation, methylation and/or demethylation reactions. Several methods for measuring laccase activity are known by a skilled person. Some of them are described in the Examples herein (e.g., enzyme assays using marked substrates, substrate analysis by chromatographic methods, as HPLC or TLC, for separating enzyme and substrate and spectrophotometric assays for measuring esterolytic activity) (see e.g., Maniatis et al. (2001) Molecular Cloning: A laboratory manual, Cold Spring Harbor Laboratory Press, Cold Spring Habor, NY). Polypeptides according to the invention are defined as functional, if they exhibit at least 20%, preferably at least 30% and more preferably at least 50% of the activity of the laccase shown in figure 9 under identical conditions. The term "activity" refers to the enzymatic activity (e.g. expressed in units, log. its oxidizing activity), which may be compared under the same conditions for the naturally occurring laccase according to figure 9 and any fragment or derivative thereof.

The term "fragment of a polypeptide" according to the invention is intended to encompass a portion of an inventive polypeptide, in particular, of the amino sequence of figure 9 of at least about 60 contiguous amino acids, preferably of at least about 80 contiguous amino acids, more preferably of at least about 100 contiguous amino acids, even more preferably of at least about 150 contiguous amino acids, most preferably of at least about 200 contiguous amino acids or longer in length. Functional fragments of polypeptides that retain laccase activity (at least 20%, more preferably at least 50% of the maximum activity of RL5 laccase) are particularly useful.

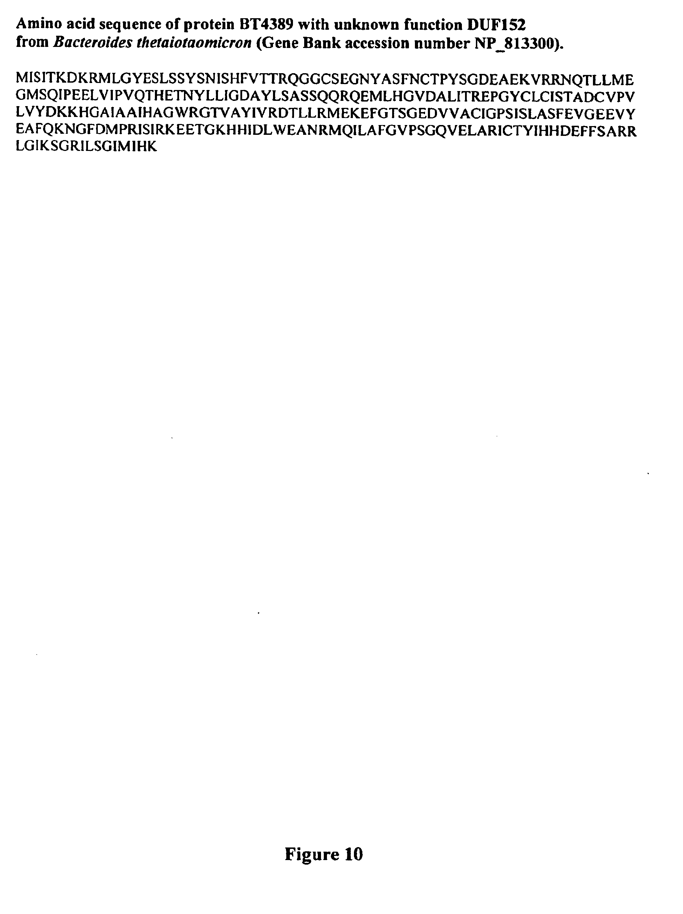

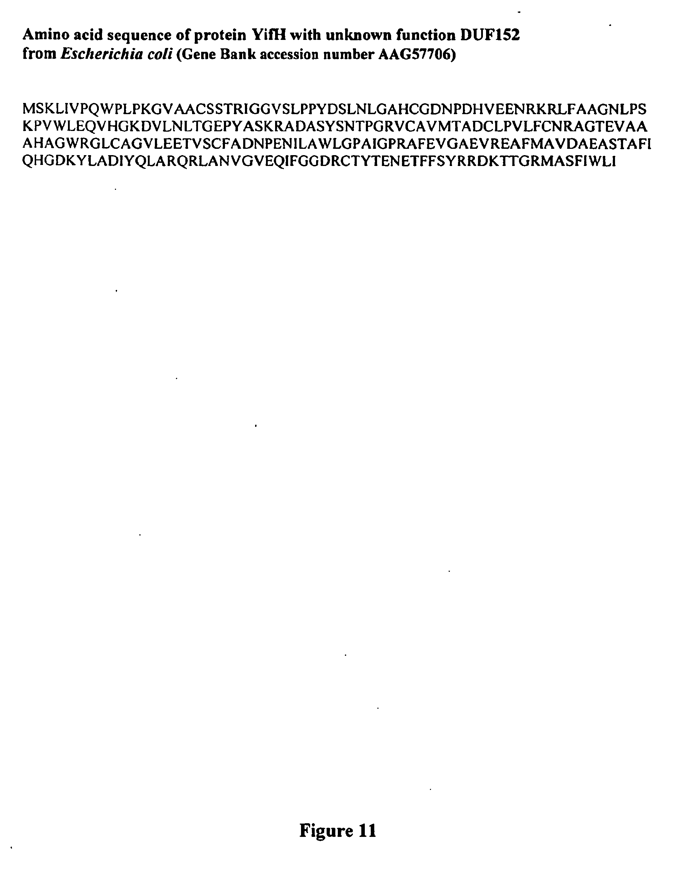

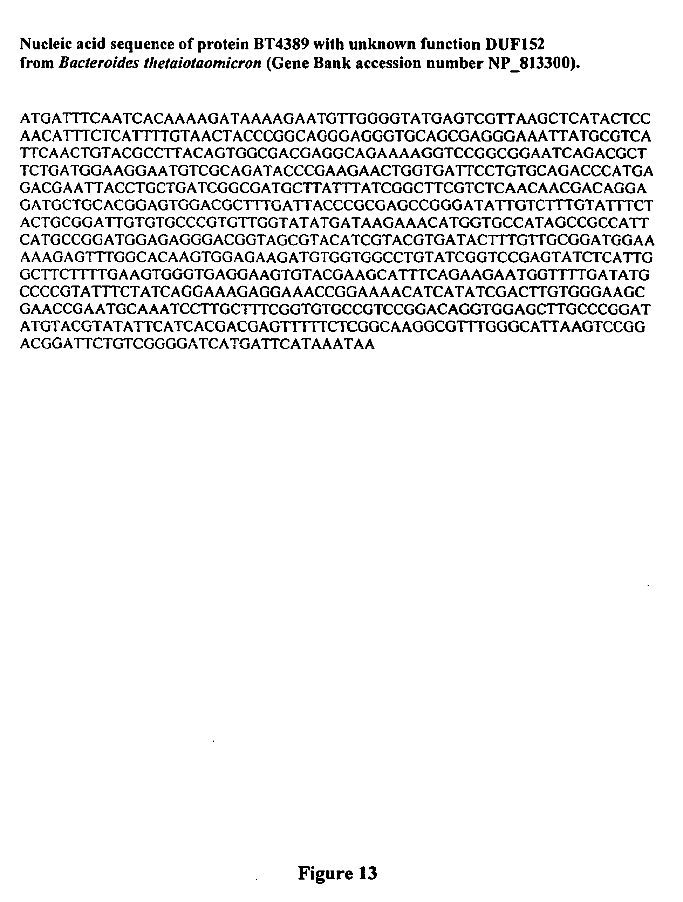

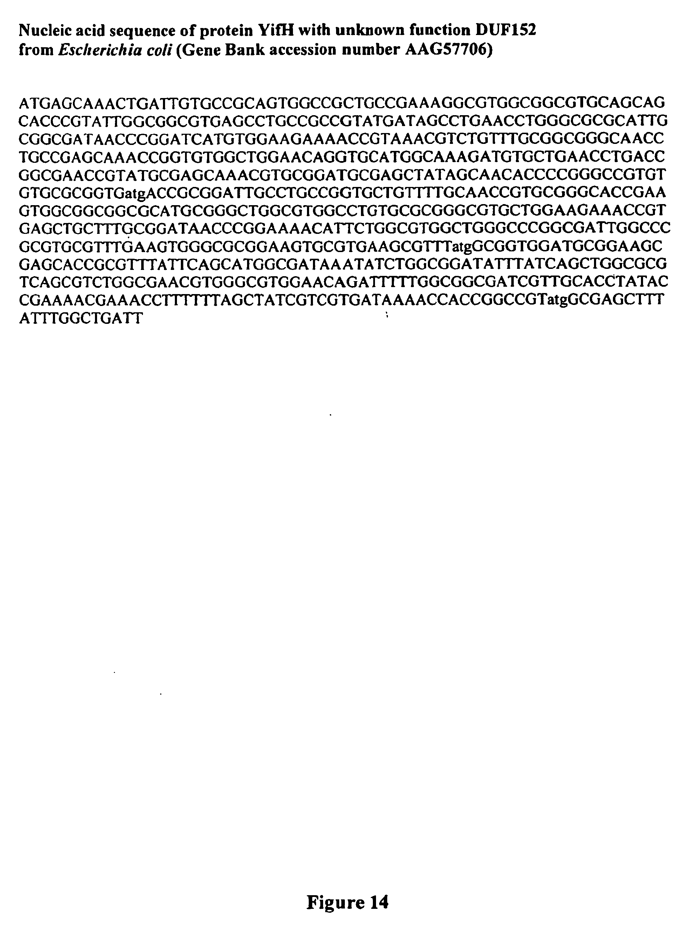

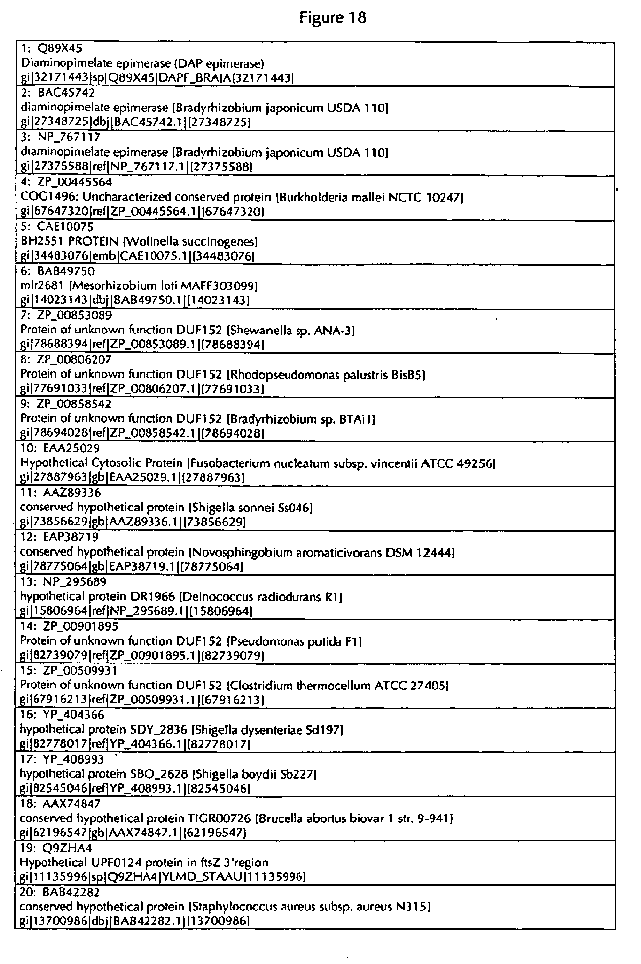

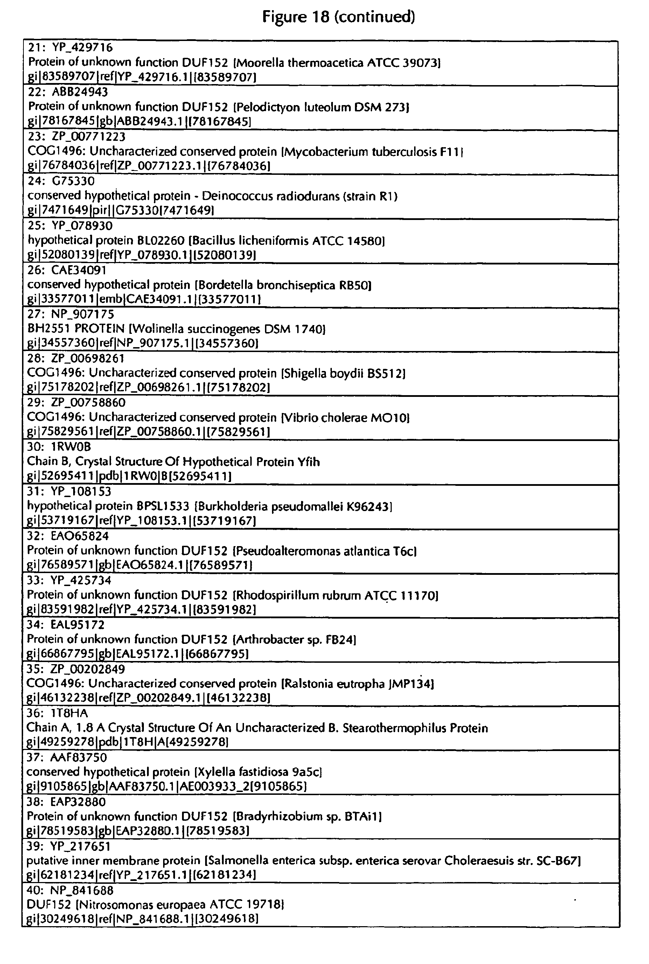

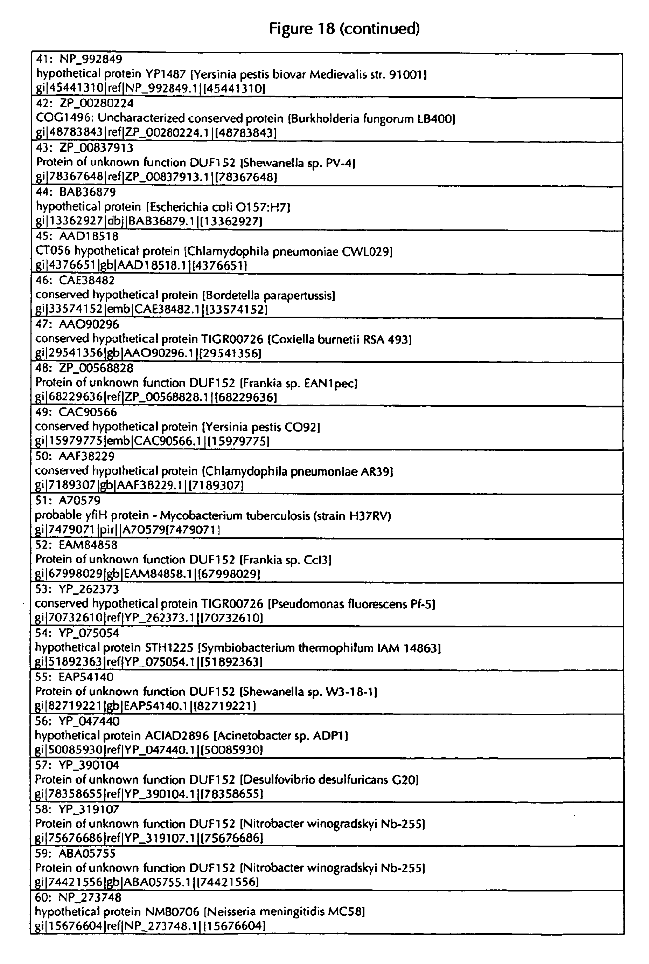

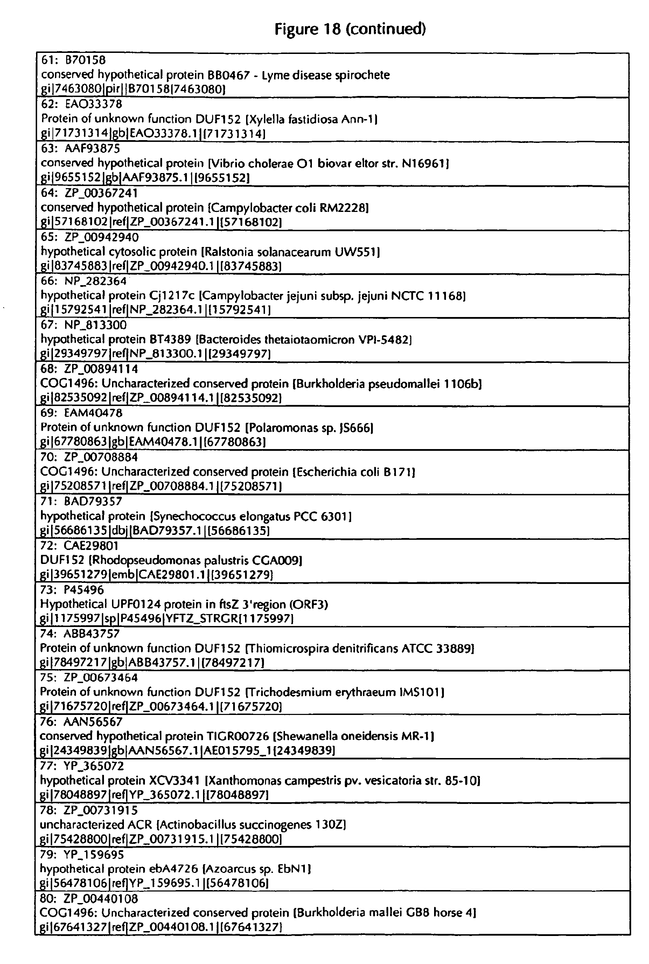

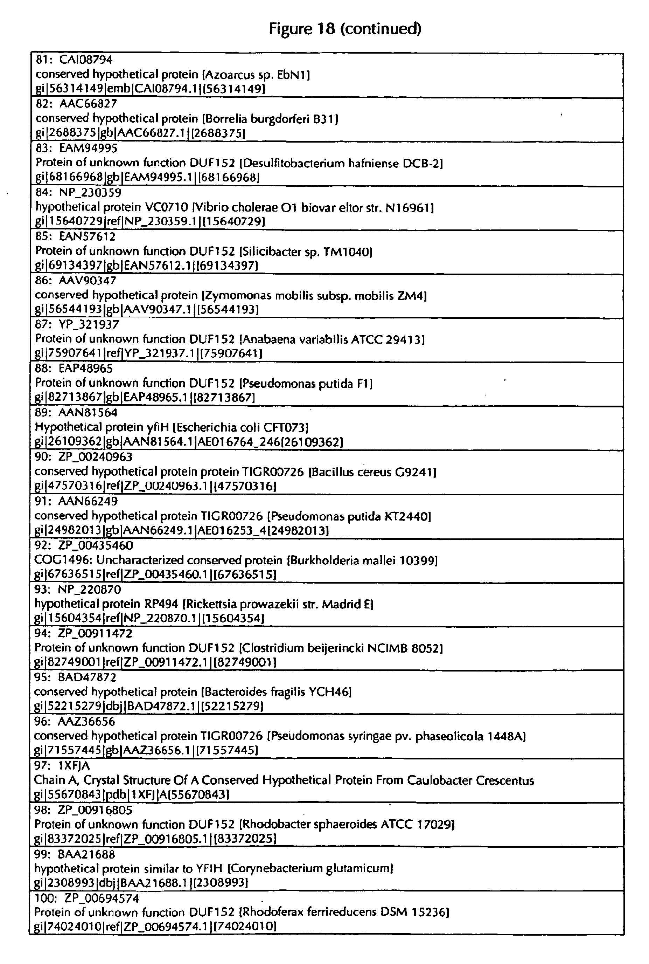

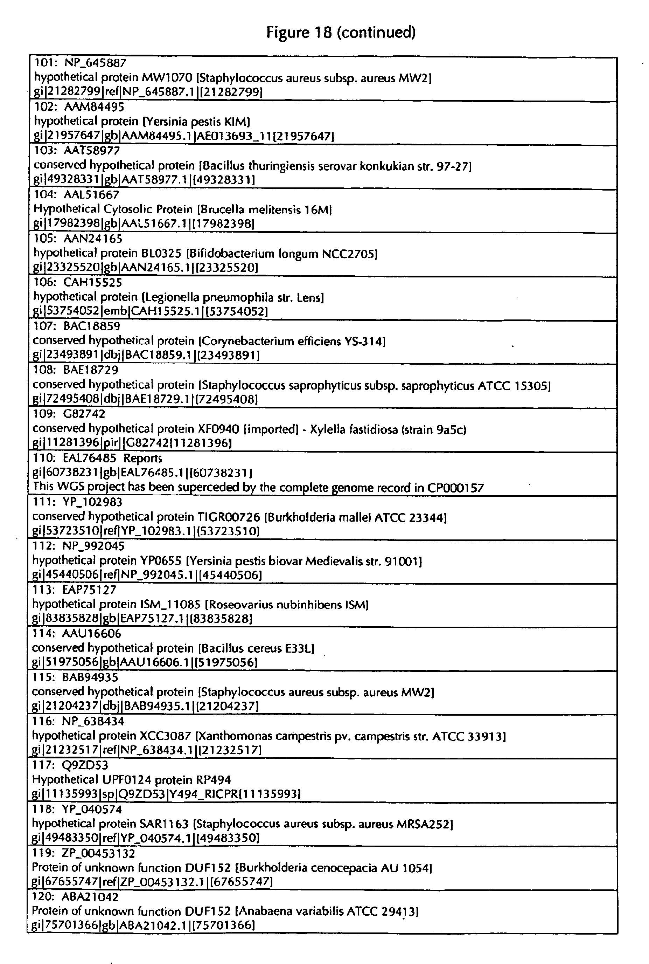

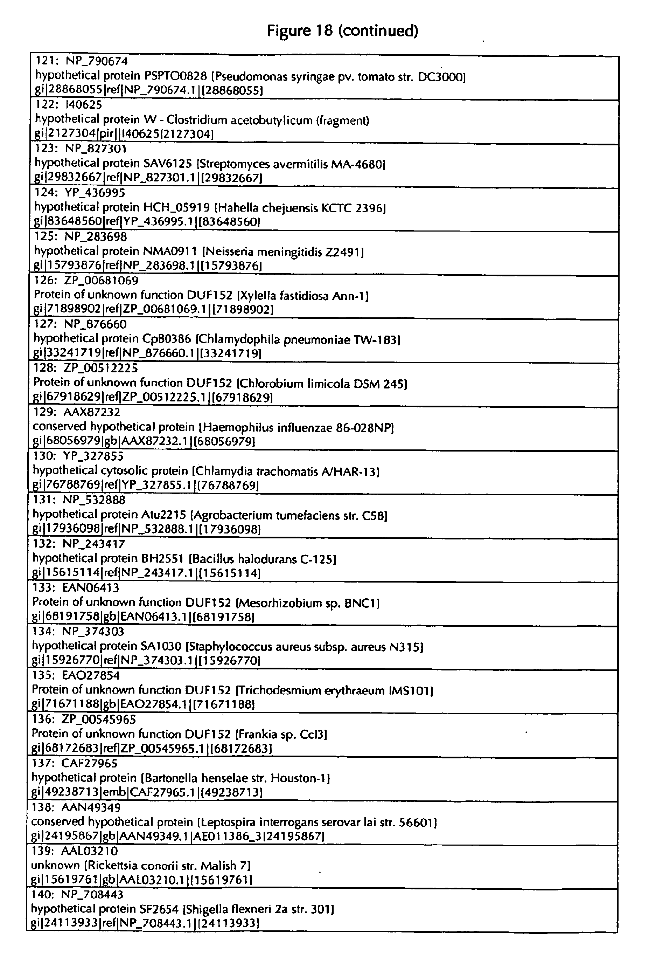

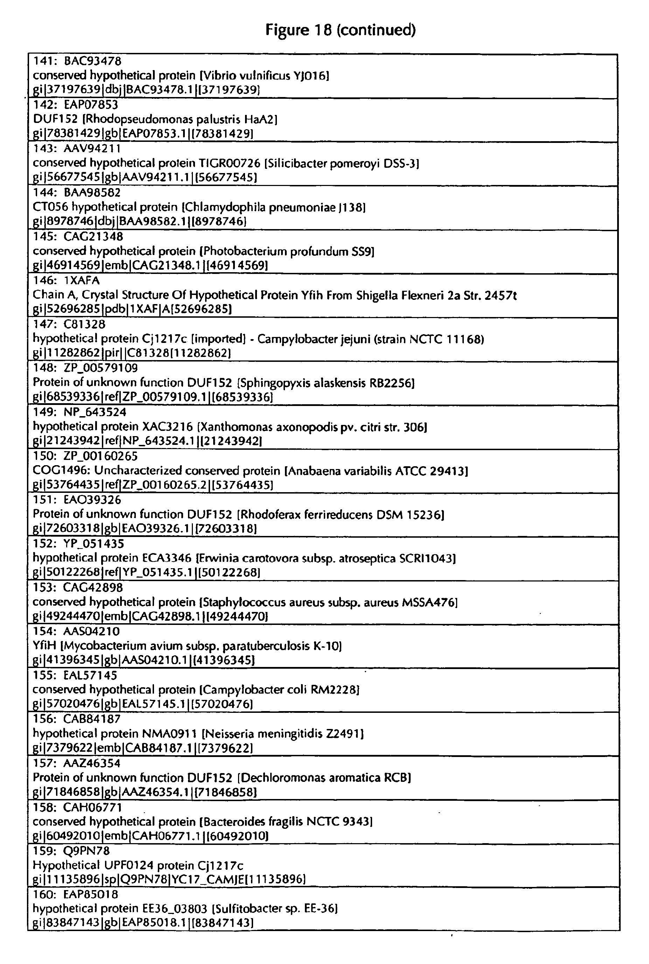

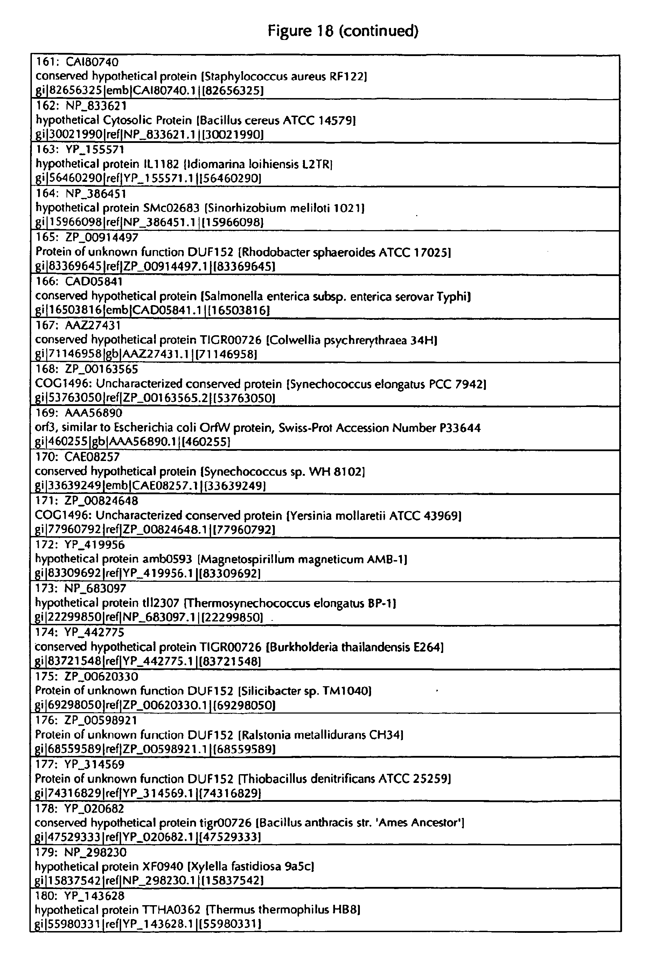

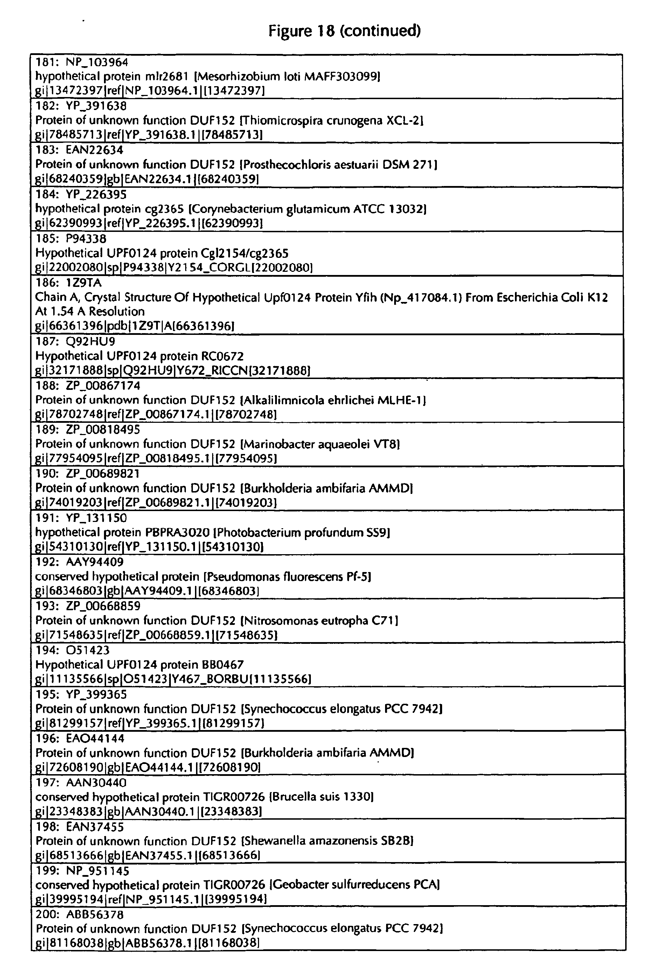

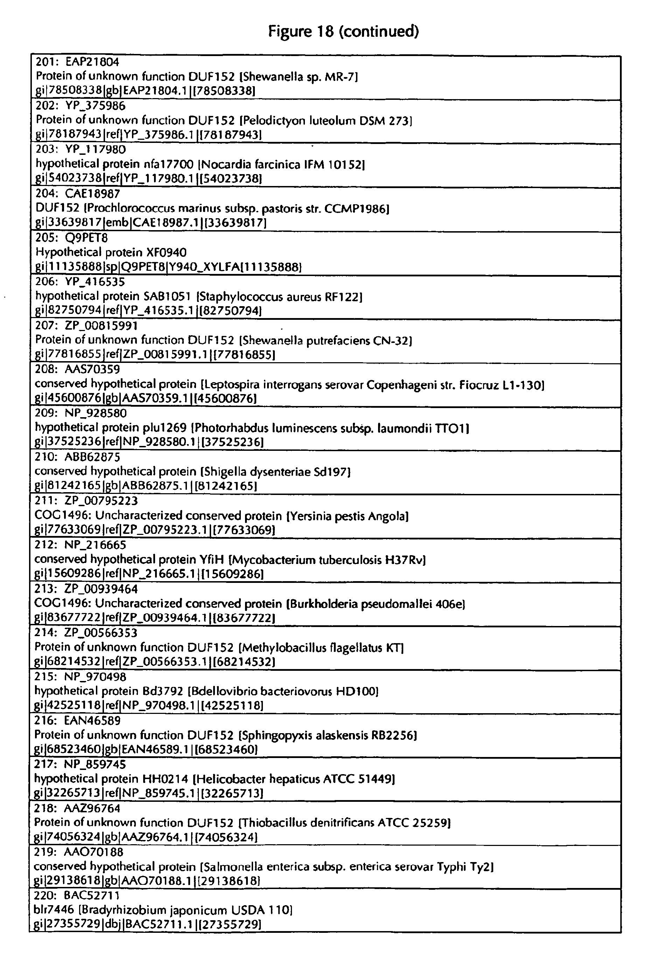

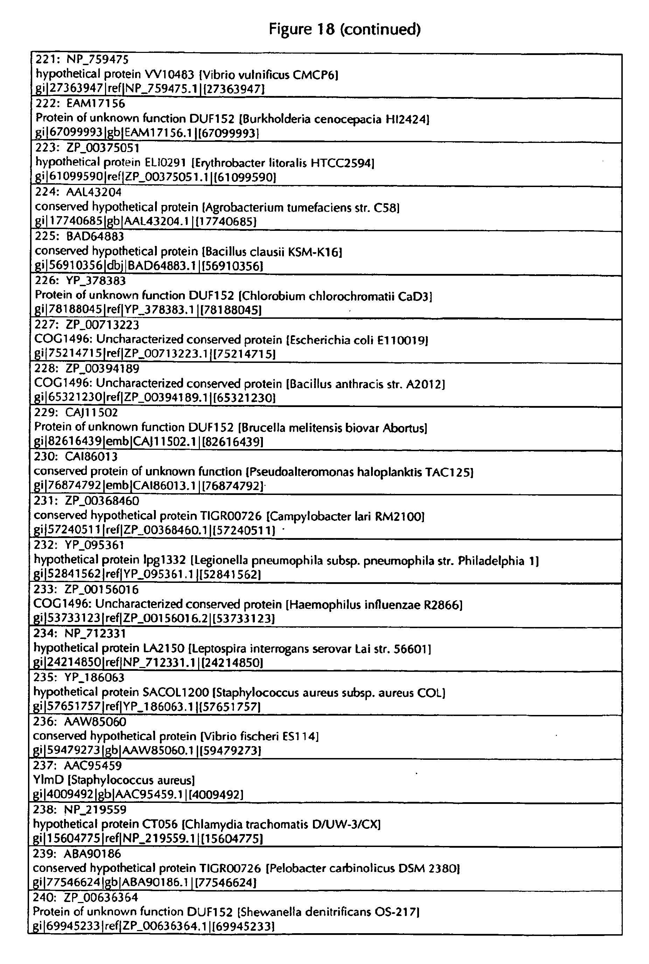

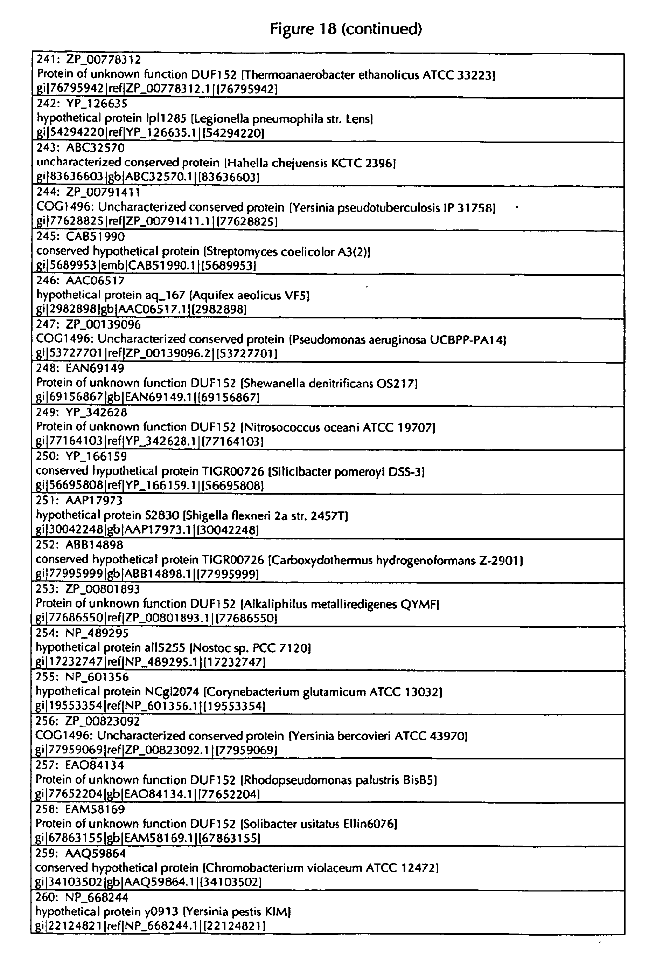

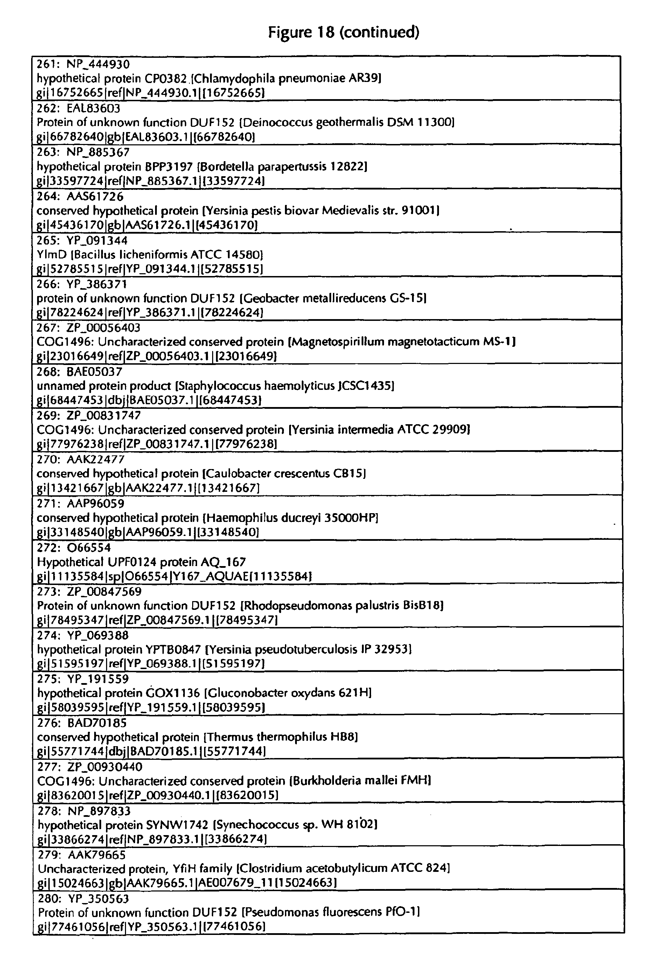

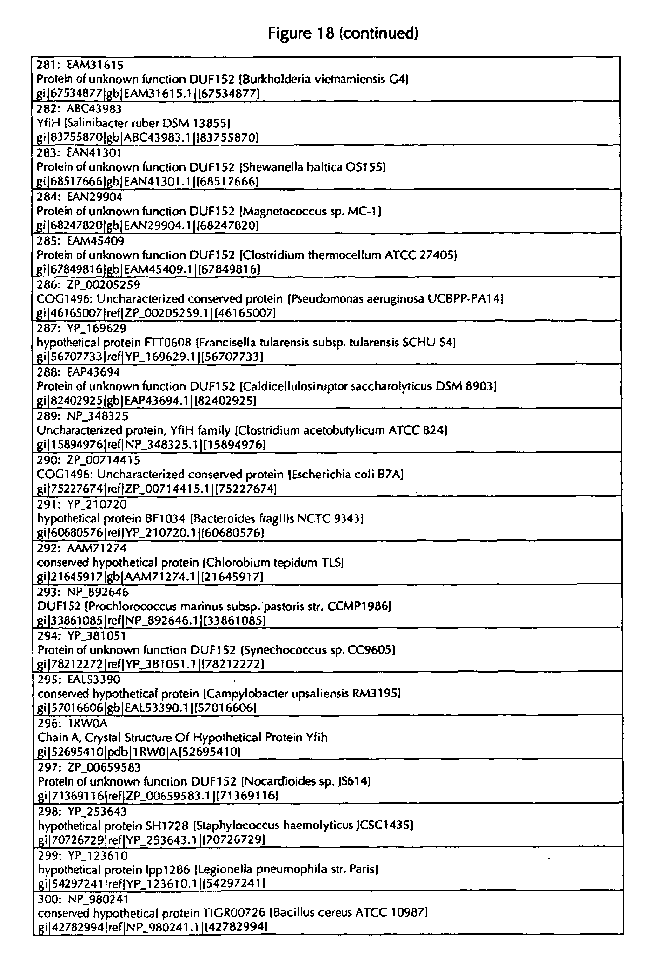

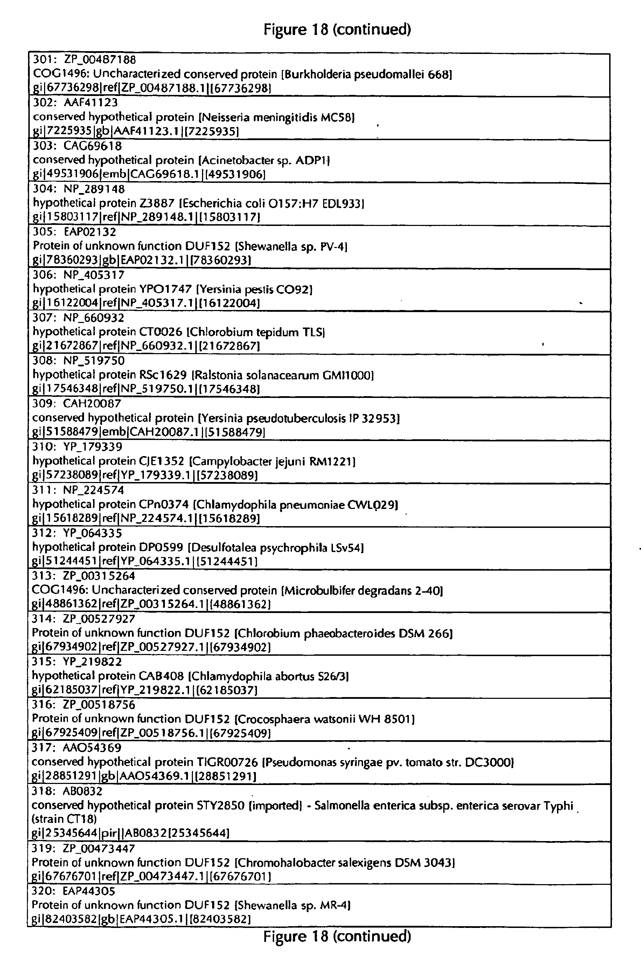

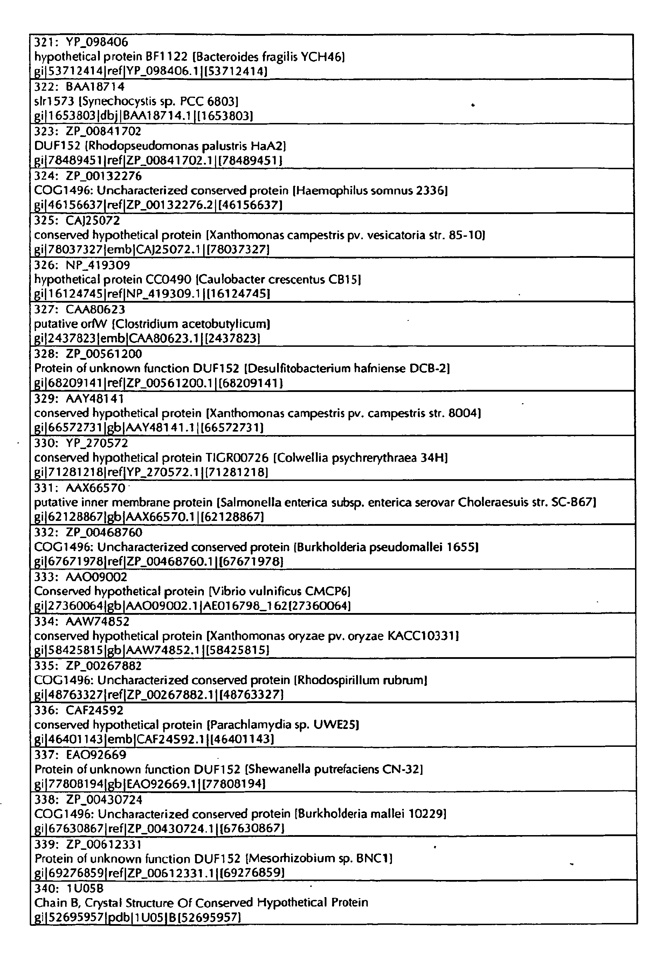

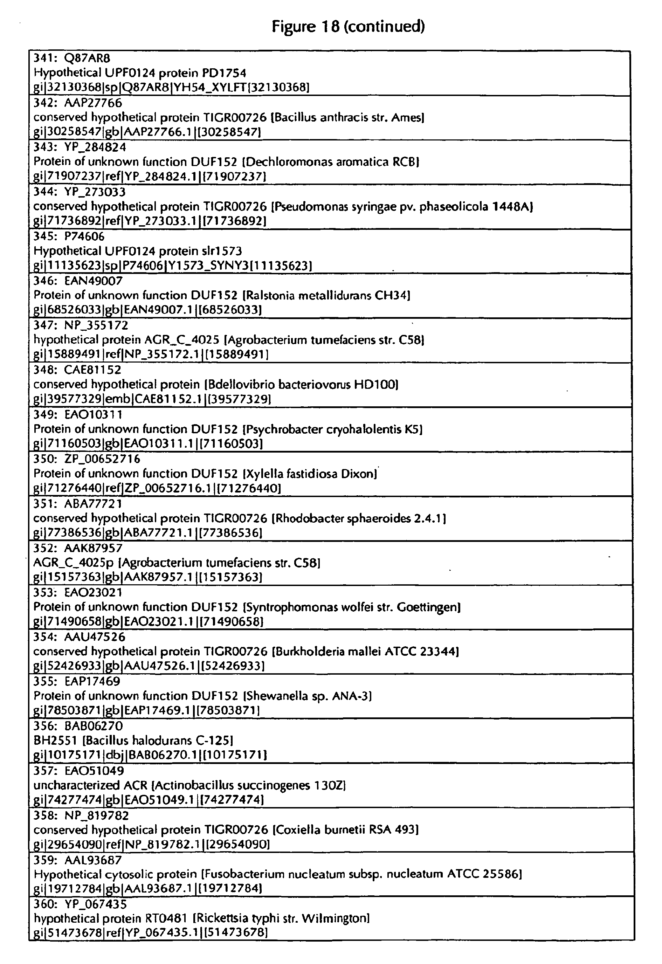

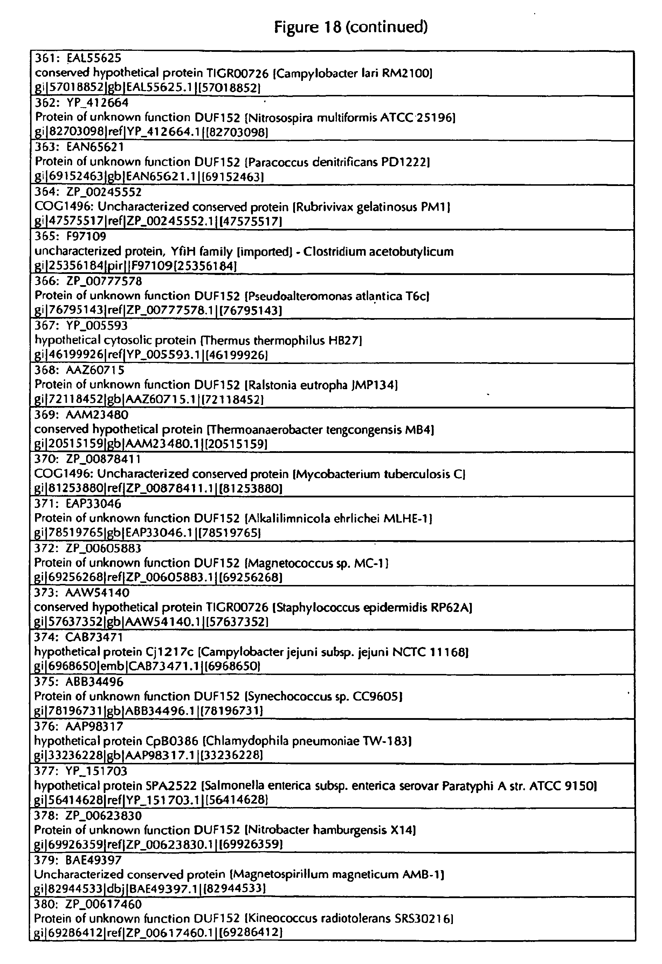

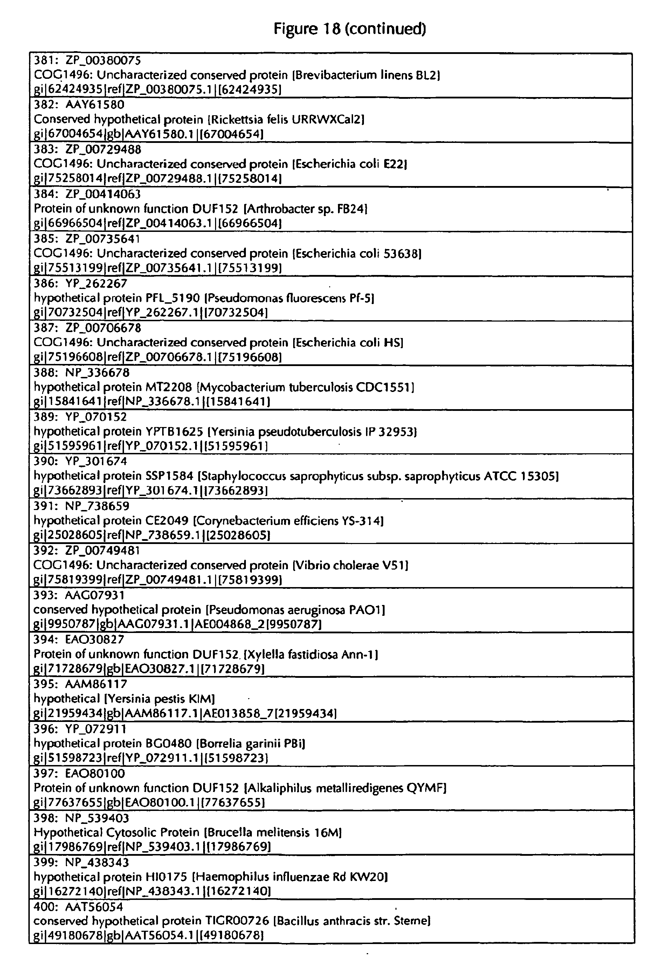

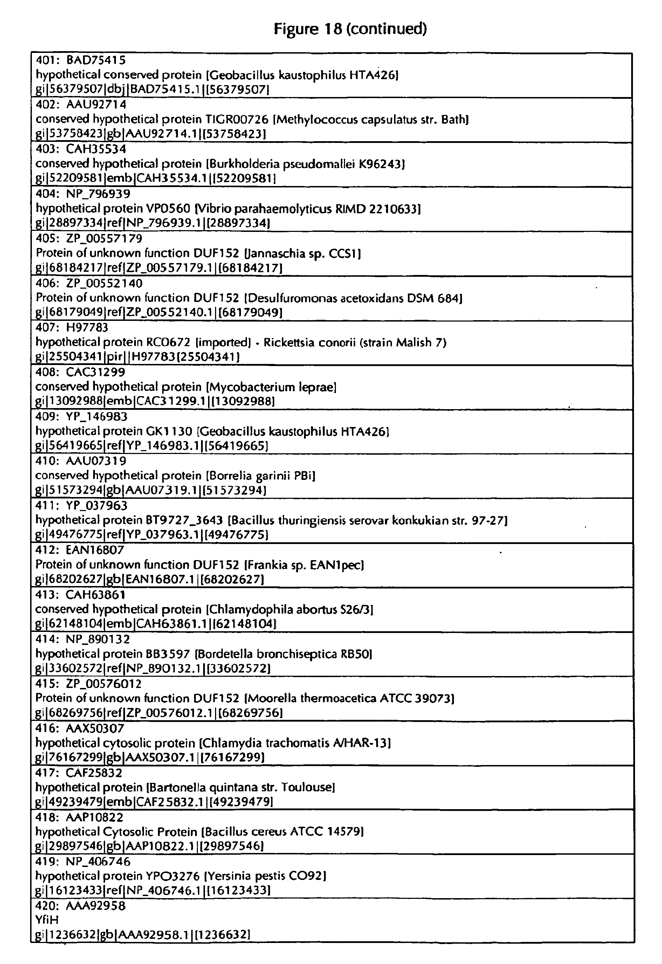

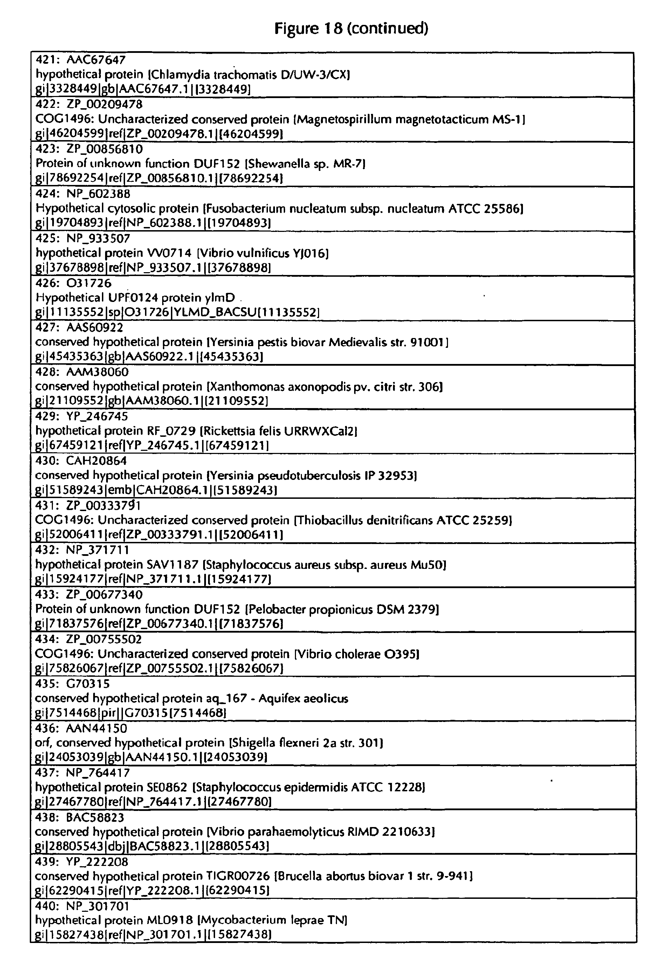

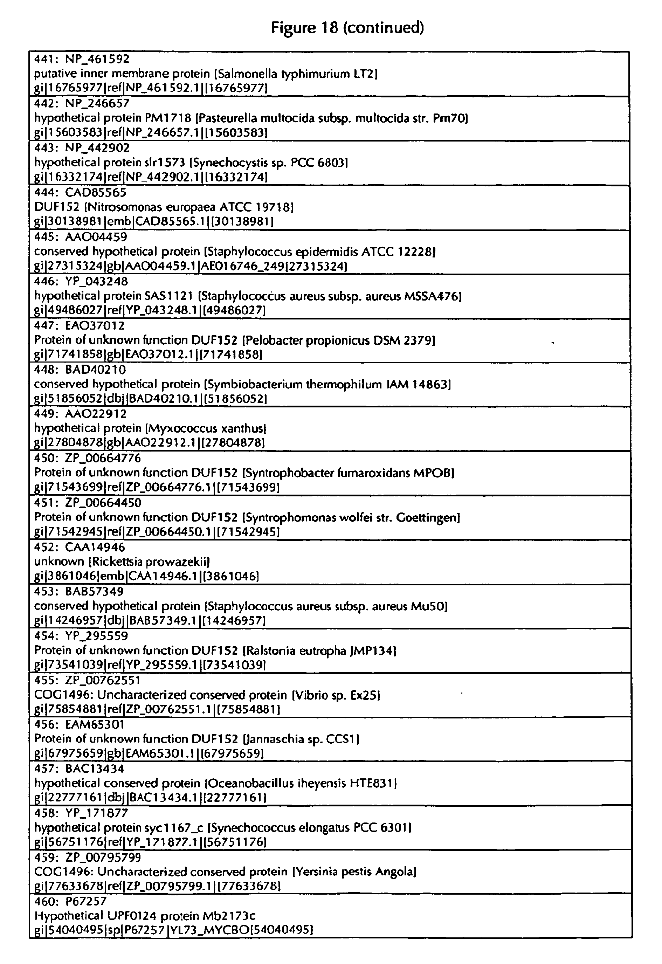

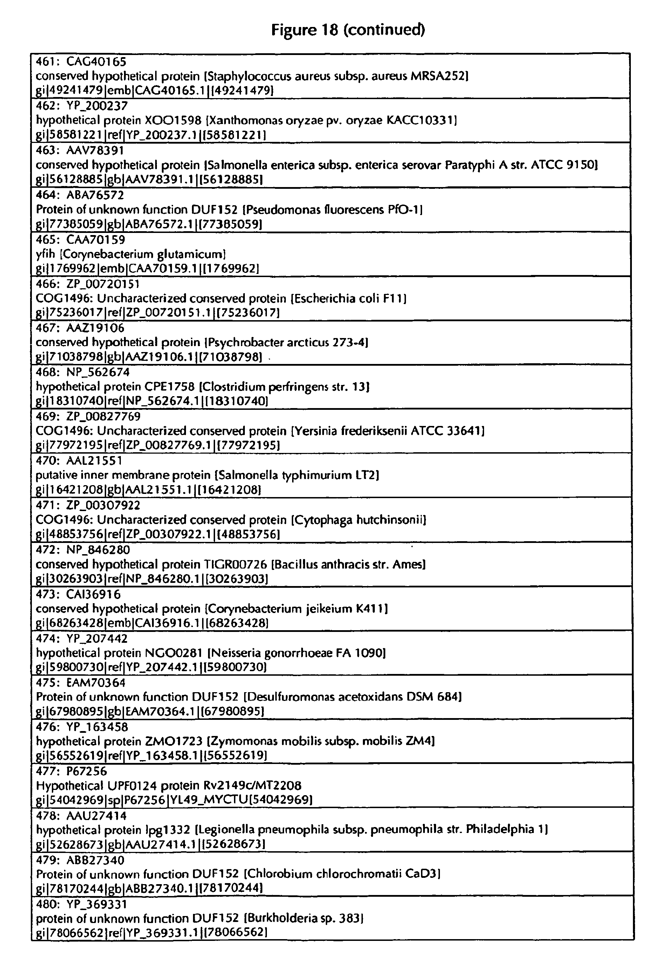

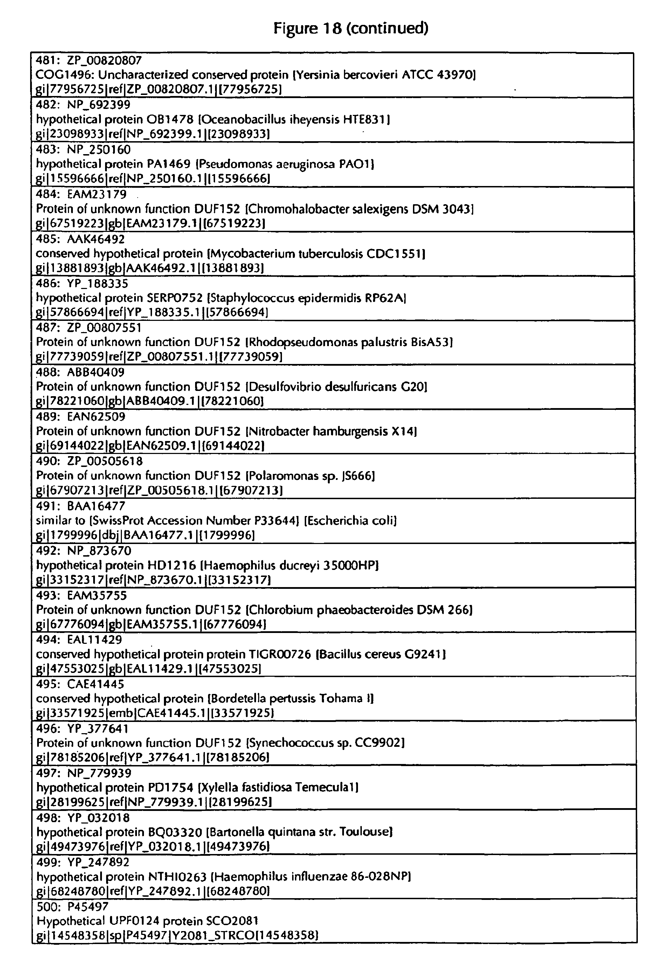

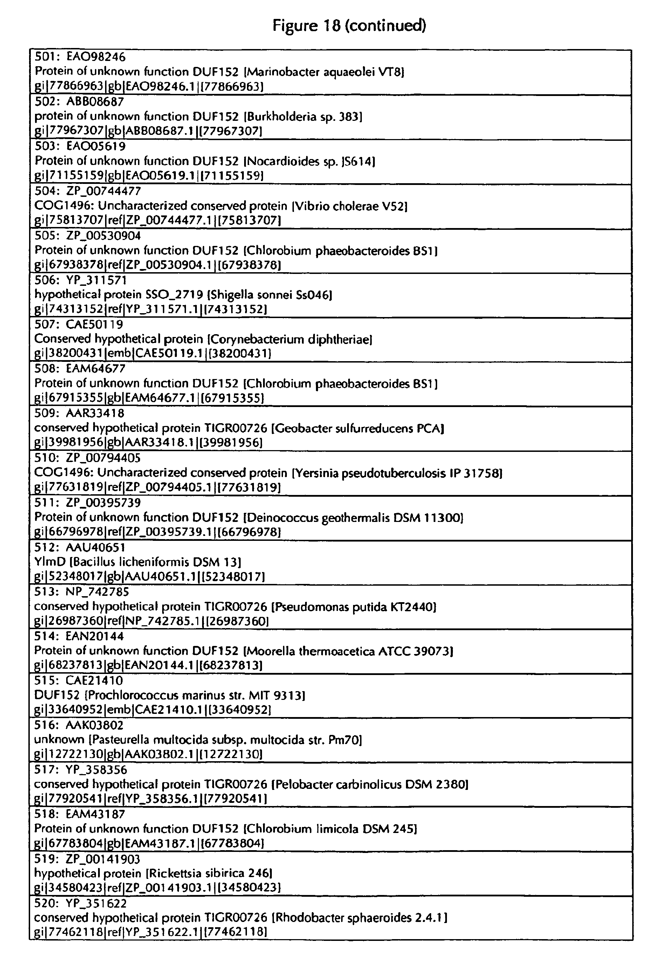

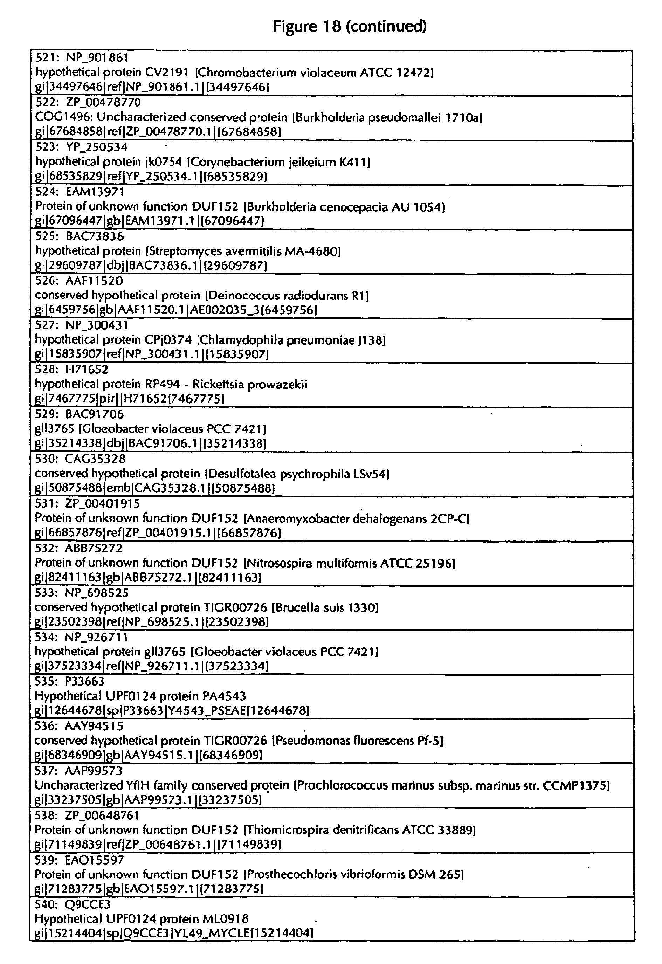

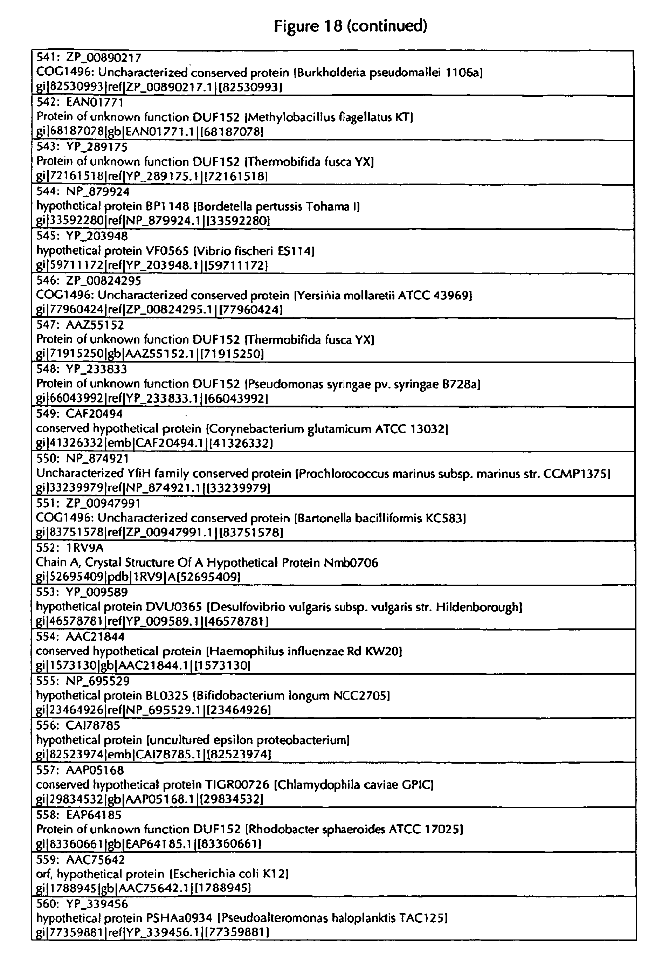

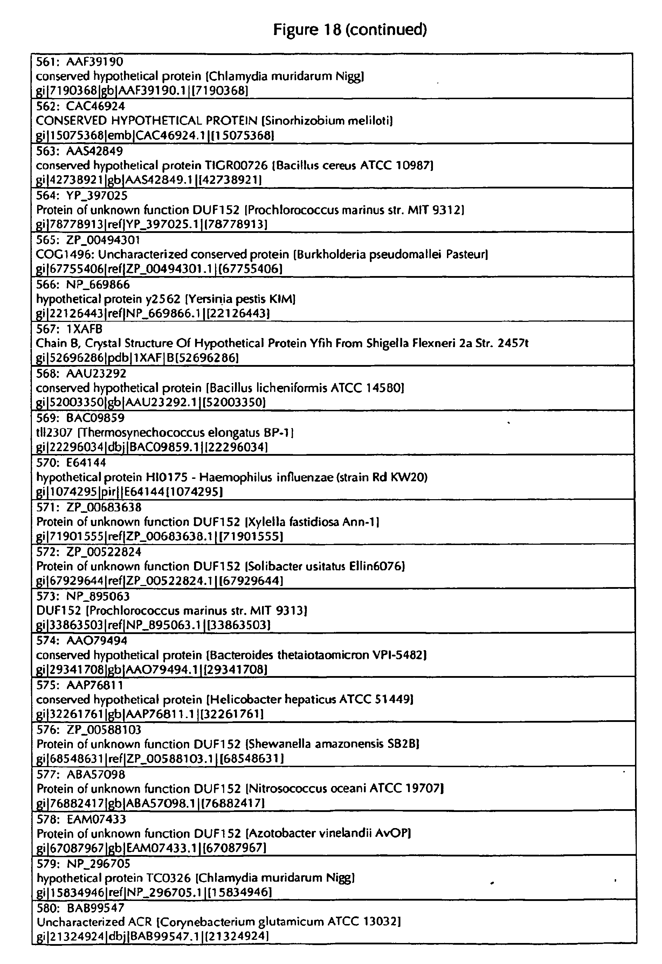

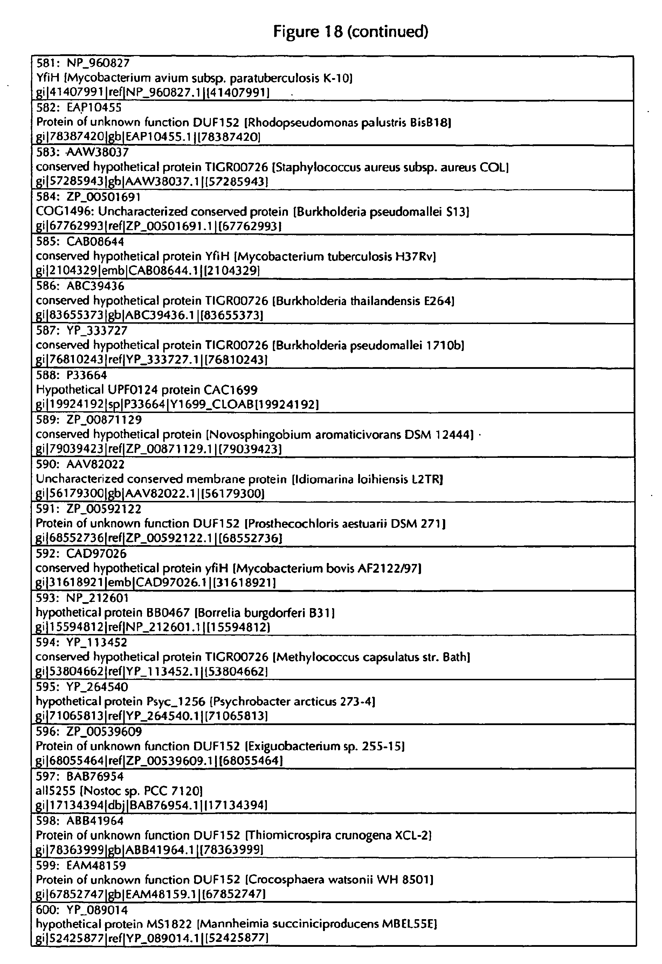

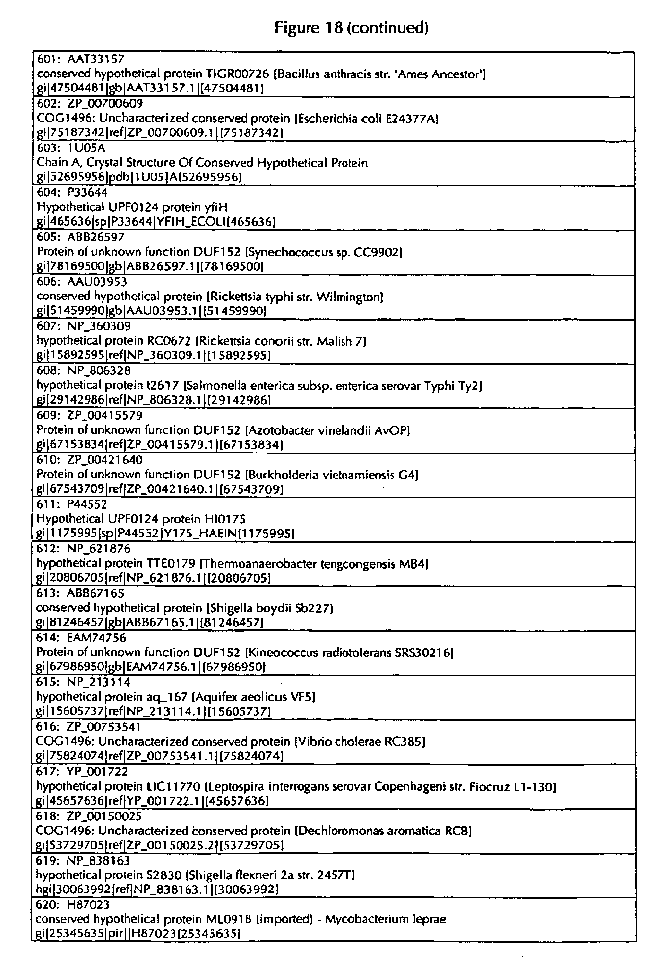

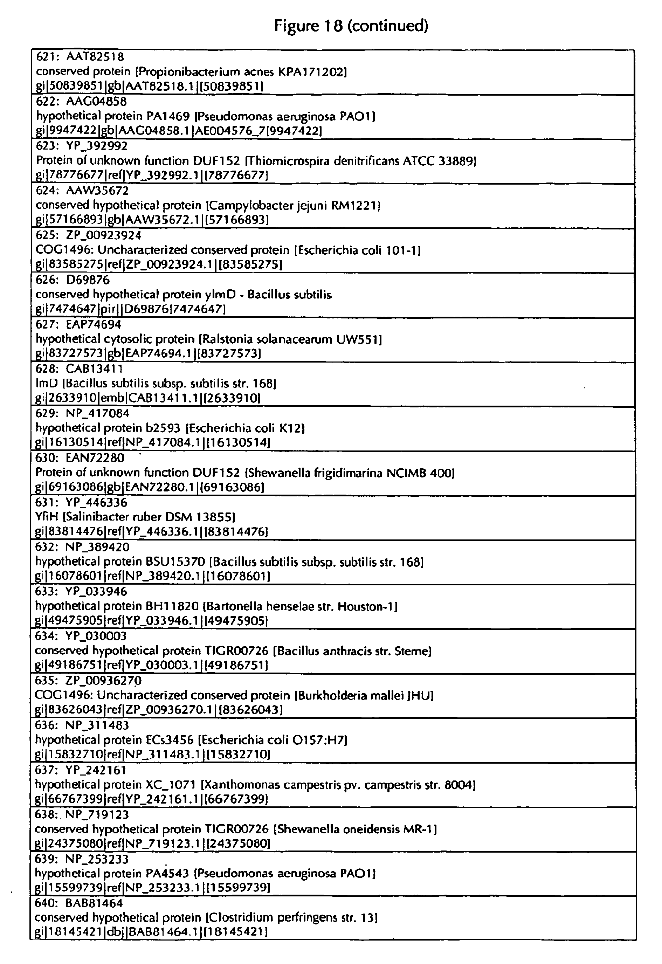

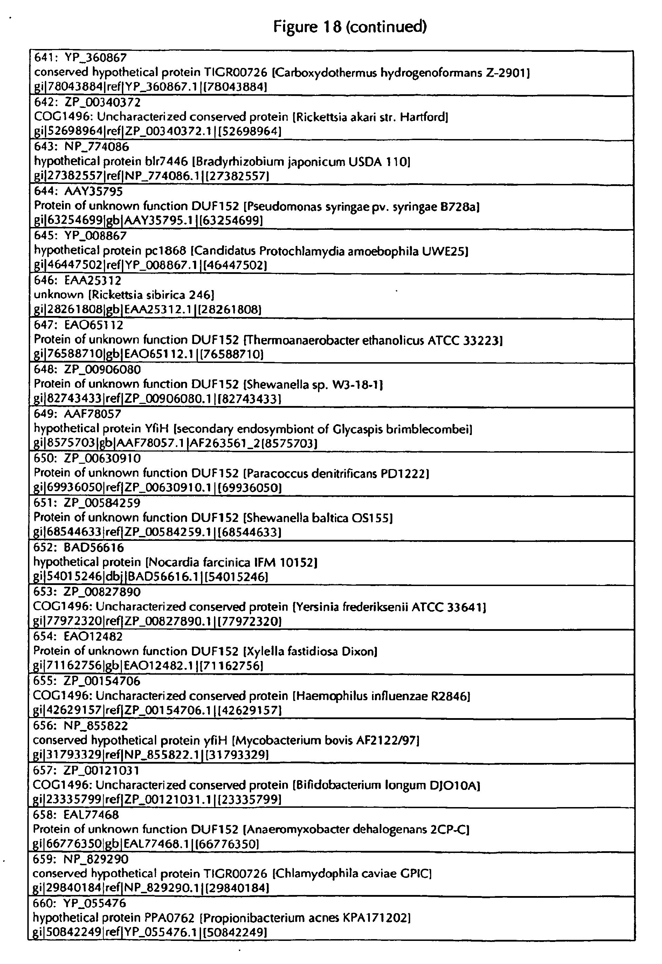

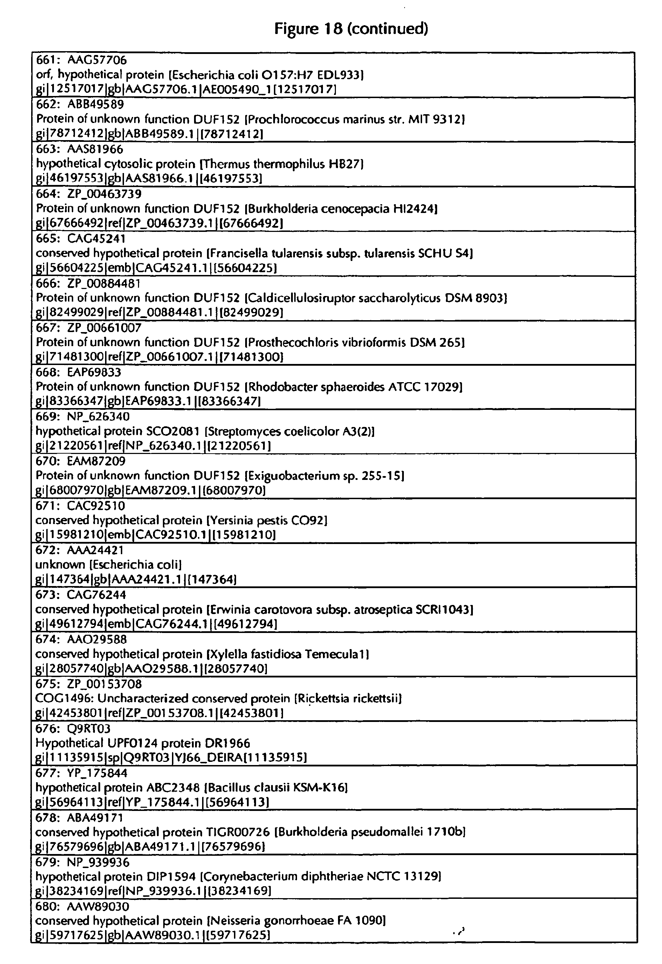

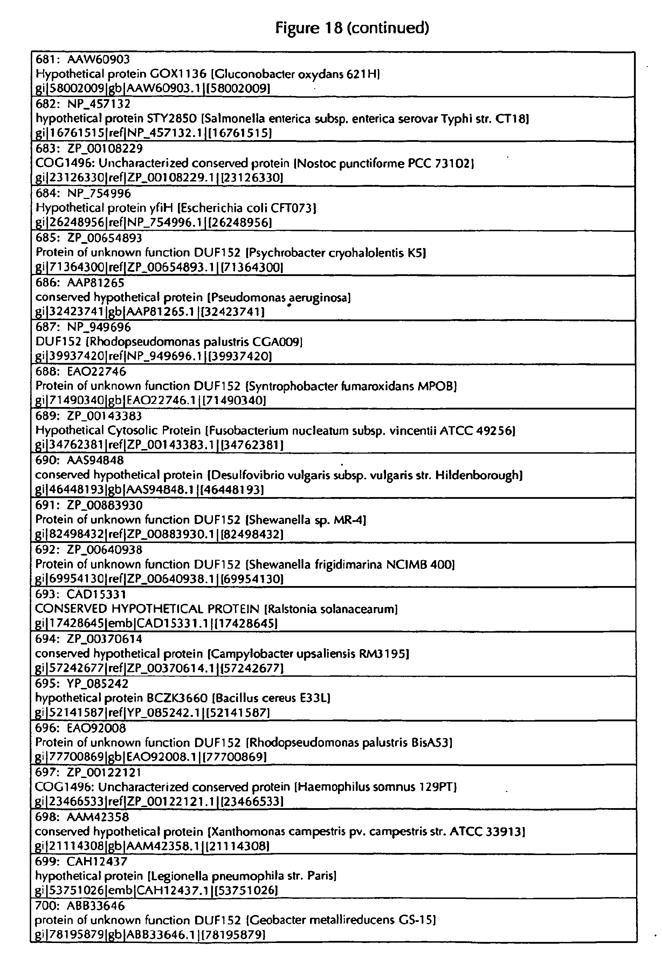

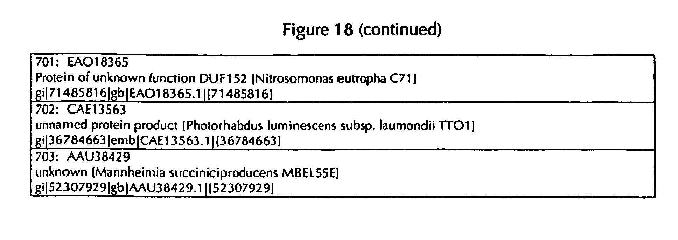

A "derivative of a polypeptide" according to the invention is intended to indicate a polypeptide which is derived from (native) polypeptides of the invention, in particular according to figure 9, by substitution of one or more amino acids at one site or two or more different sites of the (native) amino acid sequence, as described herein, deletion of one or more amino acids at either or both ends of the (native) amino acid sequence or at one or more sites within the (native) amino acid sequence, and/or insertion of one or more amino acids at one or more sites of the (native) amino acid sequence retaining its characteristic activity, particularly its laccase activity. Such derivatized inventive polypeptides can possess altered properties as compared to the native laccases, e.g. according to figures 9 to 14 or as given in figure 18, which may be advantageous for certain applications (e.g. altered pH optimum, increased temperature stability, modified kinetic properties etc.).

A derivative of a polypeptide according to the invention means a polypeptide, which has substantial identity with the natural amino acid sequence of an inventive polypeptide exhibiting laccase activity, as disclosed e.g. in figure 9. A derivative with an amino acid sequence which has at least 60% sequence identity, preferably at least 75% sequence identity, even more preferably at least 80%, yet more preferably 90% sequence identity and most preferably at least 95% sequence identity with the native laccase sequences, in particular RL5, are particularly preferred. In its most preferred embodiment, a derivative deviates by 5 modifications (substitution, insertion and/or deletion) per 100 amino acids from its naturally occuring basic protein sequence.

To determine the percent identity of two amino acid sequences, the sequences can be aligned for optimal comparison purposes (e. g., gaps can be introduced in the sequence of a first amino acid sequence). The amino acids at corresponding amino acid positions can then be compared. When a position in the first sequence is occupied by the same amino acid as the corresponding position in the second sequence, then the molecules are identical at that position. The percent identity between the two sequences is a function of the number of identical positions shared by the sequences. The determination of percent identity of two sequences can be accomplished using a mathematical algorithm. A preferred, non-limiting example of a mathematical algorithm utilized for the comparison of two sequences is the algorithm of Karlin et al. (1993), PNAS USA, 90:5873-5877. Such an algorithm is incorporated into the NBLAST program, which can be used to identify sequences having the desired identity to the amino acid sequence of the invention. To obtain gapped alignments for comparison purposes, Gapped BLAST can be utilized as described in Altschul et al. (1997), Nucleic Acids Res, 25:3389-3402. When utilizing BLAST and Gapped BLAST programs, the default parameters of the respective programs (e. g., NBLAST) can be used. The described method of determination of the percent identity of two amino acid sequences can be applied correspondingly to nucleic acid sequences.

Methods for the production of functional fragments or functional derivatives of the inventive polypeptides are well known and can be carried out following standard methods, which are well known by a person skilled in the art (see e.g., Maniatis et al. (2001) *supra*)*.* In general, the preparation of functional derivatives of inventive polypeptides can be achieved by modifying a DNA sequence which encode a native polypeptide of the invention, transformation of that DNA sequence into a suitable host and expression of the modified DNA sequence to form functional derivative of the polypeptide presumed that the modification of the DNA does not disturb the characteristic activity, particularly laccase activity. The modification of the DNA sequence may be generated by either random mutagenesis techniques, such as those techniques employing chemical mutagens, or by site-specific mutagenesis employing oligonucleotides. One of the most widely employed techniques for altering a DNA sequence is by way of oligonucleotide-directed site-specific mutagenesis (see Comack B, CURRENT PROTOCOLS IN MOLECULAR BIOLOGY, 8.01-8.5.9, Ausubel F, et al., eds. 1991). In this technique an oligonucleotide, whose sequence contains a mutation of interest, is synthesized as described supra. This oligonucleotide is then hybridized to a template containing the wild-type nucleic acid sequence. In a preferred embodiment of this technique, the template is a single-stranded template. Particularly preferred are plasmids, which contain regions such as the f1 intergenic region. This region allows the generation of single-stranded templates when a helper phage is added to the culture harboring the phagemid. After annealing of the oligonucleotide to the template, a DNA-dependent DNA polymerase is used to synthesize the second strand from the oligonucleotide, complementary to the template DNA. The resulting product is a heteroduplex molecule containing a mismatch due to the mutation in the oligonucleotide. After DNA replication by the host cell a mixture of two types of plasmid are present, the wild-type and the newly constructed mutant. The isolation can be carried out using standard methods, as e.g. separation from cell or culture medium by centrifugation, filtration or chromatography and precipitation procedures (see, e.g., Maniatis et al. (2001) *supra).* This technique permits the introduction of convenient restriction sites, such that the coding nucleic acid sequence may be placed immediately adjacent to whichever transcriptional or translational regulatory elements are employed by the practitioner. The construction protocols utilized for *E. coli* can be followed to construct analogous vectors for other organisms, merely by substituting, if necessary, the appropriate regulatory elements using techniques well known to skilled artisans.

Polypeptides of the invention as well as its functional fragments and derivatives can also be fused to at least one second moiety. Preferably, the second or further moiety/moieties does not occur in the naturally occurring laccase. The at least one second moiety can be an amino acid, oligopeptide or polypeptide and can be linked to polypeptides of the invention at any suitable position, for example, the N-terminus, the C-terminus or internally via e.g. amide, ester or ether bonds. Linker sequences can be used to fuse inventive polypeptides with the at least one further moiety/moieties. According to one embodiment of the invention, the linker sequences preferably form a flexible sequence of 5 to 50 residues, more preferably 5 to 15 residues. In a preferred embodiment the linker sequence contains at least 20%, more preferably at least 40% and even more preferably at least 50% Gly residues. Appropriate linker sequences can be easily selected and prepared by a person skilled in the art. Additional moieties may be linked to an inventive sequence, if desired. If an inventive polypeptide is produced as a fusion protein, the fusion partner (e.g., HA, HSV-Tag, in particular His6) can be used to facilitate purification and/or isolation. If desired, the fusion partner can then be removed from the polypeptide (e.g., by proteolytic cleavage or other methods known in the art) at the end of the production process.

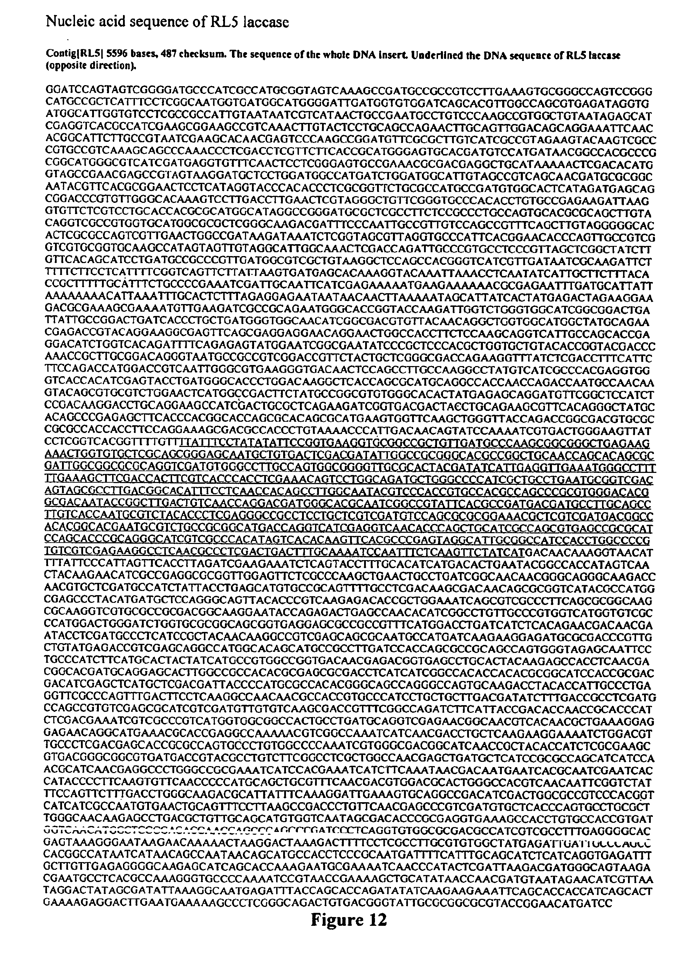

The invention provides in another embodiment a nucleic acid encoding the inventive polypeptide encoding RL5 or a functional fragment or functional derivative thereof, as described above, optionally fused to at least one second moiety. Preferably, the nucleic acid comprises or consists of the nucleic acid sequence of Figure 12.

Preferably, an inventive nucleic acid is an isolated nucleic acid. An "isolated" nucleic acid molecule or nucleotide sequence is intended to mean a nucleic acid molecule or nucleotide sequence which is not flanked by nucleotide sequences in its genomic form and/or has been completely or partially purified in order to isolate the nucleic acid (e.g., as occurring in a DNA or RNA library). For example, an isolated nucleic acid of the invention may be substantially isolated from its complex cellular milieu in which it naturally occurs. In some instances, the isolated material will be component of a composition (for example, a crude extract containing other substances), buffer system or reagent mix. Alternatively, the material may be essentially purified to homogeneity, for example as determined by PAGE or column chromatography, such as HPLC.

The nucleic acid of the invention can be DNA, for example cDNA, or RNA, for example, mRNA. The nucleic acid molecules can be double-stranded or single-stranded; single stranded RNA or DNA can be either the coding (sense) strand or the non-coding (antisense) strand. If desired, the nucleotide sequence of the isolated nucleic acid can include additional non-coding sequences such as non-coding 3'- and 5'-sequences (including regulatory sequences, for example). All nucleic acid sequences, unless otherwise designated, are written in the direction from the 5'-end to the 3'-end. The nucleic acids of the invention can be fused to a nucleic acid sequence encoding, for example, a marker sequence or a nucleotide sequence which encodes a polypeptide to assist, e.g., in isolation or purification of the polypeptide. Representative sequences include, but are not limited to those, which encode a glutathione-S-transferase (GST) fusion protein, a poly-histidine (e. g, His6), hemagglutinin, HSV-Tag, for example.

The present invention also encompasses fragments of inventive nucleic acids, whereby said "fragments" are intended to encompass a portion of a nucleotide sequence described the length of which is from at least 20 nucleotides, more preferably at least 50 nucleotides, more preferably at least about 60 nucleotides, more preferably at least about 120 nucleotides, most preferably at least about 180 nucleotides. In particular, short nucleic acid fragments (preferably from 20 to 60 nucleotides) according to the invention are useful as probes and/or as primers. Particularly preferred primers and probes selectively hybridize to the nucleic acid molecule encoding the polypeptides described herein. A "primer" is a nucleic acid fragment, which functions as an initiating substrate for enzymatic or synthetic elongation. A "probe" is a nucleic acid sequence, which hybridizes with an inventive nucleic acid sequence, a fragment or a complementary nucleic acid sequence thereof. These primers or probes may be useful to identify other naturally occurring laccases from other ecosystems.

Hybridization can be used herein to analyze whether a given fragment or gene corresponds to the inventive laccase and thus falls within the scope of the present invention. Hybridization describes a process in which a strand of nucleic acid joins with a complementary strand through base pairing. The conditions employed in the hybridization of two non-identical, but very similar, complementary nucleic acids varies with the degree of complementarity of the two strands and the length of the strands. Such conditions and hybridisation techniques are well known by a person skilled in the art and can be carried out following standard hybridization assays (see e.g., Maniatis et al. (2001) *supra).* Consequently, all nucleic acid sequences which hybridize to the inventive nucleic acid under stringent conditions or fragments thereof are encompassed by the invention.

A skilled person will recognize that polypeptides of the invention (or fragments or derivatives) can be encoded by a multitude of nucleic acid sequences due to degeneracy of the amino acid code. All of these nucleic acids are encompassed by the present invention as well.

The production and isolation of nucleic acids of the invention (see Figures 12 to 14) and their fragments can be carried out following standard methods which are well known to a skilled person (see e.g., Maniatis et al. (2001) *supra).*

Another embodiment of the present invention relates to a vector comprising the inventive nucleic acid. It has to be noted that the terms "vector", "construct" and "recombinant construct" as used herein are intended to have the same meaning and define a nucleotide sequence which comprises beside other sequences the nucleic acid sequence - or a fragment thereof - of the invention. A vector can be used, upon transformation into an appropriate host cell, to allow expression of an inventive nucleic acid. The vector may be a plasmid, a phage particle or simply a potential genomic insert. Once transformed into a suitable host, the vector may replicate and function independently upon the host genome, or may, under suitable conditions, integrate into the genome itself. A preferred vector of the invention is the pBK-CMV plasmid.

The aforementioned "other sequences" of a vector relates to the following sequences: In general, a suitable vector includes an origin of replication, for example, Ori p, colEl Ori, sequences which allow the inserted nucleic acid to be expressed (transcribed and/or translated) and/or a selectable genetic marker including, e.g., a gene coding for a fluorescence protein, like GFP, and/or genes, which confer resistance to antibiotics, such as the p-lactamase gene from Tn3, the kanamycin-resistance gene from Tn903 or the chloramphenicol-resistance gene from Tn9.

The term "plasmid" means an extrachromosomal usually self-replicating genetic element. Plasmids are generally designated by a lower "p" preceded and/or followed by letters and numbers. The starting plasmids herein are either commercially available, publicly available on an unrestricted basis or can be constructed from available plasmids in accordance with the published procedures. In addition, equivalent plasmids to those described are known to a person skilled in the art. The starting plasmid employed to prepare a vector of the present invention may be isolated, for example, from the appropriate *E. coli* containing these plasmids using standard procedures such as cesium chloride DNA isolation.

In general, a vector according to the invention relates to a (recombinant) DNA cloning vector as well as to an (recombinant) expression vector. A DNA cloning vector refers to an autonomously replicating agent, including, but not limited to, plasmids and phages, comprising a DNA molecule to which one or more additional nucleic acids of the invention have been added. An expression vector relates to any DNA cloning vector recombinant construct comprising a nucleic acid sequence of the invention operably linked to a suitable control sequence capable of effecting the expression and to control the transciption of the inserted nucleic acid of the invention in a suitable host. Also, the plasmids of the present invention may be readily modified to construct expression vectors that produce the polypeptides of the invention in a variety of organisms, including, for example, *E. coli,* Sf9 (as host for baculovirus), *Spodoptera* and *Saccharomyces.* The literature contains techniques for constructing AV12 expression vectors and for transforming AV12 host cells. U.S. Pat. No. 4,992,373, herein incorporated by reference, is one of many references describing these techniques. "Operably linked" means that the nucleic acid sequence is linked to a control sequence in a manner, which allows expression (e. g., transcription and/or translation) of the nucleic acid sequence. "Transcription" means the process whereby information contained in a nucleic acid sequence of DNA is transferred to its complementary RNA sequence.

"Control sequences" are well known in the art and are selected to express the nucleic acid of the invention and to control the transcription. Such control sequences include, but are not limited to a polyadenylation signal, a promoter (e.g., natural or synthetic promotor) or an enhancer to effect transcription, an optional operator sequence to control transcription, a locus control region or a silencer to allow a tissue-specific transcription, a sequence encoding suitable ribosome-binding sites on the mRNA, a sequence capable to stabilize the mRNA and sequences that control termination of transcription and translation. These control sequences can be modified, e.g., by deletion, addition, insertion or substitution of one or more nucleic acids, whereas saving their control function. Other suitable control sequences are well known in the art and are described, for example, in Goeddel (1990), Gene Expression Technology:Methods in Enzymology 185, Academic Press, San Diego, CA. Especially a high number of different promoters for different organism is known. For example, a preferred promoter for vectors used in *Bacillus subtilis* is the AprE promoter; a preferred promoter used in *E. coli is* the T7/Lac promoter, a preferred promoter used in *Saccharomyces cerevisiae* is PGK1, a preferred promoter used in *Aspergillus niger* is glaA, and a preferred promoter used in *Trichoderma reesei (reesei)* is cbhl. Promoters suitable for use with prokaryotic hosts also include the beta-lactamase (vector pGX2907 (ATCC 39344) containing the replicon and beta-lactamase gene) and lactose promoter systems (Chang et al. (1978), Nature (London), 275:615; Goeddel et al. (1979), Nature (London), 281:544), alkaline phosphatase, the tryptophan (trp) promoter system (vector pATH1 (ATCC 37695) designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable a person skilled in the art to ligate them to DNA encoding the polypeptides of the instant invention using linkers or adapters to supply any required restriction sites. Promoters for use in bacterial systems also will contain a Shine-Dalgarno sequence operably linked to the DNA encoding the desired polypeptides.

Useful expression vectors, for example, may consist of segments of chromosomal, non-chromosomal and synthetic DNA sequences such as various known derivatives of SV40 and known bacterial plasmids, e.g., plasmids from *E. coli* including col E1, pBK, pCR1, pBR322, pMb9, pUC 19 and their derivatives, wider host range plasmids, e.g., RP4, phage DNAs e.g., the numerous derivatives of phage lambda, e.g., NM989, and other DNA phages, e.g., M13 and filamentous single stranded DNA phages, yeast plasmids, vectors useful in eukaryotic cells, such as vectors useful in animal cells and vectors derived from combinations of plasmids and phage DNAs, such as plasmids which have been modified to employ phage DNA or other expression control sequences. Expression techniques using the expression vectors in the sense of the present invention are known in the art and are described generally in, for example, Maniatis et al. (2001) *supra.*

Another embodiment of the present invention relates to a host cell comprising a vector or the nucleic acid (or a fragment thereof) of the present invention.

In this context, "host cell" means a cell, which has the capacity to act as a host and expression vehicle for a nucleic acid or a vector according to the present invention. The host cell can be e.g., a prokaryotic, an eukaryotic or an archaeon cell. In a preferred embodiment according to the present invention, host cell means the cells of *E. coli.* Examples of host cells comprising (for example, as a result of transformation, transfection or tranduction) a vector or nucleic acid as described herein include, but are not limited to, bacterial cells (e.g., *R. marinus, E. coli, Streptomyces, Pseudomonas, Bacillus, Serratia marcescens, Salmonella typhimurium),* fungi including yeasts (e. g., *Saccharomycies cerevisie, Pichia pastoris*) and molds (e.g., *Aspergillus sp.),* insect cells (e.g., Sf9) or mammalian cells (e.g., COS, CHO).

A variety of eukaryotic host cells are available, e.g., from depositories such as the American Type Culture Collection (ATCC) and are suitable for use with the vectors of the present invention. The choice of a particular host cell depends to some extent on the particular expression vector used to drive expression of the nucleic acids of the present invention. The imperfect fungus *Saccharomyces cerevisiae* is the most commonly used eukaryotic microorganism, although a number of other strains are commonly available. For expression in *Saccharomyces sp.,* the plasmid YRp7 (ATCC-40053), for example, is commonly used (see. e.g., Stinchcomb L. et al. (1979) Nature, 282:39; Kingsman J. al. (1979), Gene, 7:141; S. Tschemper et al. (1980), Gene, 10:157). This plasmid already contains the trp gene, which provides a selectable marker for a mutant strain of yeast lacking the ability to grow in tryptophan. Suitable promoting sequences for use with yeast hosts include the promoters for 3-phosphoglycerate kinase (found on plasmid pAP12BD (ATCC 53231) and described in U.S. Pat. No. 4,935,350, issued Jun. 19, 1990, herein incorporated by reference) or other glycolytic enzymes such as enolase (found on plasmid pAC1 (ATCC 39532)), glyceraldehyde-3-phosphate dehydrogenase (derived from plasmid pHcGAPC1 (ATCC 57090, 57091)), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase, as well as the alcohol dehydrogenase and pyruvate decarboxylase genes of Zymomonas mobilis (U.S. Pat. No. 5,000,000 issued Mar. 19, 1991, herein incorporated by reference). Other yeast promoters, which are inducible promoters, having the additional advantage of their transcription being controllable by varying growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV (ATCC 39475) and described in U.S. Pat. No. 4,840,896, herein incorporated by reference), glyceraldehyde 3-phosphate dehydrogenase, and enzymes responsible for maltose and galactose (e.g. GAL1 found on plasmid pRY121 (ATCC 37658)) utilization. Yeast enhancers such as the UAS Ga1 from Saccharomyces cerevisiae (found in conjuction with the CYC1 promoter on plasmid YEpsec--hl1beta ATCC 67024), also are advantageously used with yeast promoters.

A vector can be introduced into a host cell using any suitable method (e.g., transformation, electroporation, transfection using calcium chloride, rubidium chloride, calcium phosphate, DEAE dextran or other substances, microprojectile bombardment, lipofection, infection or transduction). Transformation relates to the introduction of DNA into an organism so that the DNA is replicable, either as an extrachromosomal element or by chromosomal integration. Methods of transforming bacterial and eukaryotic hosts are well known in the art. Numerous methods, such as nuclear injection, protoplast fusion or by calcium treatment are summarized in Maniatis et al. (2001) *supra.* Transfection refers to the uptake of a vector by a host cell irrespective of whether any coding sequences is expressed. Successful transfection is generally recognized when any indication or the operation or this vector occurs within the host cell.

Another embodiment of the invention provides a method for the production of the polypeptide of the present invention comprising the following steps:
(a) cultivating a host cell of the invention and expressing the nucleic acid of the invention under suitable conditions; (b) isolating the polypeptide by suitable means.

The polypeptides according to the present invention may typically be produced by recombinant methods. Recombinant methods are preferred if high yield is desired. A general method for the construction of any desired DNA sequence is provided, e.g., in Brown J. et al. (1979), Methods in Enzymology, 68:109; Maniatis (1982), *supra.*

According to the invention an activity-based screening in metagenome library was used as a powerful technique (Lorenz, P et al. (2002), Current Opinion in Biotechnology 13:572-577) to isolate the inventive laccase from the large diversity of microorganisms found in a rumen ecosystem.

This method may be generally applied with the object to isolate other naturally occurring polypeptides with laccase activity from any ecosystem, in particular any animal ecosystem or, more preferably, from rumen of any animal.

Isolation of polypeptides from the culture medium can be carried out by conventional procedures including separating the cells from the medium by centrifugation or filtration, if necessary after disruption of the cells, precipitating the proteinaceous components of the supernatant or filtrate by means of a salt, e. g., ammonium sulfate, followed by purification by a variety of chromatographic procedures, e. g., ion exchange chromatography, affinity chromatography or similar art recognized procedures. Efficient methods for isolating polypeptides of present invention also include utilizing genetic engineering techniques by transforming a suitable host cell with a nucleic acid or a vector provided herein, which encodes the polypeptide, and cultivating the resultant recombinant microorganism, preferably *E.coli,* under conditions suitable for host cell growth and nucleic acid expression, e.g., in the presence of inducer, suitable media supplemented with appropriate salts, growth factors, antibiotic, nutritional supplements, etc.), whereby the nucleic acid is expressed and encoded polypeptide is produced.

Skilled artisans will recognize that the polypeptides of the present invention can be produced by a number of different methods well known in the art. Thus, polypeptides of the invention may be produced e.g. by *in vitro* translation of nucleic acids that encode the polypeptides, by chemical synthesis (e. g., solid phase peptide synthesis) or by any other suitable method. Recombinant methods and chemical methods are described, for example, in US Pat. No. 4,617,149, which is herein incorporated by reference in its entirety. A suitable description of the principles of solid phase chemical synthesis of polypeptides can be found in Dugas H. and Penney C. (1981), Bioorganic Chemistry, pages 54-92. For examples, polypeptides may be synthesized by solid-phase method utilizing an Applied Biosystems 430A peptide synthesizer (commercially available from Applied Biosystems, Foster City, Calif.) and synthesis cycles supplied by Applied Biosystems. Protected amino acids, such as t-butoxycarbonyl-protected amino acids, and other reagents, which are required, are commercially available from many chemical supply houses.

Inventive polypeptides showing laccase activity, nucleic acids encoding them, vectors and/or cells comprising them (hereinafter "inventive substances") can be applied in various industrial and biotechnical fields. Preferred embodiments of the invention provide the use of inventive substances, in particular inventive polypeptides, as food additive, preferably as animal food additive, more preferably as animal food additive for pasture fed animals. Its addition to animal food may be particularly advantageous for animals digesting plants with a high lignin content, e.g. ryegrass. A general problem in animal feed, in particular for feeding e.g. cattle, is inefficient or partial digestion of high lignin plants, which results in energy loss. Thus, inventive substances are usable as food additive for pasture fed animals to improve the digestion of e.g. ryegrass lignin. Accordingly, inventive substances can be formulated as slow release capsules which allow the inventive substance to catalyze the lignin decomposing reaction over time at the appropriate site of the intestine. Formulation and dosage of such capsules are chosen accordingly, e.g. depending on the animal to be fed. Furthermore, use of inventive polypeptides, nucleic acids and/or vectors for expression in plant chloroplasts is encompassed by the present invention.

Food applications of the inventive subject-matter also comprise, e.g., the use in the manufacture of food ingredients or as a food ingredient itself, for example for bread-making applications, removing or modifying problematic phenolic saccharides from clear fruit juice or fermented alcohol beverages, e.g., wine and beer, to improve the clarity, colour appearance, flavour, aroma, taste or stability. The use of inventive polypeptides with laccase activity in beverage production is particularly preferred. Known laccases are approved for use in brewing beer, e.g. it is applied during the mashing process to prevent the formation of an off-flavor compound, trans-2-nonenal. Laccases may also be immobilized on a copper-chelate carrier and e.g. used to remove phenols from white grape must during clarification of wine. Therefore, inventive polypeptides may be used for all of these applications.

In addition, inventive polypeptides having laccase enzyme activity may be used as a preparation in breath-freshening products (such as breath mints and chewing gum). It is estimated that this use of laccase enzyme preparation as a direct food ingredient would result in the consumption of up to approximately 14 milligrams per person per day of the total organic solids present in the laccase enzyme preparation.

Another embodiment relates to the use of the inventive substances for the treatment of cellulosic textiles or fibres, textile dyeing, stain bleaching, cotton fiber whitening, dye finishing, dye decolourisation, detoxification and/or laundry cleaning. Also preferred is the use of the inventive substances in detergent application, for example for bleaching stains or to inhibit so-called "dye-transfer" between cloths of different colours during wash procedures.

Another embodiment relates to the use of the inventive substances for the treatment in pulp and paper treatment, preferably in biobleaching of kraft pulp and/or by lowering the kappa number of pulp. Laccases increase wet strength properties of pulp and paper products by assisting in the polymerisation of lignin and phenolic compounds found in some raw materials. The benefits of using enzymes to modify lignin and increase strength properties are (i) reduced consumption of strength-enhancing chemicals, (ii) improved runnability of the paper machine, (iii) potential for increasing production capacity and/or (iv) easier and cheaper effluent management. Inventive polypeptides, fulfil these properties. Accordingly, inventive substances may be used for treating wood containing pulp and color and may influence the BOD/COD reduction of waste water streams, e.g. by immobilized inventive polypeptides, as well. Therefore, detoxification of industrial effluent, e.g. from the paper and pulp and petrochemical industries, is to be envisaged as use for the inventive polypeptides as well.

Enzymatic methods of altering fibres have been sought because these methods use less chemicals, are deemed more controlled and are less likely to damage the fibre than chemical methods. Since lignin and related phenolic compounds are unwanted in effluent due to oxygen demand (COD/BOD) and colour and since they are also difficult to get rid of, inventive substances, eventually combined with other substances, in particular e.g. peroxidase(s), solve this problem. Inventive substances form radicals of the phenolic compounds which in turn polymerise. The low solubility of the polymers causes the compounds to precipitate. The solid precipitate can easily and cheaply be removed from the effluent.

It is to be emphasized that inventive substances, in particular inventive polypeptides, may be used as a bioremediation agent in general in order to clean up e.g. herbicides, pesticides and certain explosives e.g. in soil. It may also be used as a cleaning agent for certain water purification systems. Therefore, use of the inventive substances for environment protection, in particular in bioremediation, biodegradation of e.g. liguin, biodetoxification and/or biodecontamination of environmental pollutants, is disclosed herewith. Inventive substances represent excellent and improved alternatives to widely used peroxidases as a bianalytical tool for monitoring polar polluents (see for review, e.g., Duran et al., Enzyme and Microbial Technol. 31 (2002) 907-931) and for the bioremediation of aromatic recalcitrant compounds.

Additionally, inventive polypeptides are usable, for example, in the following applications (without being limited to these applications)
- as biosensors and bioreporters in combination with co-substrates that are either chromogenic, fluorogenic, chemiluminescent or electroactive, including, e.g. immobilized-enzyme electrodes for measuring the phenolic contents of aqueous samples and, in more specific applications, fruit juices, tea and other beverages or biosensors for measurement of gas-phase oxygen or as a replacement for horseradish peroxidase (HRP) as the marker enzyme in enzyme-linked immunoassays,
- use for organic syntheses, e.g., for ethanol production, for asymmetric of chiral synthesis, for example to transform prochiral aldehydes or ketones to chiral alcohol, hydroxyl acids, amino acids etc.; for synthesis of polymers, such as polyphenolic polymers; for synthesis of medicinal agents including triazolo(benzo)cycloalkyl thiadiazines, vinblastine, antibiotics and dimerized vindoline; and
- in medical and personal care applications, e.g., in products for disinfection, as an ingredient in cosmetics, e.g. skin care, hair care, dental care; as deodorants for personal-hygiene products, such as toothpaste, mouthwash, chewing gum, detergent and soap.
- as a tool for medical diagnostics, aromatic diamine and aminophenol, catechol, catecholamines, hydroquinone and homogentisic acid (HGA), e.g. for detecting alcaptonuria, and
- as a catalyst for the manufacture of anti-cancer drugs
- oxidation of phenolics e.g. in wine.

It is to be understood that the inventive substances may serve as a biocatalyst suitable for a broad spectrum of applications.

In summary, the present invention discloses inventive polypeptides with laccase activity based on a naturally occurring enzyme, named RL5 laccase, which was retrieved from the bacteriophage lambda-based expression library created from DNA isolated from rumen metagenome, in particular rumen metagenome from New Zealand dairy cow. RL5 laccase shows unusually high oxidative capability and exhibits no nucleotide sequence similarity to known laccases from all public available databases (Altschul *et al.,* 1997). Deduced amino acid sequence alignment of RL5 ORF1, RL5 ORF2 and RL5 ORF3 gene products is failed to find significant similarity to known laccases and other phenol oxidases. Moreover, RL5 laccase does not contain previously predicted functional laccase motifs. RL5 is a novel, high performance catalyst for the transformation of a wide variety of substrates, possessing a variety of novel structural features.

The invention further discloses the function of members of a large class of hypothetical proteins each containing DUF152 domains of yet unknown function. According to the invention these proteins were identified as proteins with laccase activity. Said polypeptides are listed in Figure 18 (polypeptides 1 to 703). Their use as laccases and, more specifically, for the applications described in detail herein is disclosed herewith. Use of polypeptides 1 to 703 (or their derivatives or fragments) also encompasses the use of their underlying nucleic acid sequences, vectors comprising said nucleic acid sequences and cells containing said vectors for the preparation of the above polypeptides. It is noted that any definition given herein (e.g. definition of "fragments", "derivatives", "polypeptide", "functional", "activity" etc.) does include polypeptides selected from the group of sequences according to figure 18, the use of which as laccases is for the first time disclosed herewith. In particular, the BT4389 protein as well as the YifH protein belong to the group of polypeptides, which are given in figure 18.

The following Figures and Examples are only thought to illustrate the present invention without limiting the scope thereon. All references cited in the present description are incorporated in their entirety.

### Figures

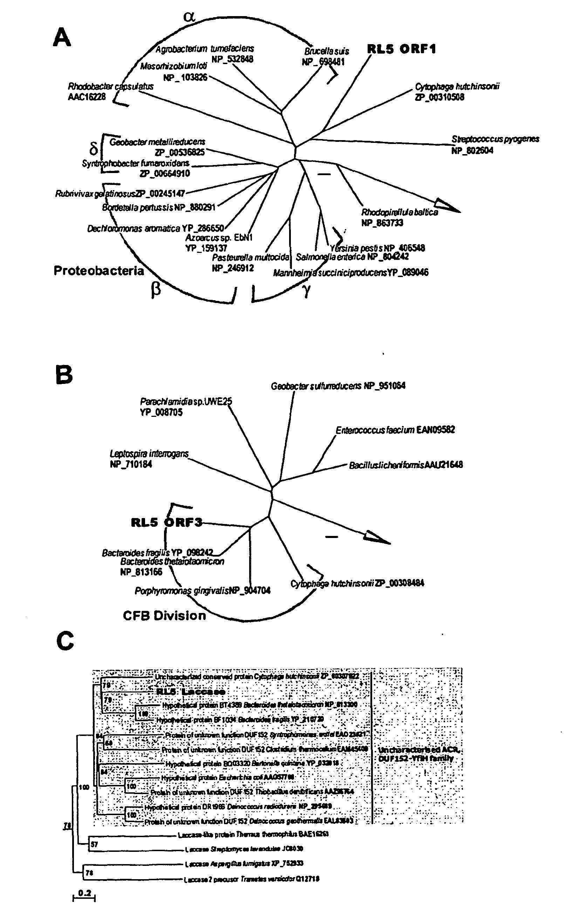

**Figure 1** shows rooted amino acid trees based on alignments of RL5 ORF's with related homologous sequences.

Previously performed searches for coding areas of new gene RL5 revealed at the centre of the whole cloned and sequenced RL5 DNA fragment (5,596 bp) the presence of three differentially transcribed ORF's (RL5 ORF1, RL5 ORF2 and RL5 ORF3)of significant length (> 700 bp):
- RL5 ORF1 (876 bp, positions 1,473 to 2,348) encodes a protein of 291 amino acids in length with a predicted molecular mass of 32.24 kDa. This product is very hydrophilic with estimated pl (isoelectric point) of 4.97 and contains only one possible transmembrane domain, typical features for cytosolic enzymes.
- RL5 ORF2 extends complementary from position 3,199 to a TAA stop codon at position 2,411 (789 bp) and encodes a product of 262 amino acids in length. This 28.28 kDa polypeptide also shows the cytosolic features with estimated pl value of 5.29.
- RL5 ORF3 (1,113 bp) is located 72 bp downstream from the ATG codon of ORF2. The product of this gene is a polypeptide of 370 amino acids in length with an pl value of 6.46 and includes several helix-turn-helix motifs typical of DNA-binding proteins.

Subsequent to the identification of RL5 ORF's, amino acid alignments with homologous proteins were performed. The proteins selected for these alignments with RL5 ORF's were found to be homologous using Fasta 3.3 homology searching against UniProt protein database (release 2.3) with BLOSUM50 matrix. Multiple sequence alignments were constructed using MacVector 7.2.2 software (Accelrys, San Diego, CA) with BLOSUM matrix, open and extended gap penalties of 10.0 and 0.05 respectively. The alignment file was then analysed with the same software for calculation of distance matrix. Neighbour-joining and Poisson-correction of distances were used to construct the phylogenetic trees shown in **Figure 1.** The bar represents 0.2 changes per amino acid.

**Figure 1A** shows the phylogenic relationship of RL5 ORF1 putative Zn-peptidase with metalloproteases. The results revealed that the RL5 ORF1 product belongs to family of Zn-peptidases involved in metal-dependent peptide bond cleavage and exhibits high similarity to *Cytophaga hutchinsonii* predicted metalloprotease (42% of identity and 62% of positive).

**Figure 1B** shows the phylogenic relationship of RL5 ORF3 recombinational DNA repair ATPases (RecF) with RecF. As result of the alignments, the function of RL5 ORF3 product was predicted. RL5 ORF3 shows a high homology to recombinational DNA repair ATPases (RecF) of *Bacteroides thetaiotaomicron* and *Bacteroides fragilis* (49% of identity and 69% of positive) and to many other proteins belonging to this family, but in less extend.

**Figure 1C** shows the phylogenic relationship of RL5 ORF2 (laccase) with DUF152 domain. The results revealed that the RL5 ORF2 protein exhibits high degree of similarity to the conserved hypothetical proteins (CHP) belonging to the family DUF152 (domain of unknown function 152), tightly associating with CHPs of *Bacteroides thetaiotaomicron, Bacteroides fragilis* and *Cytophaga hutchinsonii* (57, 53 and 52% of similarity, respectively).

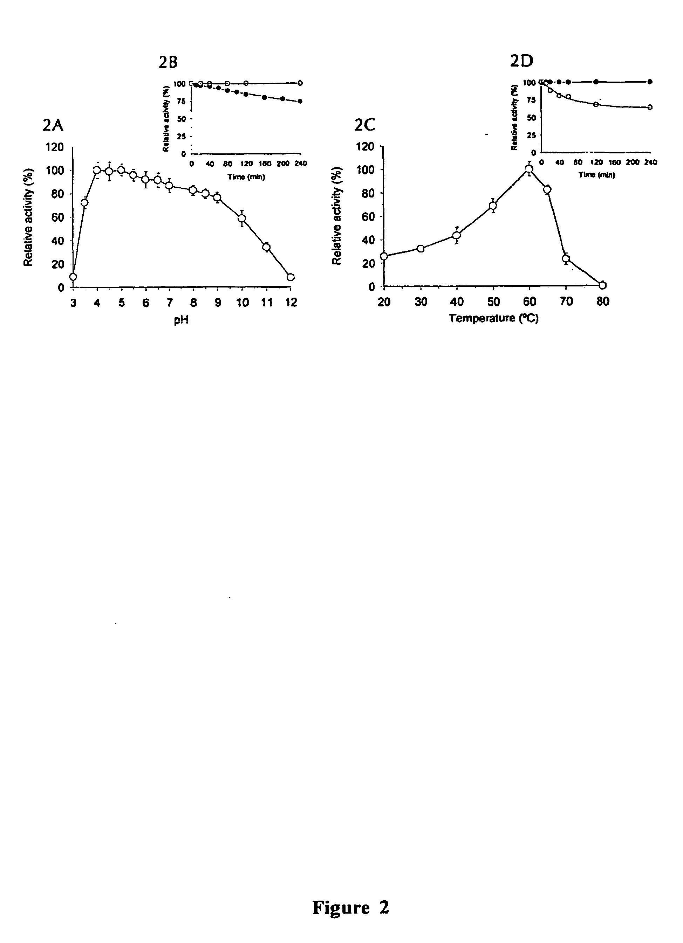

**Figure 2** shows pH- and temperature-dependent activity profile of recombinant RL5 laccase.

**Figure 2A** represents the effect of various pH values on the activity of recombinant purified RL5 laccase. The activity was measured at 40 °C using 1 mM SGZ (syringaldazine) as substrate. The buffers (100 mM) used were: citrate (pH 3.0 - 5.5), MES (pH 5.5 - 7.0), HEPES (pH 7.0 - 8.0), Tris-HCl (pH 8.0 - 9.0) and glycine-NaOH (pH 9.0 - 12). The activity of RL5 against DMP was reaching an optimum at a broad pH ranging from 3.5 - 9.0, being optimal at 4.0 - 5.0; however, it maintained more than 70% activity at pH 3.5 and 9.0 (Fig. 2A).

**Figure 2B** represents the pH inactivation of purified RL5 laccase in sodium citrate pH 4.5 (indicated with "o") or glycine-NaOH pH 9.0 (indicated with "•") over a time period of 240 min. 100% activity in the purified protein sample equals 7.2 units/mg (measured at 40 °C). The results confirmed that RL5 was highly stable over a long time period at these pHs, and thus at a broad pH ranging.

**Figure 2C** represents the effect of the temperature on the activity of recombinant purified RL5 laccase. The enzyme activity was assayed at each indicated temperature in sodium citrate pH 4.5 in the presence of 1 mM SGZ. Activity was monitored at 420 nm. Protein activity increased as the temperature was raised up to a maximum at ~60 °C. Therefore, RL5 is stable at high temperatures.

**Figure 2D** represents the thermal inactivation of purified RL5 laccase. Purified enzyme was incubated in vials at 60 °C (•) or 65 °C (o). At the indicated times, samples were withdrawn and tested for laccase activity at 40 °C by using SGZ as substrate. 100% activity in the purified protein sample equals 17.2 units/mg. As can be seen, RL5 is also highly stable at this temperatures over a long time period, for at least four hours.

Corresponding experiments were performed with BT4389 and YifH under conditions as described above for **Figures 2A to 2D** (data not shown). The activity of these enzymes was reaching an optimum at a temperature of 52 °C (for BT4389) and 44 °C (for YifH) and the thermal activity was maintained for more than 80 % at 55 °C (for BT4389) and at 50°C (for YifH). The activity of both enzymes was reaching an optimum at pH ranging from 4.5 to 6.0 (for BT4389) and from 5.5 to 8.4 (for YifH), although they maintained 80 % activity at pH 4.0 to 7.5 (for BT4389) and 5.0 to 9.0 (for YifH).

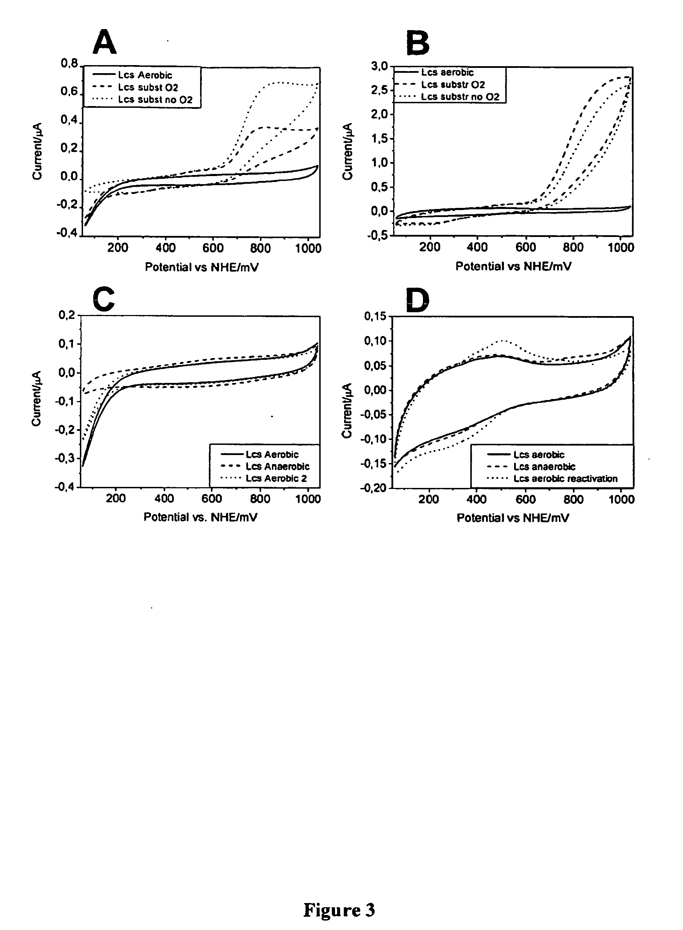

**Figure 3** shows the results of electrochemical analysis of RL5 laccase in the presence of DMP as substrate at 10 mV/s (**Figures 3A,B**) or absence of substrate (**Figures 3C,D**). RL5 was immobilized on BAS glassy carbon electrodes (Figures 3B,D) or on gold electrodes (**Figures 3A,C**). Both materials provide different enzyme orientations:
(i) the carbon electrode presents hydroxyl groups in the surface, providing a laccase substrate-like structure, which makes the enzyme to immobilize preferably with the active site facing the electrode surface; and
(ii) gold provides high hydrophobic surface that avoids the contact between the high-hydrophilic density amino acid domain of the enzyme and the electrode, being the hydrophobic domains which preferably orient to the gold surface.

Some differences in the electrochemical measurements were observed due to the different orientation of the enzyme on the electrode in the presence of substrate (DMP) (**Figures 3A,B**). Thus, when RL5 laccase was immobilized on gold (**Figure 3A**), the activity in the presence of oxygen was of 0.38 microamperes (dashed line), whereas in the absence of oxygen it was of 0.69 microamperes (dotted line). Most likely, the enzyme is taking approximately half of the electrons for the reduction of the substrate and the other half from the oxygen in the solution; however, in absence of oxygen, all the electrons are taken from the electrode, giving a higher measurement value. When using the carbon electrode (Figure 3B), the enzyme orients in the way that the measure, in the presence of oxygen, was 2.8 microamperes (dashed line). This could be explained by the fact that 93% of the electrons are taken from the electrode, either because of different enzyme orientations, or because the immobilization on carbon kept higher amount (approximately 7 times) of laccase active. In the carbon electrode the T2 copper oxidization potential site, +500 vs. normal hydrogen electrode (NHE) was determined.

Electrochemical information on electrodes modified with RL5 laccase in absence of substrate was also obtained (**Figures 3C,D**). Again, we found some differences when the electrode material was gold or glassy carbon. Thus, the electro-reduction of oxygen begins in +250 mV when the electrode was gold (**Figure 3C**), while it began at +500 mv when it was glassy carbon (**Figures 3D**). We found additional information on the glassy carbon electrode compared with the gold electrode. Thus, when removing the oxygen from solution, the T1 copper site oxidization potential of 740 mV vs. NHE was registered. In addition, when a reactivation with oxygen was performed, the three oxidation potential peaks were registred: T1 of 745 mV vs. NHE was the highest; T3 site of 500 mV vs. NHE had two copper ions, so it is the one with a higher area; T2 site had 400 mV vs. NHE. The redox potential of T1 was registered to be in the range of fungal laccases (480-785 mV) (Solomon *et al.,* 1996; Klonowska *et al.,* 2002).

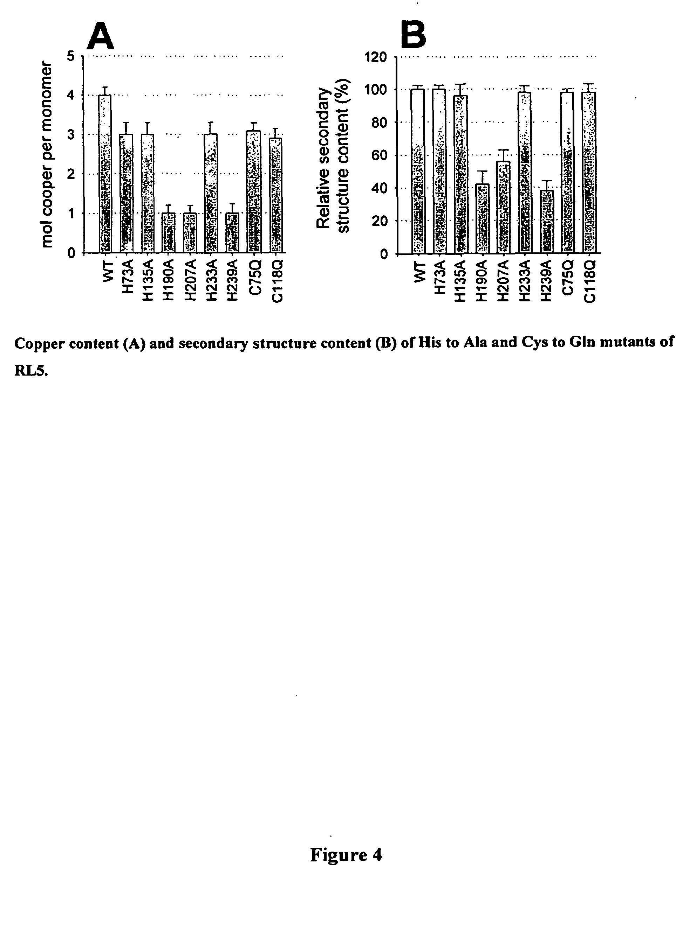

**Figure 4** represents a graphically view of the copper content (**Figure 4A**) and secondary structure content (**Figure 4B**) of histidine (H) to alanine (A) and cysteine (C) to glutamine (Q) mutants of RL5 laccase. Copper content was determined using the Perkin-Elmer Life Sciences ICP-MS. RL5 wild type and mutant variants were purified using a Ni-Sepharose column after expression with a carboxyl-terminal 6xHis tag. Previously performed sequence analysis revealed that RL5 laccase does not possess the typical conserved histidine regions that bind the copper atoms in laccases (as, e.g., described in Solomon *et al.,* 1996). To determine the amino acids of RL5, which are important for copper binding, the site-directed mutagenesis coupled with model-driven rational design was performed followed by absorption spectrophotometric and secondary structure determinations. At first, all six histidine residues in the RL5 protein, namely H73, H135, H190, H207, H233 and H239, were replaced by alanine (A) and found that H73A, H135A and H233A substitutions decreased copper binding by approx. 25% (Fig. 4A) with no substantial changes in the secondary structure (Fig. 4B). However, although the metal contents of variants containing the H190A, H207A or H239A mutations is 75% that of the wild type enzyme (Fig. 4A), they produced major global changes in secondary structure (Fig. 4B).

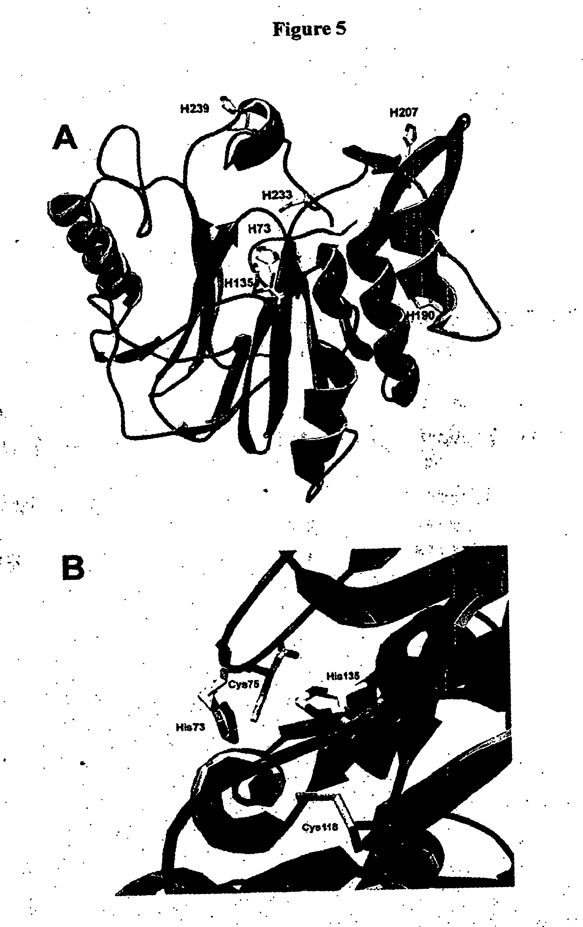

**Figure 5** show three-dimensional views of RL5 laccase structures. **Figure 5A** represents an overall three-dimensional structure of RL5 laccase, obtained by homology modelling. **Figure 5B** shows the location of the T1 copper site and residues involved in cooper coordination.

To determine other amino acid residues as those identified in **Figure 4**, which are involved in copper coordination, a three-dimensional model of RL5 laccase was created (**Figure 5A**). According to this model, the T1 site should be formed by H73, C75, C118 and H135, as indicated in **Figure 5B**. To prove that these residues are indeed copper ligands, the following mutants were produced: C75Q and C118Q. The pure mutants were analysed for their ability to coordinate copper (see Examples). As shown in **Figure 4,** both mutated RL5 proteins present 3 mol copper per monomer (see **Figure 4A**), one less than the wild type enzyme, and (no changes in secondary spectra were detected (see **Figure 4B**). The results confirmed that H73, C75, C118 and H135 were unambiguously identified as the residues involved in the T1 copper site.

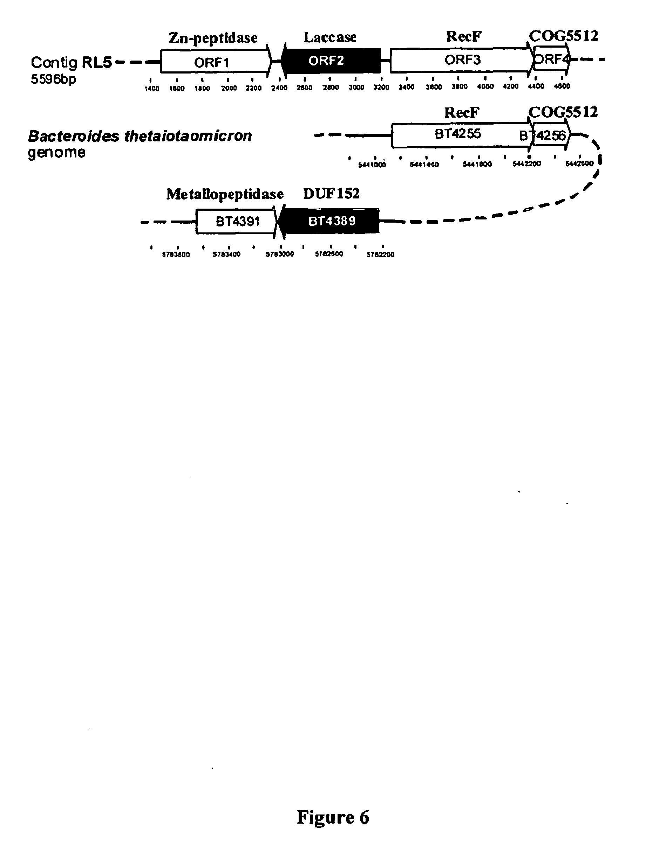

Figure 6 represents a comparison between structural organization of the genes located in flanking region of RL5 laccase in RL5 DNA fragment and similar genes found in *Bacteroides thetaiotaomicron* genome. RL5 laccase and DUF152 genes are highlighted in black.

As shown in **Figure 1,** sequencing of the RL5 DNA fragment flanking RL5 laccase gene revealed the presence of and RecF (ORF3, **Figure 1B**) and putative metalloprotease (ORF1, **Figure 1A**) genes highly similar to either *Bacteroides* species (ORF3, **Figure 1B**) or *Cytophaga hutchinsonii* (ORF1, **Figure 1A),** bacteria belonging to very diverse *Cytophaga-Flexibacter-Bacteroides* (CFB) phylum. It is well known, that CFB is one of three bacterial phyla, which dominate ruminal microflora. Thus, the probability that the inventive cloned RL5 belongs a genome fragment of bacterium belonging to CFB division was very high. To prove this statement the region immediately downstream from putative *rec*F gene (ORF3) was analyzed and revealed two additional ORFs transcribed in the same direction as *rec*F*.* The deduced ORF4 and ORF5 proteins are 62% and 60% similar to the putative Zn-ribbon-containing RNA-binding protein of COG5512 family and the thiesterase from *Bacteroides thetaiotaomicron,* respectively. Moreover, as shown in **Figure 6,** the structural organisation of ORF1, ORF2, ORF3 and ORF4 genes within the RL5 genome fragment was found to share common features with positioning of similar genes in *Bacteroides thetaiotaomicron* genome. Putative metalloprotease and DUF152 genes of *Bacteroides thetaiotaomicron* are organized in two proximal and opposite operons, as it was demonstrated in RL5 genome fragment. RecF and COG5512 genes also appear together in both *Bacteroides thetaiotaomicron* and RL5. The only difference was that these two gene tandems are combined in RL5 fragment, while in *Bacteroides thetaiotaomicron* genome they are separated by almost 350.000 bp.

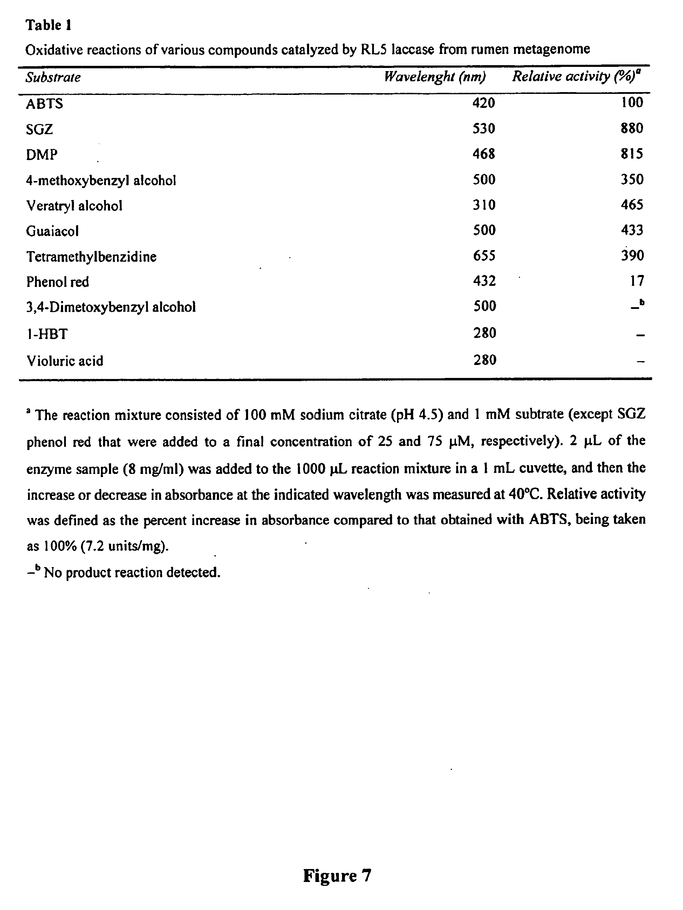

**Figure 7** shows **Table 1** representing the results of oxidative reations of various compounds catalyzed by the inventive RL5 laccase. The substrate preference of the RL5 laccase was: SGZ > DMP > veratryl alcohol > guaiacol > tetramethylbenzidine > 4-metoxybenzyl alcohol > 2ABTS > phenol red. Other substrates, such as 3,4-dimetoxybenzyl alcohol, 1-HBT and violuric acid were not substrates of RL5.

The substrate specificity towards various aromatic compounds was also measured for BT4389 and YifH (data not shown). BT4389 laccase oxidized SGZ > DMP > veratryl alcohol >> guaiacol > ABTS (600:255:113:60:28:17 units/g protein), whereas YifH oxidized SGZ > DMP >> veratryl alcohol >> guaiacol (5.6:4:2.1:0.5 units/g protein).

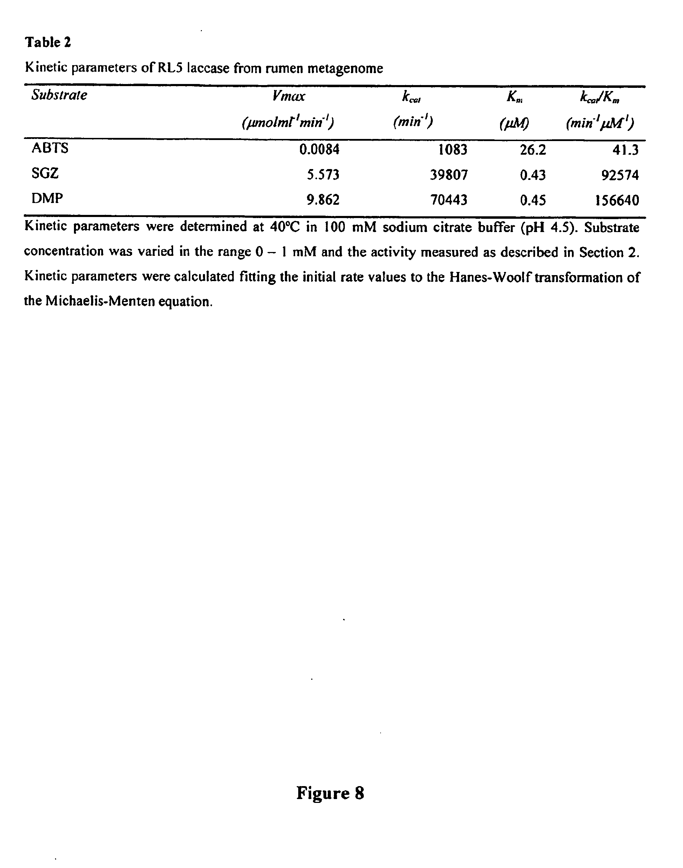

**Figure 8** shows Table 2 indicating the kinetic parameters of the inventive RL5 laccase. The kinetic parameters *K*ₘ, *k*_{cat} and *k*_{cat}/*K*ₘ values of the laccase was estimated with a series of 3 commonly used laccase substrates, namely ABTS, SGZ and DMP, in the concentration range of 0 to 1 mM. Steady-state kinetic measurements were performed at 40 °C, pH 4.5, in the presence of 14 nM protein and yielded values for the apparent *K*ₘ of 26.2, 0.43 and 0.45 µM, and *k*_{cat} of 1083, 39807 and 70443 min⁻¹ for ABTS, SGZ and DMP, respectively. These values for the *K*ₘ are ∼5 times smaller than that reported for similar enzymes, whereas the *k*_{cat} values are up to 40 times higher; moreover, the *k*_{cat}/*K*ₘ ratio of RL5 laccase greatly exceed published values for the oxidation of ABTS (up to 300 min⁻¹µM⁻¹) and specially SGZ (up to 9700 min⁻¹µM⁻¹) and DMP (up to 15000 min⁻¹µM⁻¹) (see examples in Solomon *et al.,* 1996 and reference therein; Chefets *et al.,* 1998; Klonowska *et al.,* 2002; Machczynski *et al.,* 2004).

**Figure 9** shows the amino acid sequence of RL5 laccase of the invention. The N-terminal sequence of pure protein was found to be MIELEKLDFAKSVEGVE, corresponding to amino acid residues 1-17 of the translated sequence of RL5 laccase gene.

**Figure 10** shows the amino acid sequence of BT4389 laccase from *Bacteroides thetaiotaomicron* (Gene Bank accession number NP_813300).

**Figure 11** shows the amino acid sequence of YifH laccase from *Escherichia coli* (Gene Bank accession number AAG57706).

**Figure 12** shows the nucleic acid sequence of RL5 laccase gene of the invention, in opposite direction. The coding region is underlined.

**Figure 13** shows the nucleic acid sequence of ORF (open reading frame) of RL5 laccase gene of the invention.

**Figure 14** shows the nucleic acid sequence of BT4389 laccase gene from *Bacteroides thetaiotaomicron* (Gene Bank accession number NP_813300).

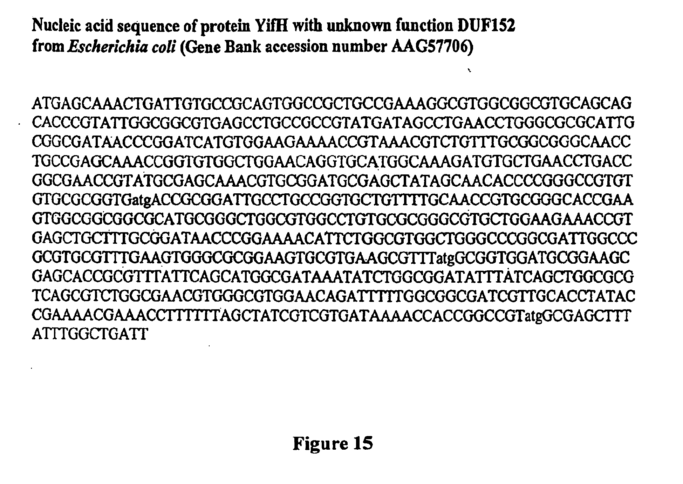

**Figure 15** shows the nucleic acid sequence of YifH laccase gene from *Escherichia coli* (Gene Bank accession number AAG57706).

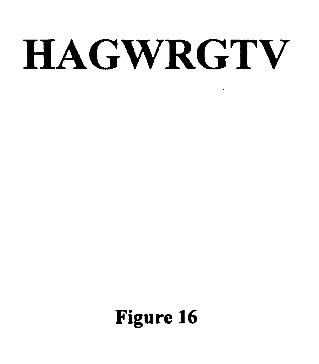

**Figure 16** shows the characteristic core amino acid sequence of a DUF152 domain (HAGWRGTV).

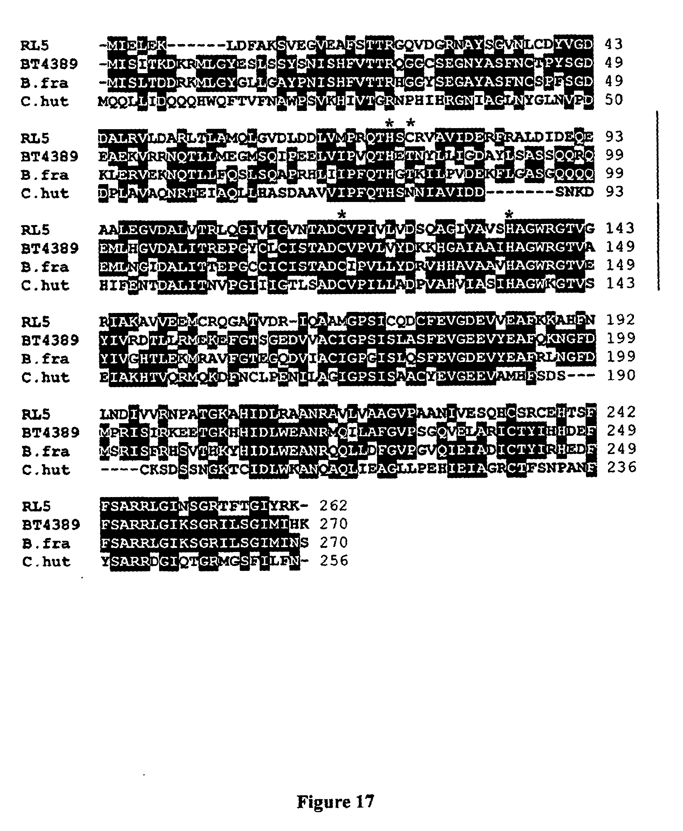

**Figure 17** shows multiple sequence alignment and identification of T1 cooper ligands (*) for the RL5 laccase. Multiple sequence alignments were made using ClustalW online tool (www.ebi.ac.uk/clustalw) for the protein sequences. Abbreviations: RL5, laccase from rumen metagenome; BT4389, B.fra and C.hut, hypothetical proteins from *Bacteroides thetaiotaomicron, Bacteroides fragilis* and *Cytophaga hutchinsonii,* respectively (Acc. no. AE016945.1, AP006841.1 and NZ AABD03000010.1). *, amino acids residues belonging to the T1 copper site.

Figure 18 shows inventive polypeptides (1 to 703), the sequences of which are given by their database (Gene Bank) accession numbers. The function of these polypeptides has not yet been found out in the art. These polypeptides contain DUF152 domains, which award - according to the invention - laccase activity to polypeptides 1 to 703. Any of these polypeptides may be used as laccase, in particular for the applications disclosed herein.

### Examples

### Materials

### Chemicals and enzymes

2,2'-Azino-bis(3-ethylbenzthiazoline-6-sulfonic acid) (ABTS), 2,6-dimetoxyphenol (DMP), 4-hydroxybenzyl alcohol, veratryl alcohol, guaiacol, 1-hydroxybenzotriazole (1-HBT) and violuronic acid were purchased from Sigma Chemical Co. (St. Louis, Missouri, USA). 3,4-dimethoxybenzyl alcohol, syringaldazine (SGZ) and phenol red were purchased from Aldrich (Oakville, Canada). All other chemicals were of analytical grade. Molecular mass markers for SDS-PAGE were obtained from Novagen (Madison, Wisconsin, USA). Restriction and modifying enzymes were from New England Biolabs (Beverly, Massachusetts, USA). DNase I grade II was from Boehringer Mannheim (Mannheim, Germany). Chromatographic media were from Amersham Pharmacia Biotech (Little Chalfont, UK). DNA manipulations were according to Sambrook *et al.* (1998) and according to manufacturer's instructions for the enzymes and materials employed.

### Bacterial strains

*E. coli* strains XL1-Blue MRF' (for library construction and screening), XLOLR (for expression of the laccase from phagemid) (both: Stratagene, La Jolla, California, USA), and TOP 10 (for site-directed mutagenesis and expression of mutants) (Invitrogen, Carlsbad, CA, USA), were maintained and cultivated according to the recommendations of suppliers and standard protocols described elsewhere (Sambrook *et al.,* 1989).

### Example 1: Retrieval of RL5 laccase

Recombinant laccase was retrieved from the bacteriophage lambda-based expression library established from DNA extracted from cow rumen fluid as described elsewhere (Ferrer et al., 2005). In detail, representative samples of total rumen contents (fluid and solid phase) were taken from one New Zealand dairy cow. Samples were collected and stored at -70°C until the DNA extraction was performed. Extraction of the total genomic DNA from rumen samples was performed using the bead-beating DNA extraction method using DNA Kit for Soil (Qbiogen, Heidelberg, Germany). For laccase selection, the infected cells were mixed with NZY soft agar containing 50 µM syringaldazine. From the selected phage, the pBK-CMV phagemid, hereinafter designated as pBK-RL5, has been excised using the co-infection with helper phage according to the Stratagene protocols (Stratagene, La Jolla, CA, USA). The insert DNA was sequenced from both ends using universal primers and primer walking procedure.

### Example 2: Site-directed mutagenesis

RL5 laccase mutants were prepared using a QuikChange XL® site-directed mutagenesis kit (Stratagene, La Jolla, CA, USA), according to the manufacturer's instructions, and using appropriate oligoniucleotides. Eight histidine residues (His or H) at positions 73, 135, 190, 207, 233 and 239 were replaced by alanine (Ala or A). Cysteine (Cys or C) residues at positions 75 and 118 were replaced by glutamine (Gln or Q). RL5-derived plasmids containing mutations were introduced into *E. coli* TOP10 by electroporation. Plasmids containing wild type and mutant genes were sequenced at the Sequencing Core Facility of the Instituto de Investigaciones Biomédicas (CSIC, Madrid) using the Applied Biosystems 377 automated fluorescent DNA sequencer. The sequencing primer used was as follows. F1: 5'-ATA GAA CTT GAG AAA TTG GAT TTT GC-3'.

### Example 3: Protein purification

### Purification of RL5 variants

Genes of the full-length wild type and mutant RL5 proteins were amplified and fused with a hexahistidine His₆ tag at the C-terminus, as follows. Genes were PCR-amplified from pBKRL5 plasmids using the oligonucleotide primers RL5F-Nde: 5'-GAA GGA GAT ATA CAT ATG ATA GAA CTT GAG AAA TTG GAT TTT GC -3' and RL5R-Kpn: 5'-TGG TAC CTT A*GT GGT GGT GGT GGT GGT G*TT TCC TAT ATA TTC CGG TGA AGG TGC G-3' (underlined are the nucleotides introduced for the histidine tag). Reactions were carried out in a total volume of 50 µL in the presence of 2 U of *Taq* polymerase (Qiagen, Hilden, Germany_) for 1 min 94°C, followed by 25 cycles of 20 s at 94°C, 60 s at 40°C, 1 min at 72°C, and a final elongation step for 5 min at 72°C and 15 min 10°C. The amplicons were purified by electrophoresis on a 0.75% agarose gel, cloned into the pCR2.1 plasmid (Invitrogen), and hybrid plasmids electroporated into *E. coli* TOP10 cells (Invitrogen), as recommended by the supplier, which were then plated on Luria-Bertani (LB) agar supplemented with kanamycin (50 µg/ml).

Transformant clones were cultured in LB supplemented with 50 µg/ml kanamycin and different concentrations of CuSO₄ ranging from 0.1 to 1 mM at 37°C in a 1 L-shaked flask. It was found that 500 µM CuSO₄ gave enzyme with the highest activity, with 10 µM giving only 10% and 1 mM giving less than 40%. When the cultures reached an OD₆₀₀ of 0.6, 1 mM IPTG was added, and incubation continued for 4 h. Cells were then collected by centrifugation (30 min, 8,000 g, 4°C), resuspended in 50 mM NaH₂PO₄ pH 6.1, 150 mM NaCl and 20 mM imidazole, after which lysozyme was added (1 mg/ml). Cell suspensions were incubated on ice for 30 min and then sonicated four times for 30 s. Cell lysates were centrifuged for 20 min at 4°C, 25,000 g, and the His₆-tagged enzymes were purified at 25°C on 1 ml HisTrap HP column (Amersham Pharmacia Biotech; Little Chalfont, UK). After washing columns with 4 ml of 20 mM NaH₂PO₄ pH 7.4, 150 mM NaCl and 20 mM imidazole, recombinant enzymes were eluted with 10 ml of 20 mM NaH₂PO₄, pH 7.4, 150 mM NaCl and 500 mM imidazole. The monitoring of enzyme activity during purification was carried out by spectrophotometric measurement of oxidation of SGZ at 530 nm. SDS-PAGE was performed on 12% (v/v) acrylamide gels, as described by Laemmli (30), in a Bio-Rad Mini Protean system. Protein concentrations were determined according to Bradford (31), with BSA as standard.

### Cloning and purification of DUF152 hypothetical proteins

Genes of the full-length DUF152 hypothetical protein from *B. thetaiotaomicron* DSM 2079 and *E. coli* were amplified and fused with a hexahistidine His₆ tag, as follows. The genes for hypothetical proteins BT4389 from *B. thetaiotaomicron* and YifH from *E. coli* were PCR-amplified from the genomic DNA using oligonucleotide primers: BT4389FNdel 5'-ACA TAT GAT TTC AAT CAC AAA AGA TAA AAG-3' and BT4389RKpn 5'-TGG TAC CTT AGT GGT GGT GGT GGT GGT GTT ATT TAT GAA TCA TGA TGA-3' (for BT4389) and DUF152Ndel F 5'-ACA TAT GAG TAA GCT GAT TGT CCC GC-3' and DUF152 Xhol R 5'-TCT CGA GCC AAA TGA AAC TTG CCA TAC G-3 (for YifH). Amplification condition was a follows: 95°C - 120 s, 30x[95°C - 45 s, 50°C - 60 s, 72°C - 120 s], 72°C - 500 s. The ca. 800-730 bp PCR products was purified by agarose gel electrophoresis, extracted by means of a Qi-aExll Gel Extraction Kit (Qiagen), cloned into the pCR2.1 plasmid by means of the TOPO TA Cloning kit (Invitrogen, California, USA), as recommended by the supplier, and electroporated into *E. coli* DH5α electrocompetent cells (invitrogen). Positive clones were selected on LB agar supplemented with kanamycin (50 µg/ml) and X-gal (5 mg/ml). Plasmids pCRBT4389 and pCRYifH harbouring the PCR-amplified DNA fragments were isolated using Plasmid Mini Kit (Qiagen) and sequenced using M13 and rM13 oligonucleotide primers. Fragments containing the coding sequences for BT4389 and yifH were excised from this plasmids by endonucleases *Nde*l and *Kpn* (for BT4389) and *Nde*l and *Xho*l (for YifH), gel-purified as above and ligated (14°C, 16 hrs, T4 DNA ligase from New England Biolabs) into the pET-3a plasmid vector (Novagen) that had been pre-digested with same endonucleases and dephosphorylated with Roche (Basel, Switzerland) shrimp alkaline phosphatase at 37°C for 1 hr. Ligation mixtures were transformed into *E. coli* DH5α electrocompetent cells (invitrogen) that were plated on LB agar supplemented with 50 µg/ml ampicillin. Culture conditions and protein purification was performed as for RL5 laccase (see above).

### Example 4: N-terminal sequencing

For N-terminal sequence determination, pure protein (20 ng) was subjected to the denaturing PAGE (10-15% polyacrylamide) in the presence of sodium dodecyl sulfate (SDS), and protein band was blotted to a polyvinylidene difluoride membrane (Millipore) using semidry blot transfer apparatus according to the manufacturer's instructions. The blotted membrane was stained with Coomassie Brilliant Blue R250, and after destaining with 40% methanol/10% acetic acid the bands were cut out and processed for N-terminal amino acid sequence.

### Example 5: High-resolution inductively coupled plasma mass spectroscopy (ICP-MS)

The metal ion content of RL5 wild type and variants was determined using a Perkin-Elmer Life Sciences ICP-MS (model PE ELAN 6100 DRC). The metal content was determined by dilution of 50 µg of enzyme with 5 ml of 0.5% (v/v) HNO₃ to digest the protein and release the metal ions and this solution was used without any further manipulation.

### Example 6: Assays with purified laccase

Laccase activity was determined spectrophotometrically in a thermostated spectrophotometer (Perkin-Elmer) at 40°C in 100 mM sodium citrate buffer, pH 4.5 (1 ml). The standard assay was performed at 530 nm, using SGZ as the oxidation substrate (ε₅₃₀=64,000 M⁻¹ cm⁻¹). The reaction was started by adding SGZ, from a stock solution in methanol, to a final concentration of 20 µM. Alternative substrates for measurement of laccase activity were ABTS (ε₄₂₀=38,000 M⁻¹ cm⁻¹), DMP (ε₄₆₈=14,800 M⁻¹ cm⁻¹), tetramethylbenzidine (ε_{655 nm} = 39,000 M⁻¹ cm⁻¹), veratryl alcohol (ε₃₁₀=9,000 M⁻¹ cm⁻¹), guaiacol (ε₄₇₀=26,600 M⁻¹ cm⁻¹), 3,4-dimetoxybenzyl alcohol (ε₃₁₀= 9,500 M⁻¹ cm⁻¹) and 4-metoxybenzyl alcohol (ε₅₀₀= 38,000 M⁻¹ cm⁻¹), at final concentrations of 1 mM. Oxidation of phenol red (75 µM) was determined by the decrease in absorbance at 432 nm. The activity:pH and activity:temperature relationships were determined by incubating the enzyme:substrate mixtures at different pH values and constant temperature (40°C), and at different temperatures (15-80 °C) and constant pH (4.5), respectively. Catalytic constants were derived by fitting the experimental data onto the Michaelis-Menten model. Control measurements without enzyme were carried out to correct for possible chemical oxidation of the substrates.

### Example 7: Electrochemical analysis

The electrochemical measurements have been performed with BAS CV-50W voltammetric analyzer. For gold surface experiments, a gold BAS disc electrode has been used. The electrode was cleaned following the following procedure: the electrode was firstly immersed in "piranha" solution, containing 1 part of hydrogen peroxide 33%v and 3 parts of sulphuric acid 98%, during 5 minutes, after which the electrode was rinsed thoroughly with milli Q water, and polished with Buehler® micropolish gamma alumina #3, 0.05 micron size, during 5 minutes, on a polishing cloth, further immersed into a milli Q water solution and exposed to ultrasounds during 15 minutes. Then, the electrode was cleaned with sulphuric acid oxidization cyclic voltammetry during 30 minutes. The electrode was rinsed three times with milli Q water and polished again for another 5 minutes with the same alumina powder. After the subsequent sonication during 15 minutes, the electrode was finally inmersed into a reductive 0.5 M NaOH solution during 30 minutes and rinsed with milli Q water. The laccase enzyme was immobilized by dropping 15 µL of the enzyme solution at a concentration of 5 mg/ml on the gold surface facing upward and covered to avoid evaporation. After 90 minutes, the second drop was added. After 180 minutes from the first addition, the laccase-modified gold electrode was measured in 100 mM phosphate buffer (pH 6.0) in the presence of oxygen. For measurements under anaerobic conditions, N₂ was bubbled during 15 minutes into the electrochemical cell. The same procedure was repeated for measuring in presence of 2,6-dimethoxyphenol, used as a test substrate. The immobilization on the carbon electrode was performed on a BAS glassy carbon electrode. The electrode was polished on a graphite-polishing cloth during 5 minutes, until mirror-like surface was achieved. The electrode was rinsed thoroughly with water and facing upwards 20 µl of the laccase solution (5 mg/ml), dropped on the classy carbon surface. This is covered and let to immobilize during 20 minutes. All the electrochemical measurements taken for the gold electrode are repeated for this electrode.

### Example 8: Molecular modelling

A search of the Protein Data Bank for proteins of known structure homologous to RL5 yielded two entries, 1 T8H and 1 RV9, showing respectively 33.2% and 31.8% sequence identity with RL5. Both are bacterial proteins of unknown biological function. The first one was obtained from *Bacillus stearothermophilus* and the second one from *Neisseria meningitides* (not published). We chose 1T8H as a suitable template. A structural alignment of RL5 and 1T8H sequences was obtained with GenTHREADER (Jones 1999) and used to retrieve a RL5 model from the Swiss-Model server (Guex and Peitsch, 1997; Guex *et al.,* 1999). Ramachandran plot of the predicted RL5 structure yielded 92% of the residues in favoured region and 7% in allowed region, indicating a good quality of the model.

### References

Altschul, S.F., Madden, T.L. Schaffer, A.A., Zhang, J,. Zhang, Z., Miller, W. and Lipman D.J. (1997). Gapped BLAST and PSI-BLAST: a new generation of protein database search programs. Nucleic Acids Res. 25, 3389-3402.
Antorini M, Herpoel-Gimbert, I., Choinowski, T., Sigoillot, J.C., Asther, M., Winterhalter, K. and Piontek, K. (2002) Purification, crystallisation and X-ray diffraction study of fully functional laccases from two ligninolytic fungi. Biochim. Biophys. Acta 1594, 109-114.
Bertand, T., Jolivalt, C., Caminade, E., Joly, N., Mougin, C. and Briozzo, P. (2002) Purification and preliminary crystallographic study of Trametes versicolor laccase in its native form. Acta Crystallogr. D Biol. Crystallogr. 58, 319-321.
Bertrand, T., Jolivalt, C., Btiozzo, P., Caminade, E., Joly, N., Madzak, C. and Mougin, C. (2002) Crystal structure of a four-cooper laccase complexed with an arylamine: insights into substrate recognition and correlation with kinetics. Biochemistry 41, 7325-7333.
Bourbonnais, R. and Paice, M.. (1990) Oxidation of non-phenolic substrates. An expanded role for laccase in lignin biodegradation. FEBS Lett. 267, 99-102.
Bradford, M.M. (1976) A rapid and sensitive method for the quantitation of microgram quantities of protein utilizing the principle of protein-dye binding. Anal. Biochem. 72, 248-254.
Chefetz, B., Chen, Y. and Hadar, Y. (1998) Purification and characterization of laccase from Chaetomium thermophilum and its role in humification. Appl. Envriron. Microbiol. 64, 3175-3179.
Claus, H. (2003) Laccases and their occurrence in prokaryotes. Arch. Microbiol. 179, 145-150.
Claus, H. (2004) Laccases: structure, reactions, distribution. Micron 25, 93-96.
Cowan, D., Meyer, Q., Stafford, W., Muyanga, S., Cameron, R. and Wittwer, P. (2005) Metagenomic gene discovery: past, present and future. Trends Biotechnol. 23, 321-32.
Eggert, C., Temp, U., Dean, J.F., Eriksson, K.E. (1996) A fungal metabolite mediates degradation of non-phenolic lignin structures and synthetic lignin by laccase. FEBS Lett. 391, 144-148.
Ferrer, M., Golyshina, O.V., Chernikova, T.N., Khachane, A.N., Martins Dos Santos, V.A.P., Strömpl, C., Elborough, K., Jarvis, G., Neef, A., Yakimov, M.M., Timmis, K.N. and Golyshin, P.N. (2005) Novel hydrolase diversity retrieved from a metagenome library of bovine rumen microflora. Env. Microbiol. In Press.
Guex, N., Diemand, A. and Peitsch M.C. (1999) Protein modelling for all. Trends Biochem. Sci. 24, 364-367.
Guex, N. and Peitsch, M.C. (1997) SWISS-MODEL and the Swiss-PdbViewer: An environment for comparative protein modelling. Electrophoresis 18, 2714-2723.
Hakulinen, N., Kiiskinen, L.L., Kruus, K., Saloheimo, M., Paananen, A., Koivula, A. Rouvinen, J. (2002) Crystal structure of a laccase from Melanocarpus albomyces with an intact trinuclear copper site. Nat. Struct. Biol. 9, 601-605.
Jones D. T. (1999) GenTHREADER: an efficient and reliable protein fold recognition method for genomic sequences. J. Mol. Biol. 287, 797-815.
Kawai, S., Umezawa, T., Shimada, M. and Hihuchi, T. (1988) Aromatic ring cleavage of 4,6-di(tert-butyl)guaiacol, a phenolic lignin model compound, by laccase of Coriolus versicolor. 1988. FEBS Lett. 236, 309-311.
Klonowska, A., Gaudin, C., Fournel., A., Asso, M., Le Petit, J., Giorgi, M. and Tron, T. (2002) Characterization of a low redox potencial laccase from the basidiomycete C30. Eur. J. Biochem. 269, 6119-6125.
Krause, D.O., Denman, S.E., Mackie, R.I., Morrison, M., Rae, A.L., Attwood, G.T. and McSweeney, C.S. (2003) Opportunities to improve fiber degradation in the rumen: microbiology, ecology, and genomics. FEMS Microbiol. Rev. 27, 663-693.
Laemmli, U.K. (1970) Cleavage of structural proteins during the assembly of the head of bacteriophage T4. Nature 227, 680-685.
Machczynski, M.C., Vijgenboom, E., Samyn, B. and Canters, G.W. (2004) Characterization of SLAC: a small laccase from Streptoomyces coelicor with unprecedented activity. Protein Sci. 13, 2388-2397.
Martins, L.O., Soares, C.M., Pereira, M.M., Teixeira, M., Costa, T., Jones, G.H. and Henriques, A.O. (2002) Molecular and biochemical characterization of a highly stable bacterial laccase that occurs as a structural component of the Bacillus subtilis endospore coat. J. Biol. Chem. 277, 18849-18859.
Mayer, A.M. and Staples, R.C. (2002) Laccases: new functions for an old enzyme. Phytochemistry 60, 551-565.
McGuirl, M.A. and Dooley, D.M. 1999. Cooper-containing oxidases. Curr. Opin. Chem. Biol. 3, 138-144.
Pickard, M.A., Roman, R., Tinoco, R. and Vázquez-Duhalt, R. (1999) Polycyclic aromatic hydrocarbon metabolism by white rot fungi and oxidation by Coriolopsis gallica UAMH 8260 laccase. Appl. Environ. Microbiol. 65, 3805-3809.
Piontek, K., Antorini, M. and Choinowski, T. (2002) Crystal structure of a laccase from the fungus Trametes versicolor at 1.90 Å resolution containing a full complement of coopers. J. Biol. Chem. 277, 37663-37669.
Ruijssenaars, H.J. and Hartmans, S. (2004) A cloned Bacillus halodurans multicooper oxidase exhibiting alkaline laccase activity. Appl. Microbiol. Biotechnol. 65, 177-182.
Sambrook, J., Fritsch, E.F. and Maniatis, T. (1989) In: Molecular Cloning: A Laboratory Manual (Cold Spring Harbor: Cold Spring Harbor Laboratory Press, 1989). [2nd ed.]
Sariaslani, F.S. (1989) Microbial enzyme,es for oxidation of organic molecules. Crit. Rev. Biotechnol. 9, 171-257.
Shah, V. and Nerud, F. (2002) Lignin degrading system of white-rot fungi and its exploitation for dye decolorization. Can. J. Microbiol. 48, 857-870.
Solano, F., Lucas-Elio, P., López-Serrano, D., Fernández, E. and Sánchez Amat, A. (2001) Dimethoxyphenol oxidase activity of different microbial blue multicooper proteins. FEMS Microbiol. Lett. 204, 175-181.
Solomon, E.I., Sundaram, U.M., Machonkin, T.E. (1996) Multicooper oxidases and oxygenases. Chem. Rev. 96, 2563-2605.
Stolz, A. (2001) Basic and applied aspects in the microbial degradation of azo dyes. Appl. Microbiol. Biotechnol. 56, 69-80.
Valderrama, B., Oliver, P., Medrazo-Soto, A. and Vázquez-Duhalt. R. (2003) Evolutionary and structural diversity of fungal laccases. Antonie van Leewenhoek 84, 289-299.

## Claims

1. Polypeptide comprising amino acids No. 80 to No. 150 of the amino acid sequence shown in Figure 9 or a functional fragment or a functional derivative thereof.

2. Polypeptide of claim 1 comprising amino acids No. 80 to No. 150, preferably No. 75 to No. 170, more preferably No. 60 to 195 most preferably No. 50 to 210 of the amino acid sequence shown in Figure 9.

3. Polypeptide of claim 1 comprising or consisting of the amino acid sequence shown in Figure 9.

4. Polypeptide of any of claims 1 to 3, wherein the polypeptide shows laccase activity.

5. Polypeptide of any of claims 1 to 3, wherein the polypeptide oxidizes aromatic and/or non-aromatic substrates.

6. Polypeptide of claim 4, wherein the aromatic substrates are selected from the group consisting of phenols, polyphenols, aromatic amines, polycyclic aromatic hydrocarbons.

7. Polypeptide of claim 4 or 5, wherein the aromatic substrates are selected from the group consisting of 2,6-dimethoxyphenol (DMP), guaiacol and 4-methoxybenzyl alcohol.

8. Polypeptide of claim 4, wherein the non-aromatic substrates are selected from non-phenolic substances, in particular benzyl alcohols, syringaldazine (SGZ), veratryl alcohol and 2,2'-Azino-bis(3-ethylbenzthiazoline-6-sulfonic acid) (ABTS).

9. Polypeptide of any of claims 1 to 8 further being coupled to a second functional peptide portion, e.g. a tag sequence.

10. Polypeptide of any of claims 1 to 9, wherein the polypeptide shows activity at optimal Kₘ values, preferably in the range of 0.20 µM to 35.0 µM, more preferably in the range of 0.30 µM to 28.0 µM, most preferably in the range of 0.40 µM to 2 µM.

11. Polypeptide of any of claims 1 to 10, wherein the polypeptide shows activity at optimal k_{cat} values, preferably in the range of 800 min⁻¹ to 80,000 min⁻¹, more preferably in the range of 1,000 min⁻¹ to 60,000 min⁻¹, most preferably in the range of 10,000 min⁻¹ to 40,000 min⁻¹, most preferably in the range of 40,000 min⁻¹ to 80,000 min⁻¹.

12. Polypeptide of any of claims 1 to 11, wherein the polypeptide shows activity at optimal k_{cat}/Kₘ ratios, preferably in the range of 40 min⁻¹µM⁻¹ to 170,000 min⁻¹µM⁻¹, more preferably in the range of 1,000 min⁻¹µM⁻¹ to 165,000 min⁻¹µM⁻1, even more preferably in the range of 50,000 min⁻¹µM⁻¹ to 160,000 min⁻¹µM⁻¹, most preferably in the range of 90,000 min⁻¹µM⁻¹ to 155,000 min⁻¹µM⁻¹

13. Polypeptide of any of claims 1 to 12, wherein the polypeptide shows activity at pH optimum, preferably in the range of pH 3.0 to 9.5, more preferably from 3.5 to 9.0.

14. Polypeptide of any of claims 1 to 13, wherein the polypeptide shows activity at temperature optimum, preferably in the range of 20°C to 75°C, more preferably in the range of 40°C to 70°C, even more preferably in the range of 50°C to 65°C, most preferably at 60°C.

15. Polypeptide of any of claims 1 to 14, wherein the polypeptide shows high stability towards its substrate.

16. Polypeptide of any of claims 1 to 15, wherein the polypeptide shows activity and high stability towards its substrate over a long time period, preferably for at least four hours.

17. Polypeptide of any of claims 1 to 16, wherein the polypeptide shows a combination of at least two features, preferably at least three features, more preferably at least four features, even more preferably at least five features, even more preferably six features most preferably seven features of claims 8 to 14.

18. Polypeptide of any of claims 1 to 17, wherein the polypeptide is derived from rumen, particularly from rumen ecosystem, preferably from bovine rumen, more preferably from New Zealand dairy cow.

19. A nucleic acid encoding a polypeptide of any of claims 1 to 18 or a functional fragment or functional derivative thereof.

20. The nucleic acid of claim 19 comprising or consisting of the nucleic acid sequence of Figure 10.

21. A vector comprising the nucleic acid of claim 19 or 20.

22. A host cell comprising the vector of claim 21 and/or the nucleic acid of claim 19 or 20.

23. A method for the production of the polypeptide of any of claims 1 to 18 comprising the following steps:
a. cultivating a host cell of claim 22 and expressing the nucleic acid under suitable conditions;
b. isolating the polypeptide with suitable means.

24. Use of a polypeptide of any of claims 1 to 18 or of a polypeptide, selected from the group consisting of protein sequences 1 to 703 according to figure 18 or their fragments or derivatives, as laccase.

25. Use of the nucleic acid of claim 19 or 20, of the vector of claim 21, of the cell of claim 22 or of a nucleic acid coding for a polypeptide, selected from the group consisting of sequences 1 to 703 according to figure 18 or a fragment or derivative thereof, of a vector comprising said nucleic acid or of a cell comprising said vector for the preparation of a polypeptide having laccase activity.

26. Use of a polypeptide, nucleic acid, vector or cell according to claim 24 or 25 as food additive, preferably as animal food additive, more preferably as animal food additive for pasture fed animals.

27. Use of a polypeptide, nucleic acid, vector or cell according to claim 24 or 25 for the treatment of cellulosic textiles or fibres, textile dyeing, stain bleaching, cotton fiber whitening, dye finishing, dye decolourisation, detoxification and/or laundry cleaning.

28. Use of a polypeptide, nucleic acid, vector or cell according to claim 24 or 25 for pulp and paper treatment.

29. Use of a polypeptide, nucleic acid, vector or cell according to claim 24 or 25 for environment protection, in particular in bioremediation, biodegradation, biodetoxification and/or biodecontamination of environmental pollutants.

30. Use of a polypeptide, nucleic acid, vector or cell according to claim 24 or 25 as biosensor.

31. Use of a polypeptide, nucleic acid, vector or cell according to claim 24 or 25 for the preparation of beverages, in particular for the preparation of wine.
